# EUROPÄISCHE PATENTSCHRIFT

(11) **EP 2 821 429 B1**
(45) Veröffentlichungstag und Bekanntmachung des Hinweises auf die Patenterteilung: **22.03.2017**
(21) Anmeldenummer: 14171233.1
(22) Anmeldetag: 05.06.2014
(51) Int. Cl.: C08G 77/388, C08L 83/08

(54) **Siloxan-Polymere mit zentralem Polysiloxan-Polymerblock mit terminalen organofunktionellen Resten umfassend Harnstoff- und/oder Carbamat-Gruppen sowie Aminosäure-Reste**
Siloxane polymers with central polysiloxane polymer block with urea and/or carbamate groups and amino acid residues with terminal organofunctional residues
Polymères siloxanes ayant un bloc polymère polysiloxane central avec des résidus organofonctionnels terminaux comprenant les groupes urée et/ou carbamate et des résidus d'acides aminés

(30) Priorität: 01.07.2013 DE 102013106906
(43) Veröffentlichungstag der Anmeldung: 07.01.2015
(73) Patentinhaber: Evonik Degussa GmbH, 45128 Essen (DE)
(72) Erfinder: Ritter, Helmut, 42111 Wuppertal (DE); Knudsen, Berit, 40699 Erkrath (DE); Knott, Wilfried, 45355 Essen (DE); Henning, Frauke, 45259 Essen (DE); Hartung, Christian, 45133 Essen (DE); Mennenga, Thiemo, 42781 Haan (DE)

(56) Entgegenhaltungen:
- WO-A1-2007/072000
- DE-A1-102011 110 921
- US-A- 4 684 538

## Beschreibung

Die Erfindung betrifft Siloxan-Polymere umfassend einen zentralen Polysiloxan-Polymerblock B mit terminalen oder am Polymerblock seitenständig gebunden seitenkammständigen organofunktionellen Resten umfassend IPDI und Aminosäure-Derivate, die über eine hydrophobe oder hydrophile Linker-Gruppe Q1', Q2' kovalent verbunden sind sowie Zusammensetzungen enthaltend diese Siloxane. Ferner werden Verfahren zu ihrer Herstellung und ihre Verwendung offenbart.

Zur Behandlung und Modifizierung der Eigenschaften von textilen Fasern sowie keratinischen Fasern als auch zur der Haut kann das Eigenschaftsprofil und der erzielte Effekt im Bereich der Haar-, Körperpflege oder Behandlung von Textilien deutlich verbessert werden, indem modifizierte Siloxane als Additive zugesetzt werden.

So lassen sich wesentliche Produkteigenschaften erheblich durch einen Zusatz von modifizierten Silikonen verbessern. Zu nennen sind die verbesserte Geschmeidigkeit von Cremes, das Hautgefühl, der Glanz von Haaren oder ihre Kämmbarkeit sowie auch die Wasserfestigkeit von Sonnencremes. So können aminofunktionelle Siloxane glanz- und griffvermittelnd in der Textil oder Haarpflege wirken. Eine Steuerung dieser Eigenschaften ist durch eine Variation von seitenständigen Aminoalkylreste der Siloxane möglich, indem der Stickstoffgehalt, der Aminoalkylrest variiert wird oder das Molekulargewicht des Siloxans eingestellt wird.

DE 10 2011 110921 A1 beschreibt Polysiloxane, die mindestens eine Betaingruppe enthalten, sowie ein Verfahren zu deren Herstellung. Sie betrifft ferner die Verwendung dieser Polymere als Pflegewirkstoff in Formulierungen, insbesondere für die Pflege und Reinigung von Haut und Hautanhangsgebilden, wie beispielsweise als Konditioniermittel für Haare.

Um das Anwendungsspektrum der Stickstoff enthaltenden Siloxane zu erweitern besteht ein Bedarf an weiteren Stickstoff-enthaltenden Siloxanen. Ein besonderer Fokus liegt dabei auf Siloxanen, deren Gerüst gezielt Bereiche mit unterschiedlichen Eigenschaften aufweist. So besteht ein Bedarf an Siloxanen, die hydrophobe Bereiche aufweisen und zugleich Bereiche aufweisen, die hydrophil oder wasserlöslich sind. Ein besonderer Bedarf besteht an Siloxanen, die sich über Wasserstoffbrückenbindungen an natürliche Oberflächen, wie keratinische Fasern oder auch textile natürliche Fasern, anlagern können. Besonders bevorzugt sollen die Siloxane bezüglich ihrer Hydrophile und/oder Hydrophobie einstellbar sein. Bevorzugt soll ein Siloxan oder ein Gemisch von Siloxanen entwickelt werden, dass sowohl als Additiv in kosmetischen Formulierungen oder bei der Behandlung von Textilien zur Anwendung kommen kann, um vorzugsweise die Kämmbarkeit der Fasern zu verbessern und zugleich eine verbesserte Haftung auf den Fasern ermöglicht, indem es mit beiden terminalen Bereichen oder zumindest an mehreren Bereichen über Wasserstoffbrückenbindungen an die Oberfläche keratinischer, synthetischer oder natürlicher textiler Fasern anhaften kann. Eine Aufgabe bestand darin, ein Gemini-Tensid (Bistensid oder Doppel-Tensid) bereitzustellen, dass zur Anwendung im Kosmetikbereich, in der Textilindustrie, in Waschmittelformulierungen sowie als Additiv zur Beeinfluss der Oberflächeneigenschaften von Lacken, Abformmassen etc. geeignet ist und beispielsweise die Spreitung von Wassertropfen etc., positiv beeinflussen kann.

Gelöst werden die Aufgaben durch das erfindungsgemäße Siloxan und Zusammensetzungen enthaltend diese entsprechend den Merkmalen der Ansprüche 1 sowie 22 und 23 betreffend die erfindungsgemäße Zusammensetzung enthaltend mindestens ein erfindungsgemäßes Siloxan sowie durch das Verfahren zur Herstellung nach Anspruch 15 als auch die erfindungsgemäße Formulierung entsprechend den Merkmalen des Patentanspruchs 24.

Überraschend konnten die Aufgaben gelöst werden durch die Bereitstellung von Siloxan-Polymeren, die einen zentralen Polysiloxan-Polymerblock B sowie mindestens zwei terminale oder mindestens eine terminale und mindestens eine seitenkettenständige, organofunktionelle Gruppe aufweisen, die jeweils abgeleitet sind aus einer Umsetzung einer Isocyanat-Gruppe von Diisocyanaten mit Aminosäuren, Aminosäure-Derivaten oder deren Salzen, wobei das gebildete Aminosäure-Isocyanat über einen Linker (Q1', Q1'AH, Q2', Q2'AH) an das Polysiloxan gebunden ist. Als Linker kommen vorzugsweise Alkylen-, -(CH₂)ₙ- (n = 2 bis 200), Aryl-, Arylalkylen, optional mit Heterotatomen O, N und/oder S, Aminoalklyen-, quartäre Aminoalkylen, Allyl-, Ester-, Amid-, Anhydride-, Harnstoff-, (Meth)acrylat-Gruppen enthaltende Verbindungen sowie substituierte und unsubstituierte Polyether, insbesondere Polyethylenglykol- [EO]v, Propylenglykol- [PO]w oder [EO]v[PO]w in Betracht.

Gegenstand der Erfindung ist mindestens ein Siloxan-Polymer der allgemeinen Formel I umfassend einen zentralen Polysiloxan-Polymerblock B,
(i) der mit organofunktionellen Resten substituiert ist, die organofunktionellen Reste umfassen vorzugsweise einen Alkylen-Rest mit 1 bis 22 C-Atomen und/oder eine Phenyl-Gruppen oder einen Polyether,
(ii) der Polymerblock B weist lineare und/oder verzweigte Strukturen mit mindestens zwei difunktionellen Siloxan-Einheiten auf,
(iii) der Polymerblock B weist an mindestens zwei terminalen Silizium-Atomen oder mindestens einem terminalen und mindestens einem seitenkettenständigen Silizium-Atom der Siloxan-Einheiten des Polymerblocks B die organofunktionellen Reste -Q1 und -Q2 auf, wobei die Reste gleich oder verschieden sind,

   Q2-B-Q1 (I)

   wobei -Q1 der allgemeinen Formel IIa und -Q2 der Formel IIb entspricht, die unabhängig gleich oder unterschiedlich sind,

   -Q1 = -Q1'-A-(C=O)-D-Q1"-A'-(C=O)-D'-Q1* (IIa)

   -Q2 = -Q2'-A-(C=O)-D-Q2"-A'-(C=O)-D'-Q2* (IIb)

   - mit A gleich -NH-, -O- oder -S- und D gleich -NH- jeweils unabhängig in Formeln IIa und IIb,
   - mit A' gleich -NH- und D' gleich -NH-, -O- oder -S- jeweils unabhängig in IIa und IIb, wobei jeder Rest Q1 und Q2 der Formeln IIa oder IIb mindestens eine bivalente Harnstoff-Gruppe und eine weitere bivalente Harnstoff- oder Carbamat-Gruppe aufweist,
   - mit Q1' und Q2' jeweils unabhängig umfassend einen bivalenten Kohlenwasserstoff-Rest mit 6 bis 200 C-Atomen optional umfassend mindestens ein Heteroatom O, N oder S, einen bivalenten Rest umfassend Aryl-, Arylalkyl-Gruppen optional umfassend mindestens ein Heteroatom O, N oder S oder Alkyl-, Aryl- oder Alkyl- und Aryl-Gruppen enthaltende Polyether-Reste,
   - mit Q1" und Q2" jeweils unabhängig umfassend einen bivalenten linearen, verzweigten und/oder cyclischen Alkyl-Rest mit 4 bis 200 C-Atomen, insbesondere Cyclohexenyl-Rest basiert, wie aus der Umsetzung von IPDI oder einen bivalenten Rest umfassend einen Aryl- und/oder Arylalkyl-Rest mit 6 bis 200 C-Atomen,
   - mit -D'-Q1* und -D'-Q2* mit D' wie vorstehend definiert und wobei -D'-Q1* und -D'-Q2* jeweils unabhängig Reste umfassen, die aus einer Aminosäure, einem Aminosäure-Derivat, deren Salze oder Mischungen davon abgeleitet sind.

Bevorzugt entspricht B den Formeln IIIa oder IIIb. Vorzugsweise ist Q1' oder Q2' jeweils unabhängig ein bifunktioneller linearer, verzweigter oder cyclischer Alkylen-Rest mit 1 bis 22 C-Atomen, ein bifunktioneller Aryl-, Arylalkylen-Rest mit 6 bis 30 C-Atomen oder Polyether. Sowie vorzugsweise mit Q1" und Q2", die jeweils unabhängig bifunktionelle lineare, verzweigte oder cyclische Alkylen-Gruppe mit 1 bis 22 C-Atomen oder bifunktionelle Aryl-, Arylalyklen-Reste mit 6 bis 30 C-Atomen sind.

Entsprechend einer besonders bevorzugten Ausführungsform weisen die erfindungsgemäßen Siloxan-Polymere der allgemeinen Formeln I, IIa und IIb als -D'-Q1* und -D'-Q2* jeweils unabhängig Reste auf, die aus einer Aminosäure, einem Aminosäure-Derivat oder deren Salzen abgeleitet sind und D' mit D' gleich -NH-jeweils unabhängig in -D'-Q1* und -D'-Q2* in alpha-Stellung zu einer Ester-, Carboxy- oder Carbonyl-Gruppe des Aminosäure-Derivates steht, wobei der Ester Alkyl-Ester mit 1 bis 25 C-Atome oder Aryl-Ester umfassen. Besonders bevorzugte Ester sind die Methyl- oder Ethylester der alpha-Aminosäuren, Derivate der alpha-Aminosäuren sowie die protonierten Formen der alpha-Aminosäuren, der Ester oder Derivate. Erfindungsgemäß werden die basischen alpha-Aminosäuren, deren Ester und/oder Amide zur Herstellung der Siloxane oder in Q1, Q2 eingesetzt. Als basische alpha-Aminosäuren gelten die Aminosäuren mit basischen, protonierbaren Seitenketten, wie Lysin, Histidin und Arginin. Besonders bevorzugt sind Histin und Arginin. Ebenso bevorzugt sind auch die polar/neutralen mit HS-, HO-, H₂N-Seitengruppen substituierte Aminosäuren und die sauren Aminosäuren Glutamin- und Asparaginsäure.

Ebenso Gegenstand der Erfindung sind Siloxan-Polymere der Formel I mit den Resten - Q1 und -Q2, wobei die Reste -D'-Q1* und -D'-Q2* jeweils unabhängig abgeleitet sind aus Aminosäuren, Aminosäure-Derivaten oder deren Salzen, insbesondere die Hydrochloride oder andere physiologisch verträgliche Salze, umfassend
- unpolare Aminosäuren umfassend Alanin, Valin, Methionin, Leucin, Isoleucin, Prolin, Tryptophan, Phenylalanin,
- basische Aminosäuren umfassend Lysin, Arginin, Histidin,
- polare und neutrale Aminosäuren Tyrosin, Threonin, Glutamin, Glycin, Serin, Cystein, Asparagin und/oder
- saure Aminosäuren ausgewählt aus Glutaminsäure und Asparaginsäure, sowie deren Mono-, Dicarbonsäureester, Amide mit primären Amino-Gruppen der Aminosäuren, Amide der Carbonsäure-Gruppen der Aminosäuren und/oder Ester mit den primären Hydroxy-Gruppen, Thioester der HS-Gruppe oder Amide mit sekundären Amino-Gruppen der Aminosäuren. Gegenstand der Erfindung sind auch Siloxan-Polymere ohne ein Lysin-Derivat, insbesondere ohne Lysin-Derivate basierend auf Amiden mit Fettsäuren. Als Carbamat-Gruppen gelten sowohl -O-(C=O)-NH- als auch -S-(C=O)-NH-Gruppen, die auch als Thiocarbamate (Thiolurethan) bezeichnet werden können.

Siloxane mit dem folgenden Substitutionsmuster haben besonders vorteilhafte Eigenschaften bezüglich einer verbesserten Kämmbarkeit von keratinischen Fasern, insbesondere Haaren, entsprechend dem nachfolgend offenbart Test aufgezeigt. Somit ist ein weiterer Gegenstand der Erfindung mindestens ein Siloxan-Polymer oder eine Zusammensetzung umfassend mindestens ein entsprechendes Siloxan-Polymer oder eine Mischung dieser, umfassend die Reste -Q1 und -Q2 der Formel I mit A gleich -O-, D gleich -NH-, A' gleich -NH- und D' gleich -NH-, -O- oder -S-, insbesondere mit D' gleich -NH, wobei die Reste -D'-Q1* und -D'-Q2* jeweils unabhängig abgeleitet sind aus Aminosäuren, Aminosäure-Derivaten, deren Salzen, protonierten Aminosäuren oder deren Derivaten, insbesondere umfassend unpolare Aminosäuren umfassend Alanin, Valin, Methionin, Leucin, Isoleucin, Prolin, Tryptophan, Phenylalanin, basische Aminosäuren umfassend Lysin, Arginin, Histidin, polare und neutrale Aminosäuren umfassend Tyrosin, Threonin, Glutamin, Glycin, Serin, Cystein, Asparagin und/oder die sauren Aminosäuren ausgewählt aus Glutaminsäure und Asparaginsäure, sowie deren Mono-, Dicarbonsäureester, Amide mit primären Amino-Gruppen der Aminosäuren, Amide der Carbonsäure-Gruppen der Aminosäuren und/oder Ester mit den primären Hydroxy-Gruppen oder Thioester der HS-Gruppe sowie deren Salze. In allen allgemeinen Formeln gilt, dass das folgende Zeichen 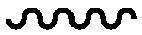 eine Bindungsstelle/ monovalente Bindungsstelle anzeigt, an die - nicht dargestellt - ein Atom, eine Gruppe bzw. Rest kovalent gebunden ist. Im Falle der substituierten Cyclohexyl-Reste basieren auf den Diisocyanatisophoron-Resten sind an den Bindungsstellen 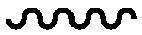 die NH(C=O)A- oder NH(C=O)D'-Reste gebunden. In den Formeln IIIa, IIIb sind die Restes Q2- und -Q1 kovalent an 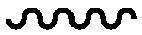 gebunden.

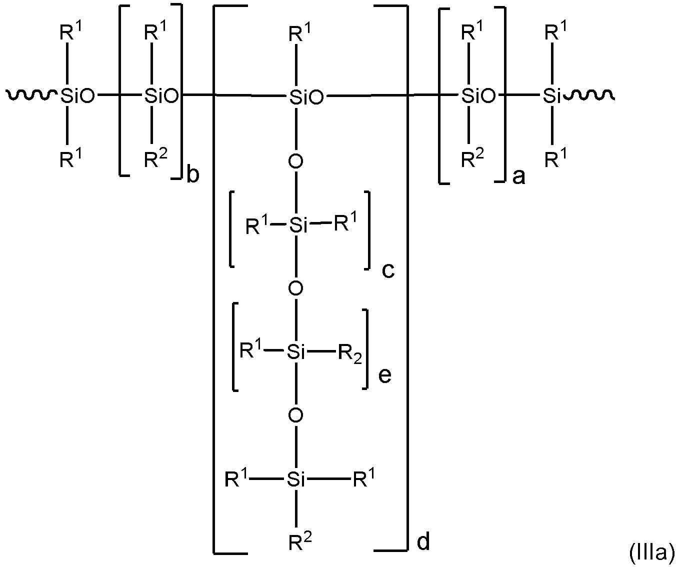

Entsprechend einer besonders bevorzugten Ausführungsform ist Gegenstand der Erfindung mindestens ein Siloxan-Polymer der allgemeinen Formel I oder Mischungen dieser, in dem der Polymer-Block B mindestens einer der allgemeinen Formeln IIIa oder IIIb entspricht, mit B gleich 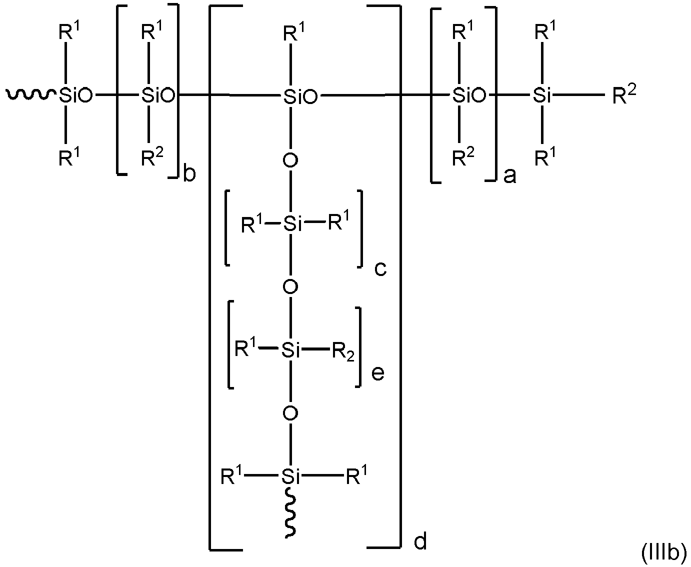
mit a, b, c, d und e in Formeln IIIa und IIIb jeweils unabhängig eine ganze Zahl
mit a von 1 bis 200, insbesondere 2 bis 150, bevorzugt 2 bis 100, besonders bevorzugt 5 bis 100, vorzugsweise 20 bis 100,
mit b von 0 bis 200, insbesondere 2 bis 150, bevorzugt 2 bis 100, besonders bevorzugt 5 bis 100, vorzugsweise 20 bis 100,
mit c von 0 bis 200, insbesondere 2 bis 150, bevorzugt 2 bis 100, besonders bevorzugt 5 bis 50, vorzugsweise 5 bis 20,
mit d von 0 bis 200, insbesondere 2 bis 150, bevorzugt 2 bis 100, besonders bevorzugt 5 bis 100, vorzugsweise 20 bis 100,
mit e von 0 bis 200, insbesondere 2 bis 150, bevorzugt 2 bis 100, besonders bevorzugt 5 bis 50, vorzugsweise 5 bis 20,
wobei (a + b +c +d +e) größer gleich 1, vorzugsweise größer gleich 20 sind, und mit R¹ in Formel IIIa oder IIIb jeweils unabhängig gleich oder verschieden, wobei R¹ umfasst Alkyl-Reste mit 1 bis 22 C-Atomen, vorzugsweise 1 bis 4 C-Atomen, besonders bevorzugt 1, 2, 3, 4, 6 C-Atome oder Phenyl-Reste, mit R² in Formel IIIa oder IIIb gleich Alkyl-Rest mit 1 bis 22 C-Atomen, vorzugsweise 1 bis 4 C-Atomen, besonders bevorzugt 1, 2, 3, 4, 6 C-Atome, ein Alkyl-Rest mit mindestens einem Heteroatom ausgewählt aus N, O S, wie ein Alkylamin, Alkylcarbonsäure, Alkylcarbonsäureamid, Alkylcarbonsäureanhydrid, (Meth)acrylat,Phenyl-Rest oder ein Rest der Formel -Q1'-A-(C=O)-D-Q1"-NH₂ und/oder Q2'-A-(C=O)-D-Q2"-NH₂. Besonders bevorzugt sind R¹ und R² ausgewählt aus Alkyl-Gruppen mit 1, 2, 3 oder 4 C-Atomen, insbesondere aus Methyl-Gruppen oder mindestens ein R² ist eine Aminoalkyl-Gruppe oder ein quartäres Alkylamin.

In besonders bevorzugten Siloxan-Polymere der Formel I, IIIa, und/oder IIIb sind die Indices b, c, d und e gleich 0 und a gleich 2 bis 200, vorzugsweise 20 bis 100, insbesondere ist a 30 oder 80 mit einer Schwankungsbreite von plus/minus 5.

Erfindungsgemäß sind die Reste -Q1 und -Q2 in der allgemeinen Formel I unabhängig ausgewählt sind aus

-Q1 = -Q1'-A-(C=O)-D-Q1"-A'-(C=O)-D'-Q1* (IIa)

-Q2 = -Q2'-A-(C=O)-D-Q2"-A'-(C=O)-D'-Q2* (IIb)

a) mit A gleich -O-, D gleich -NH-, A' gleich -NH- und D' gleich -NH-,
b) mit A gleich -NH-, D gleich -NH-, A' gleich -NH- und D' gleich -NH-,
c) mit A gleich -S-, D gleich -NH-, mit A' gleich -NH- und D' gleich -NH-,
d) mit A gleich -O-, D gleich -NH-, A' gleich -NH- und D' gleich -O-,
e) mit A gleich -NH-, D gleich -NH-, A' gleich -NH- und D' gleich -O- oder
f) mit A gleich -S-, D gleich -NH-, mit A' gleich -NH- und D' gleich -O-,
g) mit A gleich -O-, D gleich -NH-, A' gleich -NH- und D' gleich -S-,
h) mit A gleich -NH-, D gleich -NH-, A' gleich -NH- und D' gleich -S- oder
i) mit A gleich -S-, D gleich -NH-, mit A' gleich -NH- und D' gleich -S-,
wobei a) mit A gleich -O-, D gleich -NH-, A' gleich -NH- und D' gleich -NH- besonders bevorzugt ist.

Als besonders bevorzugte Diisocyanate und daraus abgeleitete Urethane haben sich in den Resten -Q1 und -Q2 der Formel I die bivalenten Reste -Q1 "- und -Q2"- gezeigt, die unabhängig ausgewählt sind aus bivalenten, linearen, verzweigten oder cyclischen Alkylen-Resten mit 4 bis 25 C-Atomen, insbesondere mit 6, 7, 8, 9, 10, 11, 12, 13 oder 14 C-Atomen, vorzugsweise Hexylen (-CH₂)₆, Heptylen, bivalentes 2,4-Toluolyl, Diphenylmethan, polymeres Diphenylmethan, 3,5,5-Trimethyl-1-methylen-3-ethylen-cyclohexan abgeleitet aus der Umsetzung von IPDI oder 4,4'-Dicyclohexylen. Erfindungsgemäß besonders bevorzugt werden Isophorondiisocyanate (IPID) eingesetzt, die aufgrund der Strukturisomerie eine gute Kontrolle des Verfahrens erlauben, da eine Isocyanat-Gruppe reaktiver ist und somit die Bildung von hochmolekularen Polymeren vermieden werden kann. Daher ist das Verfahren sehr gut reproduzierbar und die Siloxane sind mit definierten Molekulargewichten erhältlich.

Siloxan-Polymere entsprechend der Erfindung können vorzugsweise in den Resten -Q1 und -Q2 der Formel (I) mindestens als einen der bivalenten Reste -Q1 "- und -Q2"-unabhängig einen bivalenten Cyclohexan enthaltender Rest, ausgewählt aus den Formeln Va und Vb 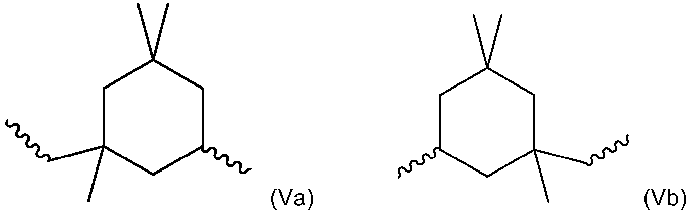 umfassen, insbesondere ist Q2"- ein bivalenter Cyclohexan enthaltender Rest der Formel Va und -Q1" der Formel Vb. Dabei wird aufgrund der unterschiedlichen Reaktivität der Isocyanat-Gruppen das Siloxan-Polymer, insbesondere der Formel I, vorzugsweise mit der dargestellten Struktur, insbesondere der Formeln Ia, Ib, Ia*, Ib*, als Hauptprodukt nach dem erfindungsgemäßen Verfahren nach Variante a) gebildet, die Verfahrensvariante b) führt bevorzugt zur Bildung der Siloxan-Polymere der Formel Ic.

Der Linker (-Q1'-A-, -Q2'-A-) ist bevorzugt aus einem olefinischen Alkohol mit 3 bis 200 C-Atomen, vorzugsweise mit 3 bis 25 C-Atomen, optional mit mindestens einem Heteroatom umfassend N, O oder S. Ebenso bevorzugt sind in den Resten -Q1 und -Q2 der Formel I die bivalenten Reste -Q1'- und -Q2'-, ausgewählt aus Alkylen-Resten mit 3 bis 22 C-Atomen optional mit mindestens einem Heteroatom umfassend N, O oder S, insbesondere -(CH₂)ₙ- mit n von 3 bis 22 optional mit mindestens einem Heteroatom umfassend N, O oder S, bevorzugt sind auch Hexylen (-CH₂)₆-, Heptylen (-CH₂)₇-, Octylen (-CH₂)₈-, Nonylen (-CH₂)₉-, Decylen (-CH₂)₁₀-, Undecylen (-CH₂)₁₁-, Dodecylen (-CH₂)₁₂-, oder mit mindestens einem Heteroatom, wie Alkylen-CO-, wie Alkylen-CO-, basierend auf der Umsetzung von 10-Undecensäure, 3-Butensäure, Acrylsäure, Methacrylsäure und 5-Hexensäure, Alkylen-O(CO)-Alkylen, Alkylen-(CO)O-Alkylen, Alkylen-NH(CO)-Alkylen, Alkylen-(CO)NH-Alkylen, Alkylen-NH(CO)NH-Alkylen, Alkylen-NH(CO)O-Alkylen, oder aus Alkyl-, Aryl- oder Alkyl- und Aryl-Gruppen enthaltenden Polyether-Resten der Formeln IVa oder IVb mit Q1' und Q2' jeweils unabhängig gleich

-T-O-(CH₂-CH₂-O-)ₓ-(CH₂-CH(R^{#})O-)_{y}-(SO)-R" (IVa)

-T-O-(CH₂-CH₂-O-)ₓ-(CH₂-CH(R^{#})O-)_{y}-R" (IVb),

mit T = bivalenter Kohlenwasserstoffrest mit 2 bis 4 C-Atomen, mit x = 0 bis 200, insbesondere mit 0 bis 100, vorzugsweise mit 0 bis 50, y = 0 bis 200, insbesondere mit 0 bis 100, vorzugsweise mit 0 bis 50, wobei x und y ganze Zahlen sind mit der Maßgabe, dass x oder y mindestens 1 ist, mit R^{#} Wasserstoff oder Methyl, R" Wasserstoff oder Alkylen, -(CH₂)₂-, -(CH(CH₃))CH₂-, Methylen, Polymethylen oder -(CH₂)₃-, insbesondere -CH₂-CH₂-, und vorzugsweise mit T = -(CH₂)₂- oder -(CH₂)₃-. Mit R" Wasserstoff in einem Edukt oder Zwischenprodukt und lineares oder verzweigtes Alkylen in einem Zwischen- oder Endprodukt.

Besonders bevorzugte bivalenten Reste -Q1 "- und -Q2"- sind Isophoron-Derivate, Cyclohexylen enthaltende Reste und Polymethylen, wie Hexamethylen.

Nachfolgende Siloxan-Polymere sind besonders bevorzugte Siloxan-Polymere und sind ausgewählt aus Siloxan-Polymeren der Formel Ia und Ib 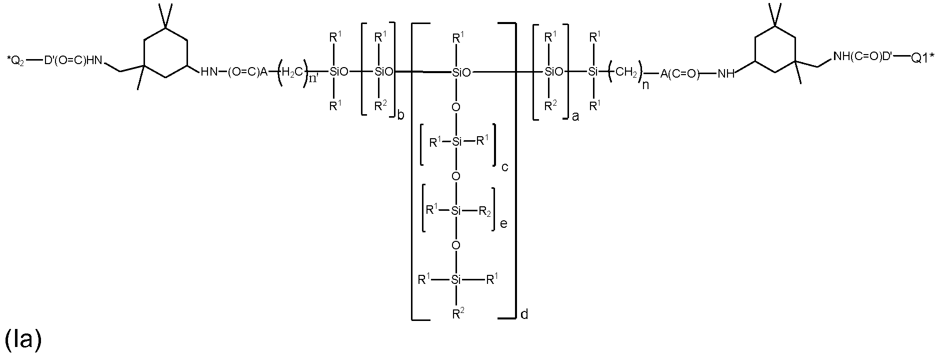 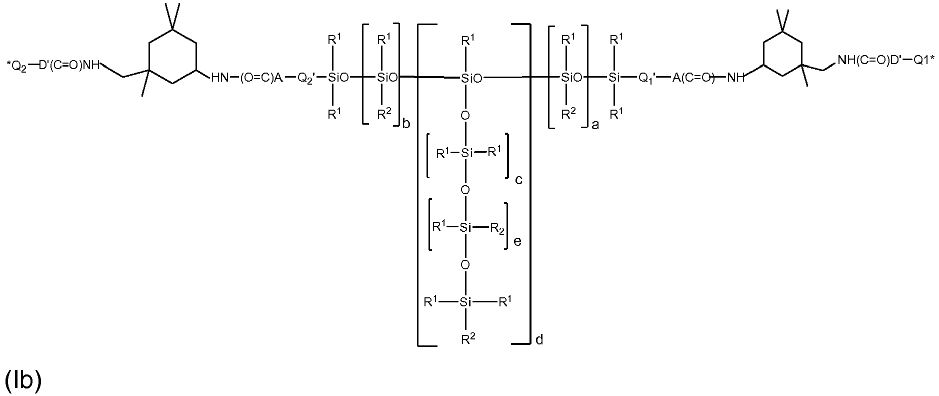 mit n oder n' jeweils unabhängig ausgewählt aus einer ganzen Zahl von 2 bis 40, insbesondere mit 3 bis 22, mit a von 1 bis 200, mit b von 0 bis 200, mit c von 0 bis 200, mit d von 0 bis 200,mit e von 0 bis 200 und mit R¹ in Formel Ia und Ib jeweils unabhängig gleich oder verschieden, wobei R¹ umfasst Alkyl-Reste mit 1 bis 4 C-Atomen oder Phenyl-Reste, mit R² gleich einem Alkyl-Rest mit 1 bis 22 C-Atomen, vorzugsweise 1 bis 4 C-Atomen, insbesondere 1, 2, 3, 4, 6 C-Atome, einem Alkyl-Rest mit mindestens einem Heteroatom ausgewählt aus N, O S, vorzugsweise Alkylamin, Glycidyloxyalkyl-Rest oder Phenyl-Rest und mit -D'-Q1* und -D'-Q2* jeweils unabhängig abgeleitet aus Aminosäuren, Aminosäure-Derivaten oder deren Salzen, und in Formel Ib mit Q1' und Q2' jeweils unabhängig gleich ein lineare, cyclisches, verzweigtes Alkylen mit 2 bis 40 C-Atomen oder eine Formel IVa oder IVb

-T-O-(CH₂-CH₂-O-)ₓ-(CH₂-CH(R^{#})O-)_{y}-(SO)-R" (IVa)

-T-O-(CH₂-CH₂-O-)ₓ-(CH₂-CH(R^{#})O-)_{y}-R" (IVb)

mit T = bivalenter Kohlenwasserstoffrest mit 2 bis 4 C-Atomen, mit x = 0 bis 200, insbesondere mit 0 bis 150, 1 bis 150, 5 bis 150, 5 bis 100, y = 0 bis 200, insbesondere mit 0 bis 150, 1 bis 150 5 bis 150 5 bis 100, wobei x und y ganze Zahlen sind mit der Maßgabe, dass x oder y mindestens 1 ist, mit R^{#} Wasserstoff oder Methyl, R" Alkylen, -(CH₂)₂-, -(CH(CH₃))CH₂-, Methylen, Polymethylen oder -(CH₂)₃-, insbesondere -CH₂-CH₂-, Ethylen, insbesondere mit T = -(CH₂)₂- oder -(CH₂)₃-,
R² zu einem Teil die Bedeutung der Reste R¹ haben können und die übrigen Reste R² unabhängig voneinander Reste der Formeln Id sein können, R² = -M-Z⁺ A⁻(Ic), wobei Reste R² jeweils ein Rest der Formel -M-Z⁺ A⁻ sind, Z⁺ ein Rest der Formel Id 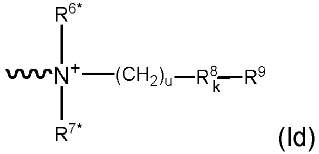
   ist, R^{6*}, R^{7*} jeweils gleich oder unterschiedliche Alkylreste mit 1 bis 22 Kohlenstoffatomen oder Alkenylreste mit 2 bis 22 Kohlenstoffatomen, worin die Alkyl- oder Alkenylreste Hydroxylgruppen aufweisen können,
   mit R⁸ ist -O-(C=O)- oder -NH(C=O)-,
   R⁹ ein einwertiger Kohlenwasserstoffrest mit 1 bis 22 Kohlenstoffatomen sein kann, oder u = 0 bis 6 in Formel Id,
   k = 0 oder 1 ist in Formel Id
   M ein zweiwertiger Kohlenwasserstoffrest mit mindestens 4 Kohlenstoffatomen ist, der eine Hydroxylgruppe aufweise kann und der durch ein oder mehrere Sauerstoffatome unterbrochen sein kann,
   A⁻ ein anorganisches oder organisches Anion, das von einer üblichen physiologisch verträglichen Säure HA herrührt, ist.

Eine Ausführungsform der Erfindung umfasst Siloxan-Polymere ausgewählt aus Siloxan-Polymeren der Formel Ia* und Ib* 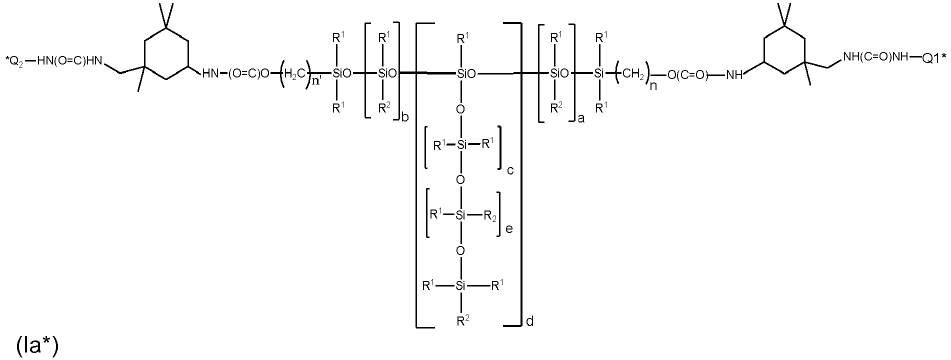 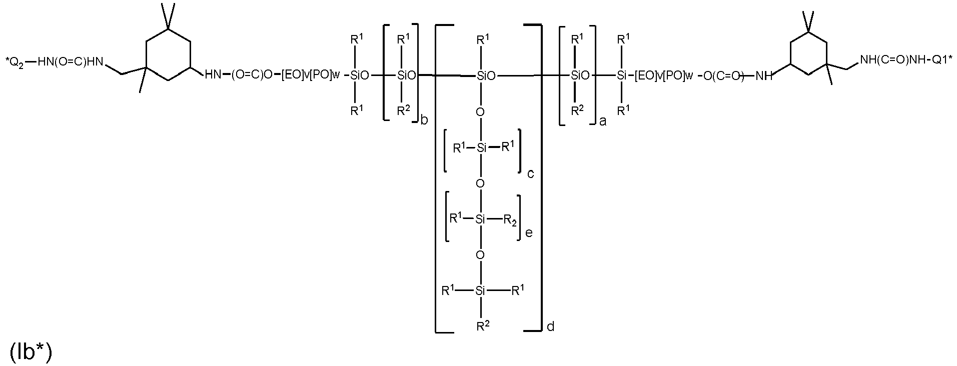 mit n oder n' jeweils unabhängig ausgewählt aus einer ganzen Zahl von 3 bis 22, mit a von 1 bis 200, insbesondere mit a von 20 bis 100, mit b von 0 bis 200, mit c von 0 bis 200, mit d von 0 bis 200, mit e von 0 bis 200, jeweils wie vorstehend definiert, und mit R¹ in Formel Ia* und Ib* jeweils unabhängig gleich oder verschieden, wobei R¹ umfasst Alkyl-Reste mit 1 bis 4 C-Atomen oder Phenyl-Reste, mit R² Alkyl-Rest mit 1 bis 22 C-Atomen, vorzugsweise 1 bis 4 C-Atomen, insbesondere 1, 2, 3, 4 C-Atome, einen Alkyl-Rest mit mindestens einem Heteroatom ausgewählt aus N, O S, wie Alkylamin, Glycidyloxyalkyl-Rest, oder Phenyl-Rest und mit Q1* und Q2* jeweils unabhängig ausgewählt aus Aminosäuren, Aminosäure-Derivaten oder deren Salzen, wobei das Fragment -NH-Q1* und -NH-Q2* durch Reaktion der sekundären alpha-Amino-Gruppen der Aminosäuren, deren Derivaten oder der Salze mit Isocyanat die Harnstoff-Gruppe bildet, und wobei die Aminosäuren umfassen unpolare Aminosäuren Alanin, Valin, Methionin, Leucin, Isoleucin, Prolin, Tryptophan, Phenylalanin, basische Aminosäuren umfassend Lysin, Arginin, Histidin, polare und neutrale Aminosäuren umfassend Tyrosin, Threonin, Glutamin, Glycin, Serin, Cystein, Asparagin und/oder die sauren Aminosäuren ausgewählt aus Glutaminsäure und Asparaginsäure sind, sowie deren Mono-, Dicarbonsäureester, Amide mit primären Amino-Gruppen der Aminosäuren, Amide der Carbonsäure-Gruppen der Aminosäuren und/oder Ester mit den primären Hydroxy-Gruppen oder Thioester der HS-Gruppe oder deren Salzen, und in Formel Ib* mit [EO]v[PO]w, mit v von 0 bis 200 und w von 0 bis 200, insbesondere jeweils unabhängig ist mindestens v oder/und w 5 bis 100, oder gleich Q1' und Q2' jeweils unabhängig gleich Formeln IVa oder IVb,

-T-O-(CH₂-CH₂-O-)ₓ-(CH₂-CH(R^{#})O-)_{y}-(SO)-R" (IVa)

-T-O-(CH₂-CH₂-O-)ₓ-(CH₂-CH(R^{#})O-)_{y}-R" (IVb),

mit T = bivalenter Kohlenwasserstoffrest mit 2 bis 4 C-Atomen, mit x = 0 bis 200, y = 0 bis 200, vorzugsweise wie vorstehend definiert, bevorzugt ist x und/oder y 5 bis 100, wobei x und y ganze Zahlen sind mit der Maßgabe, dass x oder y mindestens 1 ist, mit R^{#} Wasserstoff oder Methyl, R" Wasserstoff oder Alkylen, -(CH₂)₂-, -(CH(CH₃))CH₂-, Methylen, Polymethylen oder -(CH₂)₃-, insbesondere -CH₂-CH₂-, insbesondere mit T = -(CH₂)₂- oder -(CH₂)₃-.

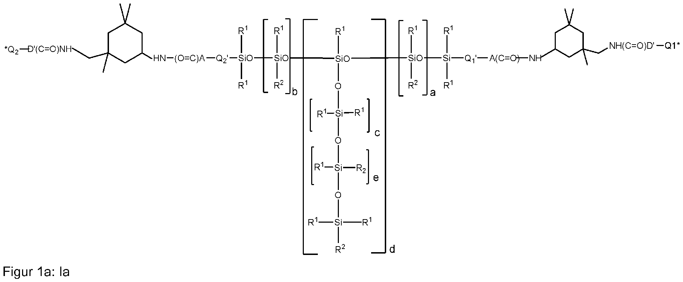

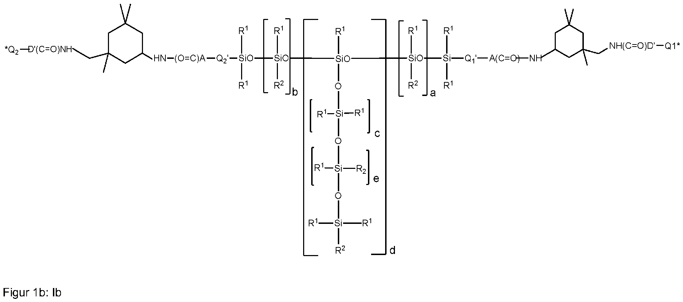

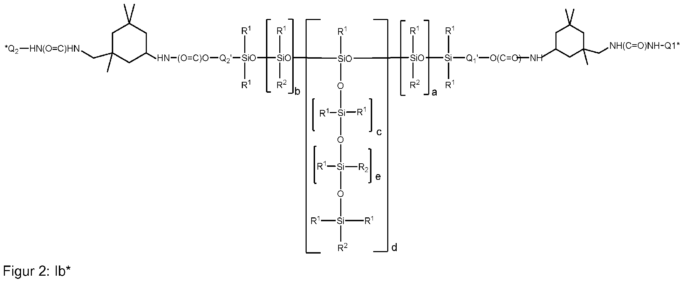

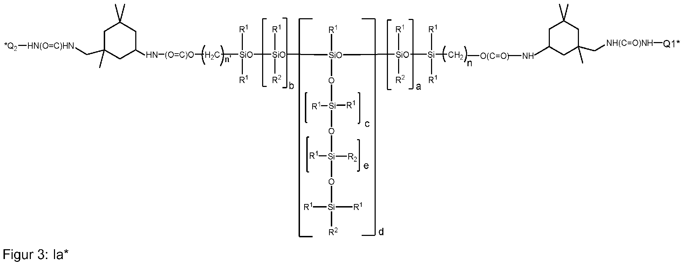

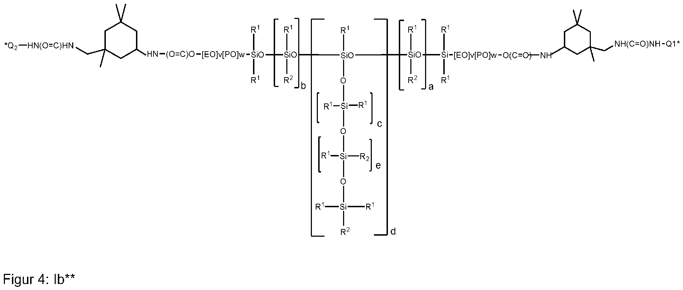

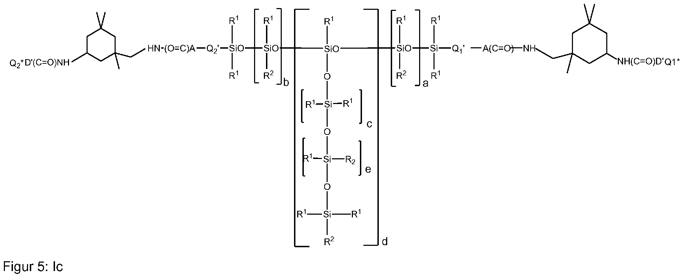

Die Figuren 1 bis 5 stellen beispielhaft die nach dem erfindungsgemäßen Verfahren erhältlichen Siloxan-Polymere der Formeln Ia, Ib, Ic dar, ohne die Erfindung auf diese Beispiele zu beschränken. Figur 1 a stellt ein Siloxan-Polymer der allgemeinen Formel Ia dar, das aus der Umsetzung mit Isophorondiisocyanat und einer Aminosäure erhältlich ist, wobei seitens der Aminosäure eine Hydroxy- oder Amino-Gruppe mit Isocyanat reagiert haben kann, der Linker ist ein Alkylen mit n oder n' jeweils unabhängig eine ganze Zahl zwischen 2 und 40. Figur 1b** stellt eine konkretisierte Verbindung für Q1' und Q2' mit einem Polyether als Linker dar. Figur 2 stellt eine bevorzugte Ausführungsform der allgemeinen Formel Ib dar, die ebenfalls durch eine Umsetzung mit einem Isophorondisocyanat und einer Aminosäure erhältlich ist, wobei die alpha-Amino-Gruppe der Aminosäure mit Isocyanat umgesetzt wurde und die zweite Isocyanat-Gruppe des Isophorons mit einem Hydroxy-funktionalisierten Siloxan bspw. einem Hydroxyalkyl-funktionalisierten Siloxan umgesetzt wurde. Figur 3 konkretisiert in Formel Ia* die Formel Ia dahingehend, dass Q2' und Q1' jeweils ein bivalentes Alkylen sind. In Figur 4 wird Ib* mit einem bivalenten Polyether dargestellt -[EO]v[PO]w-, mit v und w wie vorstehend definiert. Die Siloxan-Polymere der Formeln I können auch mit einem Aminoalkyl-funktiononalisierten Siloxan zu einem Siloxan-Polymer mit zwei Harnstoff-Gruppen und Aminosäure-Derivaten als terminale Gruppen umgesetzt werden. Figur 5 zeigt ein mögliches Isomer, wenn das Verfahren nach einer alternativen Route der Variante b) über die Herstellung von Aminosäure-Isocyanaten erfolgt, indem ein Aminosäure-Derivat mit IPDI zu einer Verbindung der Formel IX umgesetzt wird und anschließend eine Umsetzung mit einem Siloxan-Derivat der Formel VI erfolgt (Figur 5: Ic).

Entsprechend einer besonders bevorzugten Alternative kann in dem erfindungsgemäßen Verfahren ebenfalls ein Siloxan der allgemeinen Formel XI eingesetzt werden, insbesondere wie nachstehend erläutert. In dem bevorzugten Verfahren ist R¹⁷ dann gleich Wasserstoff oder -Q1'-A-(C=O)-D-Q1"-NCO, -Q2'-A-(C=O)-D-Q2"-NCO oder -Q1'-AH, -Q2'-AH.

Ebenfalls ist ein Siloxan der allgemeinen Formel XI als erfindungsgemäßes Siloxan-Polymer, insbesondere der allgemeinen Formel I, nach dem Verfahren erhältlich, wobei R¹⁷ jeweils unabhängig gleich -Q1 und -Q2 für a2 größer gleich 1 sind, insbesondere mit a2 gleich größer gleich 2.
1) Generell ist bevorzugt mindestens ein Siloxan der allgemeinen Formel XI nach dem erfindungsgemäßen Verfahren erhältlich mit a) mit R¹⁷ wie für Siloxan-Polymere in a) definiert.
2) Ebenso kann bevorzugt mindestens ein Siloxan der allgemeinen Formel XI in dem Verfahren eingesetzt werden mit R¹⁷ entsprechend der Definition in b) für die allgemeine Formel XI

   Mₐ₁M^{A}ₐ₂M^{B}ₐ₃D_{b1}D^{A}_{b2}D^{B}_{b3}T_{c1}T^{A}_{c2}T^{B}_{c3}Q_{d1} (XI)

   mit
   - M: = [R¹⁶₃SiO_{1/2}]
   - M^{A}: = [R¹⁷R¹⁶₂SiO_{1/2}]
   - M^{B}: = [R¹⁸R¹⁶₂SiO_{1/2}]
   - D: = [R¹⁶₂SiO_{2/2}]
   - D^{A}: = [R¹⁷₁R¹⁶₁SiO_{2/2}]
   - D^{B}: = [R¹⁸₁R¹⁶₁SiO_{2/2}]
   - T: = [R¹⁶SiO_{3/2}]
   - T^{A}: = [R¹⁷SiO_{3/2}]
   - T^{B}: = [R¹⁸SiO_{3/2}]
   - Q: = [SiO_{4/2}],
mit R¹⁶ unabhängig voneinander gleiche oder verschiedene lineare oder verzweigte, gesättigte oder ungesättigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl,
a) für das Siloxan-Polymer, insbesondere der Formel I, dargestellt über die Formel XI mit R¹⁷ ist jeweils unabhängig -Q1, -Q2 für ein Siloxan-Polymer der allgemeinen Formel I, wobei das Siloxan der Formel XI (ohne Reste R¹⁷, d.h. M^{A} = [-R¹⁶₂SiO_{1/2}], D^{A}= [-R¹⁶₁SiO_{2/2}], und/oder T^{A} = [-SiO_{3/2}]) dem Fragment B der Formel I entspricht und die Formel XI mit R¹⁷ der Formel I mit Q2-B-Q1 gleichkommt.
b) im Verfahren zur Herstellung der Siloxan-Polymere: Alternativ kann ein Siloxan der Formel XI mit R¹⁷ im Verfahren zur Herstellung mindestens eines Siloxan-Polymers, insbesondere der Formel I, eingesetzt werden, mit R¹⁷ umfassend -Q1'-AH, -Q2'-AH, -Q1'-A-(C=O)-D-Q1"-NCO, OCN-Q2"-D-(O=C)-A-Q2'-, Wasserstoff für Si-H-Gruppe, -OH, -OR¹⁶, insbesondere -OMe, -AH, besonders bevorzugt ist in einer Alternative R¹⁷ ein gesättigter Kohlenwasserstoffrest mit endständiger -OH oder -NH₂-Gruppe, vorzugsweise mit 8 bis 30, besonders bevorzugt mit 8 bis 20 C-Atomen, insbesondere in M^{A} und optional D^{A} oder R¹⁷ gleich R¹⁸ in M^{B}, D^{B} und/oder T^{B},
R¹⁸ unabhängig voneinander gleiche oder verschiedene lineare oder verzweigte, gesättigte oder olefinisch ungesättigte Kohlenwasserstoffreste mit 8 bis 30 Kohlenstoffatomen, beispielsweise Decyl-, Dodecyl, Tetradecyl-, Hexadecyl-, Octadecyl-, insbesondere Hexadecyl- und Octadecyl-,
einen aromatischen Kohlenwasserstoffrest mit 6 bis 40 Kohlenstoffatomen, einen Alkylarylrest mit 7 bis 40 Kohlenstoffatomen,
einen durch ein oder mehrere Heteroatome (Sauerstoff, NH, NR' mit R' gleich einem gegebenenfalls Doppelbindungen enthaltenden C1 bis C30-Alkylrest, insbesondere-CH₃) unterbrochenen linearen oder verzweigten gegebenenfalls Doppelbindungen enthaltenden aliphatischen Kohlenwasserstoffrest mit 2 bis 30 Kohlenstoffatomen,
einen durch eine oder mehrere Funktionalitäten, ausgewählt aus der Gruppe -OH, -O-C(O)-, -(O)C-O-, -NH-C(O)-, -(O)C-NH, - (CH₃)N-C(O)-, -(O)C-N(CH₃)-, -S(O₂)-O-, -O-S(O₂)-, -S(O₂)-NH-, -NH-S(O₂)-, -S (O₂)-N(CH₃)-, -N(CH₃)-S(O₂)-, unterbrochenen linearen oder verzweigten gegebenenfalls Doppelbindungen enthaltenden aliphatischen Kohlenwasserstoffrest mit 2 bis 30 Kohlenstoffatomen,
einen endständig OH, OR', NH₂, N(H)R', N(R')₂ (mit R' gleich einem gegebenenfalls Doppelbindungen enthaltenden C1- bis C30-Alkylrest) funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder
einen blockweise oder statistisch aufgebauten Polyether gemäß -(R⁵-O)ₙ-R⁶, wobei R⁵ ein 2 bis 4 Kohlenstoffatome enthaltender linearer oder verzweigter Kohlenwasserstoffrest, n 1 bis 100, vorzugsweise 2 bis 60, ist und R⁶ Wasserstoff,
einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen,
oder ein Rest -C(O)-R⁷ mit R⁷ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen, besonders bevorzugt ist in einer Alternative R¹⁸ ein gesättigter Kohlenwasserstoffrest mit endständiger -NH₂-Gruppen, vorzugsweise mit 8 bis 30 C-Atomen, besonders bevorzugt mit 8 bis 20 C-Atomen, mit
a1 = 0-200, bevorzugt 1-60, insbesondere 0,
a2 = 0-30, bevorzugt 1-20, insbesondere 2-10, wie 2, 3, 4, 5, 6, 7, 8, 9, 10,
a3= 0-30, bevorzugt 1-20, insbesondere 0, wie 1, 2, 2, 3, 4, 5, 6, 7, 8, 9, 10, 15, 20,
b1 = 2 bis 5000, bevorzugt 10 bis 1000, insbesondere 10-500, besonders bevorzugt 2 bis 100, vorzugsweise 10 bis 100,
b2 = 0 bis 100, bevorzugt 1 bis 30, insbesondere 1 bis10 oder 0,
b3 = 0 bis 100, bevorzugt 0 bis 30, insbesondere 1 bis 10 oder 0,
c1 = 0 bis 30, bevorzugt 1 bis 30,
c2 = 0 bis 30, bevorzugt 0 bis 5, insbesondere 0,
c3 = 0 bis 30, bevorzugt 0 bis 5, insbesondere 0,
d1 = 0 bis 30, bevorzugt 0 bis 5, vorzugsweise 0
mit der Maßgabe, dass mindestens einer der Indices ausgewählt aus a1, a2 und a3 ungleich 0 ist, insbesondere mit (a2+b2+c2) größer gleich 1, vorzugsweise ist a2 eine ganze Zahl zwischen 2 und 10, vorzugsweise 2 bis 5, wie 2, 3, 4 oder 5, wobei weiter bevorzugt ist, dass b1 von 10 bis 150, vorzugsweise 10 bis 100 ist. Optional können zusätzlich a1 und/oder a3 eine ganze Zahl zwischen 2 und 10, vorzugsweise 2 bis 5, wie 2, 3, 4 oder 5 sein. Mit der Maßgabe, dass (c1 + c2 + c3) eine ganze Zahl größer gleich 1 ist, wenn die Summe aus (a1+a2+a3) eine ganze Zahl größer 2 ist. Entsprechend einer Alternative können auch a1 und/oder a3 zusätzlich eine ganze Zahl größer 1 sein.

Erfindungsgemäß bevorzugte im Verfahren eingesetzte mindestens eine Gruppe ausgewählt aus Hydroxy- und Amino-Gruppe aufweisende Siloxane sind gekennzeichnet durch die Parameterkennzeichnung ausgewählt aus der Gruppe:
a1 = 0, a2= 2, a3 = 0, b1 = 10-100, b2 = 0, b3 = 0, c1= 0, c2 = 0, c3 = 0 und d1 = 0,
a1 = 0, a2= 2, a3 = 0, b1 = 10-100, b2 = 1-30, b3 = 0, c1= 0, c2 = 0, c3 = 0 und d1 = 0;
a1 = 0, a2= 2, a3 = 0, b1 = 20 -40, b2 = 1-30, b3 = 0, c1= 0, c2 = 0, c3 = 0 und d1 = 0,
a1 = 0, a2= 2, a3 = 0, b1 = 41-90, b2 = 1-30, b3 = 0, c1= 0, c2 = 0, c3 = 0 und d1 = 0,
a1 = 0, a2= 2, a3 = 0, b1 = 5-350, b2 = 0, b3 = 0, c1= 0, c2 = 0, c3 = 0 und d1 = 0,
a1 = 0, a2= 2, a3 = 0, b1 = 15-200, b2 = 0, b3 = 0, c1= 0, c2 = 0, c3 = 0 und d1 = 0,
a1 = 0, a2= 2, a3 = 0, b1 = 10-150, b2 = 0, b3 = 1 bis 5, c1 = 0, c2 = 0, c3 = 0 und d1 = 0;
a1 = 0, a2= 0, a3 = 2, b1 = 5-350, b2 = 0, b3 = 0, c1= 0, c2 = 0, c3 = 0 und d1 = 0,
a1 = 0, a2= 0, a3 = 2, b1 = 15-200, b2 = 0, b3 = 0, c1= 0, c2 = 0, c3 = 0 und d1 = 0,
a1 = 2, a2= 0, a3 = 2 bis 5, b1 = 10-150, b2 = 1-30, b3 = 0, c1≥ 0, c2 ≥ 0, c3 ≥ 0 und d1 = 0; mit (c1 + c2 + c3) größer gleich 1 bis 3
a1 = 0, a2= 2, a3 = 0, b1 = 10-150, b2 = 0, b3 = 1-2, c1= 0, c2 = 0, c3 = 0 und d1 = 0,
a1 = 0, a2= 2, a3 = 0, b1 = 51-90, b2 = 0, b3 = 1-2, c1= 0, c2 = 0, c3 = 0 und d1 = 0,
a1 = 0, a2= 0, a3 = 2, b1 = 10-50, b2 = 0, b3 = 1-2, c1= 0, c2 = 0, c3 = 0 und d1 = 0,
a1 = 0, a2= 1, a3 = 1, b1 = 10-150, b2 = 0, b3 = 1 bis 5, c1 = 0, c2 = 0, c3 = 0 und d1 = 0.

Die in Formeln I, II, III, IV, XI sowie allen dazugehörigen Unterstrukturen, die beispielsweise mit arabischen Buchstaben benannt sind, wiedergegebenen Indexzahlen a, b, c, d, e, f, a1, a2, a3, b1, b2, b3, c1, c2, c3, d1, d2, d3, v, w, n, n' etc. und die Wertbereiche der angegebenen Indices verstehen sich als die Mittelwerte der möglichen statistischen Verteilung der tatsächlich vorhandenen Strukturen und/oder deren Mischungen. Dies gilt auch für als solche an sich exakt wiedergegebene Strukturformeln, wie beispielsweise für Formel I, II, III und III, oder IIa, IIb, IIIa, IIIb.

Statistische Verteilungen können blockweise aufgebaut sein mit einer beliebigen Anzahl an Blöcken und einer beliebigen Sequenz oder einer randomisierten Verteilung unterliegen, sie können auch alternierend aufgebaut sein oder auch über die Kette einen Gradienten bilden, insbesondere können sie auch alle Mischformen bilden, bei denen gegebenenfalls Gruppen unterschiedlicher Verteilungen aufeinander folgen können. Spezielle Ausführungen können dazu führen, dass die statistischen Verteilungen durch die Ausführung Beschränkungen erfahren. Für alle Bereiche, die nicht von der Beschränkung betroffen sind, ändert sich die statistische Verteilung nicht.

Vorzugsweise ist R¹⁷ in M^{A} und/oder D^{A} optional T^{A} ausgewählt aus den beiden nachfolgenden Formeln IX1 und IX2 oder umfassen einen Rest der Formeln XIIa oder XIIb.

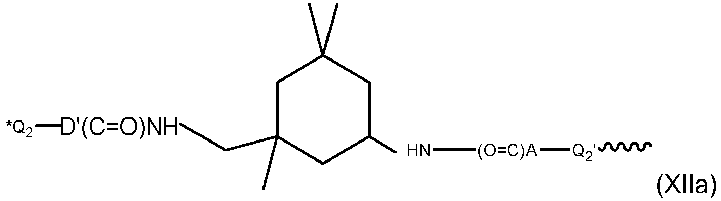

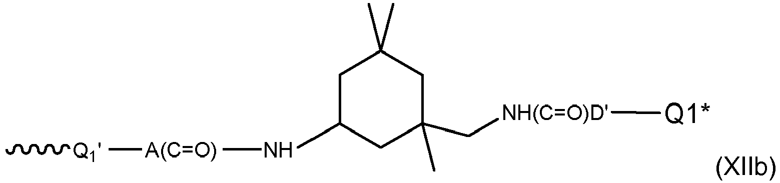

Zusätzlich zu den Aminosäure-Isocyanaten der allgemeinen Formeln (IXa bis IXd) können auch weitere substituierte Isocyanat-Derivate, vorzugsweise aus der Umsetzung mit Diaminen umfassend eine tertiäre und eine primäre Amino-Gruppen an Kohlenwasserstoffen optional umfassend O oder N in der Reaktion mit einem reakiven Hydroxy- oder Amino-funktionellen Siloxan, bspw. der Formel VI umgesetzt werden. Die Umsetzung kann auch in einer Mischung umfassen Aminosäure-Isocyanate erfolgen. In Formeln IX1 und IX2 kann Z = -Q1*, Q2* abgeleitet sein aus Formel XIII. oder einem Amin, vorzugsweise einem Diamin, wie DMPAPA, etc.

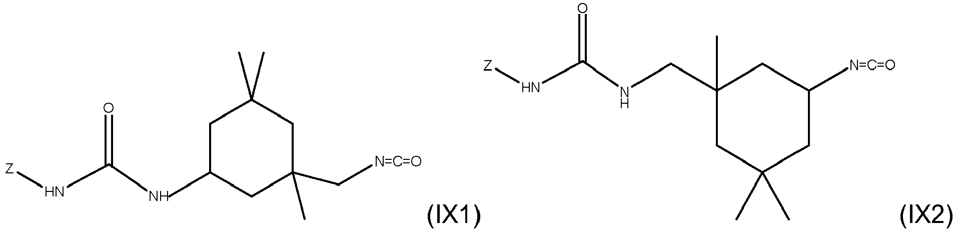

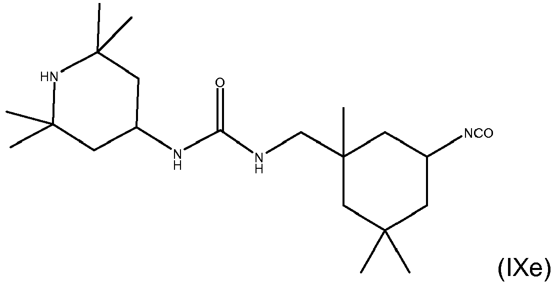

Nach einer Alternative kann zusätzlich zur Umsetzung mit Aminosäuren eine Umsetzung mit sterisch gehinderten Aminen mit einer primären Amino- oder HydroxyGruppe erfolgen, besonders bevorzugt sind die Diamine mit einem sterisch gehindertem Stickstoff als basische Gruppe, besonders bevorzugt sind HALS-Amin der Formel XIII (4-Amino-2,2,6,6,-tetramethylpiperidin) oder N,N-Dimethylaminopropylamin (DMAPA) bzw. 3-(Dimethylamino)propylamine (CAS:109-55-7), N-(3-Aminopropyl)imidazole (CAS: 5036-48-6), Dimethylethanolamin (CAS:108-01-0), Dimethylaminoethoxyethanol (CAS:1704-62-7); Trimethylaminoethylethanolamine (CAS: 2212-32-0) oder deren Salzen. Denkbar ist auch eine Umsetzung von seitenkettenständigen, funktionellen Gruppen oder Si-OH-Gruppen mit einem der vorgenannten Amine, vorzugsweise der Formel XIII.

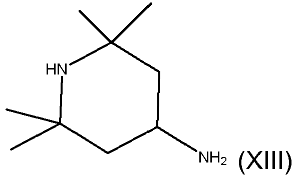

Ebenfalls Gegenstand der Erfindung ist ein Verfahren zur Herstellung eines Siloxan-Polymers, insbesondere der Formel I, vorzugsweise der Formel XI, sowie Siloxan-Polymere und Zusammensetzungen enthaltend diese Siloxan-Polymere erhältlich nach dem Verfahren mit einem zentralen Polysiloxan-Polymerblock B, insbesondere eines Verfahrens zur Herstellung mindestens eines Siloxan-Polymers der allgemeinen Formel (I), wie vorstehend beschrieben, sowie von Zusammensetzungen enthaltend diese Siloxan-Polymere oder Mischungen der Siloxan-Polymere mit einem zentralen Polysiloxan-Polymerblock B, indem a) ein Polysiloxan-Diisocyanat der Formel VII,

OCN-Q2"-D-(O=C)-A-Q2'-B-Q1'-A-(C=O)-D-Q1"-NCO (VII)

mit einer Aminosäure, einem Aminosäure-Derivat oder deren Salz, insbesondere mit einer sekundären Amino-Gruppen aufweisenden Aminosäure oder einem Derivat davon umgesetzt wird, wobei die Umsetzung vorzugsweise im molaren Verhältnis von mindestens 1 zu 1 in Bezug auf die Isocyanat-Gruppen des Polysiloxans zu Amino- oder Hydroxy-Gruppen der Aminosäure oder des Aminosäure-Derivates erfolgt,
und ein Siloxan-Polymer der allgemeinen Formel (I)

Q2-B-Q1 (I)

erhalten wird, wobei -Q1 der allgemeinen Formel IIa und -Q2 der Formel IIb entsprechen,

-Q1 = -Q1'-A-(C=O)-D-Q1"-A'-(C=O)-D'-Q1* (IIa)

-Q2 = -Q2'-A-(C=O)-D-Q2"-A'-(C=O)-D'-Q2* (IIb)

- mit A gleich -NH-, -O- oder -S- und D gleich -NH- jeweils unabhängig in Formeln IIa und IIb,
- mit A' gleich -NH- und D' gleich -NH-, -O- oder -S- jeweils unabhängig in Formeln IIa und IIb, wobei jeder Rest Q1 und Q2 der Formel IIa oder IIb jeweils unabhängig mindestens eine bivalente Harnstoff-Gruppe und eine weitere bivalente Harnstoff- oder eine Carbamat-Gruppe aufweist, oder
b) ein Polysiloxan der Formel VI

HA-Q2'-B-Q1'-AH (VI)

mit einem Aminosäure-Isocyanat ausgewählt aus den Formeln IXa,, IXb, IXc und IXd

Q2*-NH(CO)NH-"2Q-NCO (IXa)

Q2*-NH(CO)NH-Q2"-NCO (IXc)

Q1*-NH(CO)NH-"1Q-NCO (IXb)

Q1*-NH(CO)NH-Q1"-NCO (IXd)

umgesetzt wird,
- mit A gleich -NH-, -O- oder -S- und D gleich -NH- jeweils unabhängig in Formeln VII, I, VI, IXa, IXb, IXc und IXd, insbesondere umfassend IXa und IXb,
- mit Q1' und Q2' jeweils unabhängig umfassend einen bivalenten Kohlenwasserstoff-Rest mit 6 bis 200 C-Atomen optional umfassend mindestens ein Heteroatom umfassend O, N oder S, einen bivalenten Rest umfassend Aryl-, Arylalkyl-Gruppen oder einen bivalenten Rest
   umfassend Aryl-, Arylalkyl-Gruppen optional umfassend mindestens ein Heteroatom O, N oder S oder Alkyl-, Aryl- oder Alkyl- und Aryl- Gruppen enthaltende Polyether-Reste, jeweils unabhängig in Formeln VII, I und/oder VI,
- mit Q1" und Q2" jeweils unabhängig umfassend einen bivalenten linearen, verzweigten und/oder cyclischen Alkyl-Rest mit 4 bis 200 C-Atomen, insbesondere einem cyclischen C6 Alkyl-Rest mit Alkylseitenketten, oder einen bivalenten Rest umfassend einen Aryl- und/oder Arylalkyl-Rest mit 6 bis 200 C-Atomen, jeweils unabhängig in Formeln VII, IIVa und/oder IVb,
- mit -NH-Q1* und -NH-Q2* umfassend jeweils unabhängig Reste, die aus einer Aminosäure, Aminosäure-Derivat oder deren Salz abgeleitet sind. Optional jeweils unabhängig mit -O-Q1* und -O-Q2*, -S-Q1* und -S-Q2* umfassend jeweils unabhängig Reste, die aus einer Aminosäure, Aminosäure

Die Umsetzung in Schritt a) kann in Gegenwart eines Katalysators ablaufen, wie den im Stand der Technik bekannten Katalysatoren zur Polyurethanherstellung und Isocyanattrimerisierung. Beispielhaft genannt sind tertiäre Amine wie Triethylamin, Tetraethylendiamin, oder starke Basen wie DBU, sowie Zinn- und Bismuthverbindungen, wie beispielsweise Dibutylzinnlaurat oder Zinn(II)-octoat

Nach einer Verfahrensvariante umfassen die Formel IVa, IVb und I als -NH-Q1* und -NH-Q2* jeweils unabhängig Reste, die aus einer Aminosäure, Aminosäure-Derivat oder deren Salz abgeleitet sind und -NH in -NH-Q1* und -NH-Q2* jeweils in alpha-Stellung zu einer Ester- oder einer Carboxy-Gruppe der Aminosäure, dem Aminosäure-Derivat oder deren Salz steht, wobei der Ester Alkyl-Ester mit 1 bis 25 C-Atome oder Aryl-Ester umfasst. Bevorzugt sind Methyl-, Ethyl- oder Phenyl-Ester. HD'-Q1*, HD'-Q2* mit HD' jeweils gleich H₂N- und Q1* und Q2* Aminosäure-Rest.

Das erfindungsgemäße Verfahren kann insbesondere die folgenden Schritte umfassen sowie einzelne Schritte umfassen:
(I) H-B-H (x) + Q1'-AH (VIIIa), Q2'-AH (VIIIb) → HA-Q2'-B-Q1'-AH (VI)
(II) HA-Q2'-B-Q1'-AH + Diisocyanat → OCN-Q2"-D-(O=C)-A-Q2'-B-Q1'-A-(C=O)-D-Q1"-NCO (VII)
(III) OCN-Q2"-D-'(O=C)-A-Q2'-B-Q1'-A-(C=O)-D-Q1"-NCO (VII) + Aminosäure → Q2-B-Q1 (I)
oder alternativ
(Ia) Diisocyanat (bspw. IPDI) + Aminosäure → Q2*-NH(CO)NH-"2Q-NCO (IXa) + Q1*-NH(CO)NH-"1Q-NCO (IXb) und optional Q2*-NH(CO)NH-Q2"-NCO (IXc) und/oder Q1*-NH(CO)NH-Q1"-NCO (IXd)
(Ib) H-B-H (x) + Q1'-AH (VIIIa), Q2'-AH (VIIIb) → HA-Q2'-B-Q1'-AH (VI)
(II) HA-Q2'-B-Q1'-AH (VI) + Q2*-NH(CO)NH-"2Q2-NCO (IXa)/ Q1*-NH(CO)NH-"1Q-NCO (IXb) → Q2-B-Q1 (I)

Zur Herstellung der Siloxan-Polymere kann zunächst
(i) ein Polysiloxan-Gruppen enthaltender linearer und/oder verzweigter Polymerblock B, insbesondere der Formeln IIIa und/oder IIIb oder der Formel XI mit R¹⁷ = H und a2 größer gleich 2, b1 größer gleich 1 oder a1 größer gleich 1, a2 größer gleich 1 und b1 größer gleich 1, mit mindestens zwei terminalen Si-H-Gruppen oder mindestens einer terminalen Si-H-Gruppe und mindestens einer seitenkettenständigen Si-H-Gruppe, bspw. H-B-H (X), wobei -H zwei Si-H Gruppen entspricht, umgesetzt werden
(ii) mit einer olefinischen Verbindung umfassend Alkylen und optional mindestens ein Heteroatom wie N, O, S, insbesondere Alkenylenol, Alkylenamin, Alkylencarbonsäure, Alkylenester, Alkylenamid oder einen olefinischen Polyether umgesetzt wird, wobei die olefinische Verbindung jeweils unabhängig eine Allyl- oder Vinyl-Gruppe aufweist und den Formeln VIIIa und/oder VIIIb entspricht

   Q1' -AH (VIIIa) Q2'-AH (VIIIb)

   mit Q1' und Q2' jeweils unabhängig umfassend ein Alkenylen mit 6 bis 200 C-Atomen optional umfassend mindestens ein Heteroatom O oder N , Aryl- oder Arylalkyl-Gruppen optional umfassend mindestens ein Heteroatom O oder N, olefinischen Polyether mit -AH in Formeln VIIIa und VIIIb unabhängig ausgewählt aus -OH oder -NH₂. Die Umsetzung erfolgt vorzugsweise in (iii), in Gegenwart eines Katalysators, wie einem Karstedt-Katalysator, zu einem Polysiloxan der Formel VI,

   HA-Q2'-B-Q1'-AH (VI)

   wobei jeweils unabhängig in Formeln VIIIa, VIIIb und VI mit AH unabhängig ausgewählt sind aus -OH und -NH₂, und mit -Q2'- und -Q1'- jeweils unabhängig umfassend einen bivalenten Kohlenwasserstoff-Rest mit 6 bis 200 C-Atomen optional umfassend mindestens ein Heteroatom O oder N , einen bivalenten Rest umfassend Aryl-, Arylalkyl-Gruppen optional umfassend mindestens ein Heteroatom O oder N oder olefinischen Polyether.

Zur Umsetzung von olefinischen Verbindungen mit der Si-H-Gruppe werden Hydrosilylier-Katalysatoren eingesetzt. Üblich ist die Verwendung eines Karstedt-Katalysators. Generell sind Platinkatalysatoren bevorzugt, bei denen Platin(0) vorliegt.

Mercaptoalkyl substituierte Siloxane, insbesondere der Formel I, VI oder XI kann der Fachmann nach ihm aus dem Stand der Technik bekannten Verfahren über eine Kondensation und/oder Equilibrierung herstellen.

In einem nachfolgenden Verfahrensschritt kann das Polysiloxan der Formel VI

HA-Q2'-B-Q1'-AH (VI)

mit A ausgewählt aus -O, -NH bzw. AH ausgewählt aus -OH, -NH₂, und -SH mit -Q2'- und -Q1'- wie vorstehend definiert, umgesetzt werden mit einem Diisocyanat zu einem Polysiloxan-Diisocyanat der Formel VII, vorzugsweise ist das Diisocyanat IPDI,

OCN-Q2"-D-(O=C)-A-Q2'-B-Q1'-A-(C=O)-D-Q1"-NCO (VII)

mit -Q2"- und/oder -Q1 "- unabhängig ausgewählt aus einem bivalenten, linearen, verzweigten und/oder cyclischen Alkyl-Rest mit 4 bis 200 C-Atomen, insbesondere ein Isophoron-Rest, oder einem bivalenten Rest umfassend einen Aryl- und/oder Arylalkyl-Rest mit 6 bis 200 C-Atomen, wobei das molare Verhältnis von HA-Gruppen im Polysiloxan zu Isocyanat-Gruppen mindestens 1 zu 1 beträgt, insbesondere ist das Verhältnis 1 : 100 bis 1: 1, vorzugsweise 1 : 10 bis 1: 1.

Besonders bevorzugt wird ein Diisocyanat mit einem Aminosäure-Derivat oder dessen Salz, wie den Hydrochloriden von Methyl- oder Ethylestern der alpha-Aminosäuren mit Amino-Gruppe in alpha-Position, zu einem Aminosäure-Isocyanat umgesetzt, insbesondere zu einem Aminosäure-Isocyanat der ausgewählt aus den Formeln IXa und IXb.

Im nächsten Verfahrensschritt kann das hergestellte Polysiloxan-Diisocyanat der Formel VII oder ein beliebiges über ein anderes Verfahren hergestelltes Polysiloxan-Diisocyanat der Formel VII

OCN-Q2"-D-(O=C)-A-Q2'-B-Q1'-A-(C=O)-D-Q1"-NCO (VII)

mit B gleich einem linearen und/oder verzweigten Polysiloxan-Polymerblock B,
mit -Q2'- und -Q1'- jeweils unabhängig umfassend einen bivalenten Kohlenwasserstoff-Rest mit 6 bis 200 C-Atomen optional umfassend mindestens ein Heteroatom O, N oder S, einem bivalenten Rest umfassend Aryl-, Arylalkyl-Gruppen optional umfassend mindestens ein Heteroatom O, N oder S, oder Alkyl-, Aryl- oder Alkyl- und Aryl-Gruppen enthaltende Polyether-Reste, mit A jeweils unabhängig gleich -NH-, -O- oder -S- und D gleich -NH- jeweils unabhängig in Formel VII, und mit -Q2"- und/oder -Q1 "- unabhängig ausgewählt aus einem bivalenten, linearen, verzweigten und/oder cyclischen Alkyl-Rest mit 4 bis 200 C-Atomen, oder einem bivalenten Rest umfassend einen Aryl- und/oder Arylalkyl-Rest mit 6 bis 200 C-Atomen,
umgesetzt werden mit einer Aminosäure, Aminosäure-Derivate oder deren Salzen, insbesondere mit einer sekundären Amino-Gruppe der Aminosäure oder des Aminosäure-Derivats, vorzugsweise mit einem Methyl- oder Ethylester einer alpha-Aminosäure oder einem Salz der genannten Verbindungen.

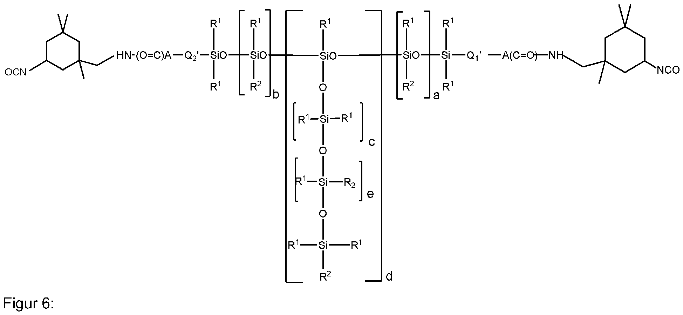

Figur 6 stellt ein erhältliches Diisocyanat nach Formel VII mit D jeweils gleich -NH- dar. Im erfindungsgemäßen Verfahren können di-funktionelle Isocyanate ausgewählt aus der Gruppe umfassend beispielsweise; Toluol-2,4-diisocyanat (TDI), Diphenylmethandiisocyanat oder Methylendiphenyldiisocyanat (MDI), Hexamethylendiisocyanat (HMDI), 2,2,4-Trimethylhexan-1,6-diisocyanat (TMDI), Polymeres Diphenylmethandiisocyanat (PMDI), Isophorondiisocyanat (IPDI), 4,4'-Diisocyanatodicyclohexylmethan (H12MDI) eingesetzt werden, wobei die aliphatischen Produkte bevorzugt sind, und Isophorondiisocyanat (IPDI) besonders bevorzugt ist.

Einige dieser Isocyanate weisen Stereozentren auf. Insbesondere wird auf die Isomere des Isophorons hingewiesen. Ausdrücklich sind alle denkbaren Isomere im Umfang dieser Erfindung eingeschlossen. So kann beispielsweise Isophorondiisocyanat in ein cis- und ein trans-Isomer unterschieden werden. Besonders bevorzugt ist Isophorondiisocyanat aus einem cis/trans Gemisch von 5:1 bis 1:5, bevorzugt 3:1 bis 1:3, weiter bevorzugt 1:1. Ein besonders bevorzugtes, kommerzielles Produkt besteht aus einem cis/trans Gemisch von 3:1. Der Einsatz von kommerziellem Isophorondiisocyanat ist bevorzugt. Isophorondiisocyanat ist unter anderen Bezeichnungen erhältlich, welche als Synonyma im Umfang dieser Erfindung eingeschlossen sind: 3-Isocyanatmethyl-3,5,5-trimethylcyclohexylisocyanat, 5-Isocyanato-1-(isocyanatomethyl)-1,3,3-trimethylcyclohexane, CA RN: 4098-71-9. Es sind diverse Handelsnamen üblich, häufig enthalten sie den Namen des Stammmoleküls Isophoron, es sind jedoch auch andere Handelsnamen geläufig: z.B. Desmodur®I (BAYER), Isocur IPDI 22-200 (ISO-ELEKTRA), VESTANAT® IPDI (EVONIK INDUSTRIES), welche ebenfalls im Umfang der vorliegenden Erfindung eingeschlossen sind. Übliche Spezifikationen für Isophorondiisocyanat sind: Gesamtchlorgehalt <400 mg/kg, hydrolysierbares Chlor <200 mg/kg, Reinheit >99,5 Gew.-%, Brechungsindex n25D 1,483 (DIN 51 423, Teil 2), NCO-Gehalt 37,5 - 37,8 Gew.-% (EN ISO 11 909 / ASTM D 2572), das kommerzielle Produkt wird als farblos bis leicht gelb beschrieben. Die genannten Isocyanate können optional zumindest teilweise Prepolymere umfassen.

Als Isocyanat-Gruppen haltige Verbindungen eignen sich alle bekannten Isocyanate. Bevorzugt im Sinne der erfindungsgemässen Lehre sind z. B. aromatische, aliphatische und cycloaliphatische Polyisocyanate mit einer zahlenmittleren Molmasse von unter 800 g/mol. So sind beispielsweise geeignet Diisocyanate ausgewählt aus der Reihe 2,4-/2,6-Toluoldiisocyanat (TDI), Methyldiphenyldiisocyanat (MDI), Triisocyanatononan (TIN), Naphthyldiisocyanat (NDI), 4,4'-Diisocyanatodicyclohexylmethan, 3-Isocyanatomethyl-3,3,5-trimethylcyclohexylisocyanat (Isophorondiisocyanat = IPDI), Tetramethylendiisocyanat, Hexamethylendiisocyanat (HDI), 2-Methylpentamethylendiisocyanat, 2,2,4-Trimethyl-hexamethylendiisocyanat (THDI), Dodecamethylendiisocyanat, 1,4-Diisocyanato-Cyclohexan, 4,4'-Diisocyanato-3,3'-dimethyl-dicyclohexylmethan, 4,4'-Diisocyanatodicyclohexylpropan(2,2), 3-Isocyanatomethyl-1-methyl-1-isocyanatocyclohexan (MCI), 1,3-Diisooctylcyanato-4-methyl-cyclohexan, 1,3-Diisocyanato-2-methyl-cyclohexan und [alpha],[alpha], [alpha]',[alpha]'-Tetramethyl-m- oder -p-xylylen-diisocyanat (TMXDI), sowie aus diesen Verbindungen bestehenden Gemischen.

Bevorzugte Ausgangsstoffe für die Herstellung der Urethan-Gruppen und vorzugsweise der Harnstoff-Gruppen haltigen Verbindungen sind Isophorondiisocyanat (IPDI) und/ oder 4,4'-Diisocyanatodicyclohexylmethan.

Bevorzugt werden die folgenden Aminosäuren oder Aminosäure-Derivat mit einer sekundäre Amino-Gruppe in dem Verfahren eingesetzt, und insbesondere wird die sekundäre Aminosäure-Gruppe mit einer Isocyanat-Gruppe umgesetzt.

Die Aminosäure, das Aminosäure-Derivat oder Salz ist vorzugsweise ausgewählt aus den folgenden Aminosäuren, Aminosäure Derivaten oder deren Salzen umfassend unpolare Aminosäuren ausgewählt aus Alanin, Valin, Methionin, Leucin, Isoleucin, Prolin, Tryptophan, Phenylalanin, basische Aminosäuren umfassend Lysin, Arginin, Histidin, polare und neutrale Aminosäuren umfassend Tyrosin, Threonin, Glutamin, Glycin, Serin, Cystein, Asparagin und/oder saure Aminosäuren ausgewählt aus Glutaminsäure und Asparaginsäure, wobei die Derivate der Aminosäuren umfassen die Mono-, Dicarbonsäureester, Amide der primären Amino-Gruppen der Aminosäuren, Amide der Carbonsäure-Gruppen der Aminosäuren und/oder Ester mit den primären Hydroxy-Gruppen oder Thioester der HS-Gruppe der Aminosäuren, insbesondere die Alkylester der Aminosäuren, bevorzugt die Methyl-, Ethyl-, Phenyl-Ester der Aminosäuren oder deren Salzen. Ebenso bevorzugt können Ester mit 1 bis 15 C-Atomen, Hydroxycarbonsäureester, Fruchtsäureester, Fettsäureester der Aminosäuren eingesetzt werden.

Gegenstand der Erfindung sind auch Zusammensetzungen erhältlich nach dem erfindungsgemäßen Verfahren, insbesondere umfassend Siloxan-Polymere mit mindestens zwei Harnstoff- und zwei Carbamat-Gruppen und umfassend einen Aminosäure-Rest, der vorzugsweise über die alpha-ständige Amino-Gruppen an das Siloxan-Polymer gebunden ist. Der Aminosäure-Rest kann ein Derivat einer Aminosäure oder ein Salz sein. Das Salz ist vorzugsweise ein physiologisch verträgliches Salz, umfassend Hydrochloride, Asparaginsäure, Fruchtsäuren, allgemein Hydroxysäuren, Mineralsäuresalze sowie weitere dem Fachmann bekannte pharmakologisch verträgliche Salze, insbesondere von Carbonsäuren.

Entsprechend einer weiteren Ausführungsform ist Gegenstand der Erfindung eine Zusammensetzung enthaltend Siloxan-Polymere mit einem zentralen Polysiloxan-Polymerblock B sowie Mischungen umfassend (i) mindestens ein Siloxan-Polymer der allgemeinen Formel I sowie Mischungen umfassend dieses Polymer,
(ii) mindestens ein Siloxan-Polymer der allgemeinen Formel Ia sowie Mischungen umfassend dieses Polymer oder
(iii) mindestens ein Siloxan-Polymer der allgemeinen Formel Ib sowie Mischungen umfassend dieses Polymer sowie der Formel Ia* und/oder Ib* als auch deren Mischungen oder Mischungen enthaltend diese.

Ein weiterer Gegenstand der Erfindung ist ein Zwischenprodukt zur Herstellung von Siloxan-Polymeren, insbesondere der Formel I, ausgewählt aus Aminosäure-Isocyanaten ausgewählt aus den Formeln IXa, IXb, IXc und IXd, insbesondere IVa*, IVb*, und optional IXc* und IXd*, oder deren Salzen oder Mischungen der Aminosäure-Derivate.

Q2*-NH(CO)NH-"2Q-NCO (IXa)

Q2*-NH(CO)NH-Q2"-NCO (IXc)

Q1*-NH(CO)NH-"1Q-NCO (IXb)

Q1*-NH(CO)NH-Q1"-NCO (IXd)

Mit -"2Q- und -"1Q- mit der Bedeutung, das die sekundären Isocyanat-Gruppen mit der Aminosäure reagiert hat und die primäre -CH2-NCO-Gruppe später mit einem Polysiloxan der allgemeinen Formel (VI) HA-Q2'-B-Q1'-AH reagieren kann, insbesondere mit B gleich Formel IIIa oder IIIb sowie alternativ in der Bedeutung der Formel XI mit R¹⁷ jeweils unabhängig gleich -Q1'-AH oder HA-Q2'- ungesetzt werden kann.
Mit Q2*, Q2", Q1* und Q1", wie vorstehend definiert, insbesondere mit Aminosäure-Isocyanaten der Formeln IXa* und IVb* oder deren Salzen

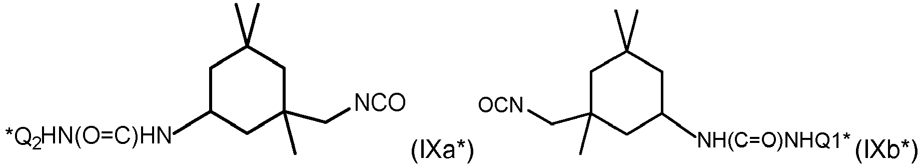

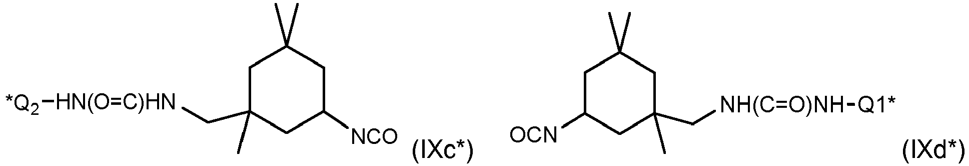

Die erfindungsgemäßen Siloxan-Polymere der Erfindung weisen vorteilhafte Eigenschaften auf, da sie die Kämmbarkeit von keratinischen Fasern, insbesondere von Haaren vorteilhaft beeinflussen. Vor Allem wird die Kraft, die zum Kämmen aufgebracht werden muss, sowohl gegenüber unbehandelten Haaren als auch gegenüber mit einer Standardformulierung behandelten nassen Haaren deutlich vermindert. Die Siloxane werden zuvor eine Minute aus den behandelten Haaren ausgespült. Gemessen wird anschließend die Verminderung der aufzubringenden Kraft zum Kämmen einer definierten Haarprobe. Die erfindungsgemäßen Siloxane vermindern die Kraft um etwa 51 bis 55 %, wobei das Siloxan mit n = 80, IPDI + Methyltryptophan die besten Ergebnisse aufzeigt. Die Matrixformulierung umfasst 0.5 Gew.-%, Ceterareth-25, 5.0 Gew.-% Cetyl-Alkohol, 1.0 Gew-% Cetrimonium-Chlorid, ad 100 Gew.-% Wasser, der pH-Wert beträgt etwa 4.3. Die erfindungsgemäßen Formulierungen umfassen zudem 0.5 Gew.-% Siloxan und ad 100 Gew.-% Wasser. Ebenso zeigen das Siloxan mit n = 80 + IPDI + Histidin sowie das Siloxan mit n = 30, + IPDI + Methyltryptophan eine Reduktion der Kämmkraft um 50 %. Der pH-Wert kann mit Zitronensäure reguliert werden.

Daher ist ein Gegenstand der Erfindung eine Formulierung, enthaltend mindestens ein Siloxan-Polymer oder eine Mischung enthalten mindestens ein Siloxan-Polymer oder über das Zwischenprodukt hergestelltes Siloxan-Polymer und mindestens einen Hilfsstoff. Vorzugsweise ist die Formulierung eine kosmetische Spülung für Haare, pflegendes Haut oder Haarprodukt, Lack, Haarspray, Haarfärbemittel, Farbe, Mundspülung, pharmazeutische Formulierung, Abformmasse (technisch, pharmazeutisch, kosmetisch, dental), Reinigungsmittel, Holzpflegeprodukt, Lackpflege.

Ein weiterer Gegenstand der Erfindung ist die Verwendung der Siloxan-Polymere, der erhältlichen Zusammensetzungen umfassend Siloxan-Polymere nach dem erfindungsgemäßen Verfahren als Additiv in kosmetischen Formulierungen, als Additiv in pharmazeutischen Formulierungen, in Lacken, Pasten, als Schaumstabilisator oder Schaumadditiv für Polyurethanschäume, insbesondere Polyurethanhartschäume und Polyurethanweichschäume, als Griffverbessere oder Imprägniermittel bei der Herstellung von Fasern, Textilien, in kosmetischen Formulierungen zur Behandlung, Nachbehandlung und Schutz von keratinischen Fasern, insbesondere in Haarkonditionierungsformulierungen. sowie Haut und Hautanhangsgebilden, als Additiv in Waschmittel-, Weichspülerformulierungen, in kosmetischen Formulierungen umfassend Cremes, Spülungen, Haarwachsmittel, Waschmittel, Festiger, Pflegespülungen, pflegende Pasten, Sprays, Haarsprays, zur Verbesserung der Kämmbarkeit von keratinischen oder textilen Fasern natürlichen oder synthetischen Ursprungs.

Ein weiterer Gegenstand der vorliegenden Erfindung ist die Verwendung der erfindungsgemäßen Siloxane und/oder der Siloxane erhältlich nach dem erfindungsgemäßen Verfahren zur Herstellung von Formulierungen, insbesondere von Pflege- und Reinigungsformulierungen für die Anwendung im häuslichen und industriellen Umfeld. Bevorzugte Pflege- und Reinigungsformulierungen für die Anwendung im häuslichen und industriellen Umfeld sind in diesem Zusammenhang Textilpflegemittel, wie beispielsweise Weichspüler, und Pflegemittel für harte Oberflächen.

Die generelle Synthese eines Isocyanat-terminierten PDMS erfolgt durch Umsetzung Hydroxy-terminierter PDMS verschiedener Kettenlängen, wie (n= 30 bzw. 80) (Die konkreten Verbindungen sind in den Herstellbeispielen näher erläutert; **14** bzw. **15**) mit Isophorondiisiocyanat (**16**), welches sich auf Grund seiner unterschiedlich reaktiven Isocyanat-Gruppen für eine Funktionalisierung der PDMS besonders empfiehlt. So kann ausgeschlossen werden, dass die Diisocyanat-Komponente zweifach mit den Hydroxylgruppen der PDMS reagiert und keine freie Isocyanat-Gruppe für eine weitere Reaktion mit einem Substituenten zur Verfügung steht. Des Weiteren kann dadurch eine Vergelung des Reaktionsgemisches bedingt durch die Bildung hoher Molekülmassen verhindert werden. Das Isophorondiisocyanat (**16**) wird hierfür zunächst in einer sekurierten Apparatur vorgelegt und unter Zugabe katalytischer Mengen Triethylamin mit dem zugetropften α,ω-Bis(hydroxyhexyl)-polydimethylsiloxan (PDMS-30 bzw. PDMS-80) (**14** bzw. **15**) in Substanz umgesetzt.

Die Eignung des kurzkettigen Valin-terminierten PDMS **20** für den Einsatz in Konditionierungsspülungen wurde entsprechend der vorstehend beschriebenen Anwendungstests beurteilt. PDMS-30-Val (**20**) wirkt sich demnach sowohl unter Zusatz eines Ammoniumchlorids als auch in reiner Form deutlich positiv auf die Entwirrung, die Kämmbarkeit und das Nass-Gefühl der Haarmuster nach deren Behandlung aus. Die Beurteilung erfolgte durch ein trainiertes Paneel von 4 Personen anhand von nassen Griffmustern mit den Noten 1, 2, 3, 4 und 5 bewertet, wobei 5 beste Sensorik, also ein sehr glattes Gefühl bedeutet und im Kontrast dazu der Wert 1 ein struppiges bzw. rauhes Gefühl vermittelt.

Da es sich jedoch bei Valin um eine eher unpolare Aminosäure handelt, wird die Funktionalisierung der PDMS über das gezeigte Diisocyanat auf eine Aminosäure angewendet, die einen polareren Charakter aufweist, wie Tryptophan.

Die mittels eines Diisocyanat hergestellten Tryptophan-PDMS-Derivate **23** und **24** sowie das langkettige Valin-PDMS-Derivat **21** werden ebenfalls zur Beurteilung ihrer Eignung in Haarpflegeprodukten ersten Anwendungstests nach bekanntem Verfahren unterzogen. Gemessen wird dabei die Kraft, die zum Kämmen eines Haarmusters benötigt wird, und in Form der verbesserte Kämmbarkeit des Präparats angegeben werden kann. Die Produkte **21, 23** und **24** wurden unter Zusatz einer Ammoniumchlorid-Verbindung in einer festgelegten Test-Formulierung auf die Haarmuster aufgetragen und wiederum nach einer bestimmten Zeit gründlich ausgespült. Alle mit den hergestellten PDMS-Aminosäure-Derivaten behandelten Haarmuster zeigen eine gute Reduzierung der Kämmkräfte, wobei die Derivate mit eher basischem Charakter wie PDMS-30-Trp (**23**) und PDMS-80-Trp (**24**) eine leicht verbesserte Kämmbarkeit der Präparate hervorrufen im Vergleich zu PDMS-80-Val (**21**).

Ebenfalls Gegenstand der Erfindung sind Verfahren zur Aufreinigung der Siloxan-Polymere, indem die Siloxan-Polymere in einer alkoholischen Lösung oder in Alkohol gelöst und in einer wässrigen Phase oder in Wasser ausgefällt werden. Die aufgereinigten Siloxan-Polymere können im Vakuum getrocknet werden.

Ebenfalls Gegenstand der Erfindung sind Verfahren zur Aufreinigung der Siloxan-Polymere, indem die Polysiloxane als Rohprodukt in einer Mischung, insbesondere umfassend Dichlormethan und Dimethylformamid, mit dest. Wasser und optional ges. wässr. Natriumhydrogencarbonat-Lösung gewaschen wird. Die organischen Phasen der Mischung werden vereinigt, über einem Trockenmittel getrocknet und das Lösungsmittel aus der Mischung im Vakuum entfernt. Das erhaltene Rohprodukt wird in Alkohol, insbesondere Ethanol gelöst, und in dest. Wasser ausgefällt und optional zentrifugiert. Die Trocknung kann im Vakuum, vorzugsweise im Hochvakuum, erfolgen.

Sofern im Umfang dieser Erfindung auf Naturstoffe Bezug genommen wird, z.B. Aminosäure, sind grundsätzlich alle Isomeren damit gemeint, bevorzugt sind die jeweils in der Natur vorkommenden Isomere, im hier genannten Falle also die alpha-Aminosäuren. Zur Definition von Naturstoffen wird auf den Umfang des "Dictionary of Natural Products", Chapman and Hall/CRC Press, Taylor and Francis Group, z.B. in der online Ausführung von 2011: http://dnp.chemnetbase.com/verwiesen.

Wo immer Moleküle beziehungsweise Molekülfragmente ein oder mehrere Stereozentren aufweisen oder aufgrund von Symmetrien in Isomere unterschieden werden können oder aufgrund anderer Effekte z.B. eingeschränkter Rotation in Isomere unterschieden werden können, sind alle möglichen Isomere von der vorliegenden Erfindung mit eingeschlossen. Isomere sind dem Fachmann bekannt, in besonderer Weise wird auf die Definitionen von Prof. Kazmaier der Universität des Saarlandes verwiesen, z.B. http://www.uni-saarland.de/fak8/kazmaier/PDF_files/vorlesungen/ Stereochemie%2Strassb%20Vorlage.pdf. Insbesondere sind alle Möglichkeiten, die sich aus den stereochemischen Definitionen der Taktizität ergeben eingeschlossen, z.B. isotaktisch, syndiotaktisch, heterotaktisch, hemiisotaktisch, ataktisch. Bevorzugt im Sinne der Erfindung sind Polyether und Polyetherfragmente mit zumindest teilweiser ataktischer Substituentenfolge.

Die folgenden Beispiele erläutern die erfindungsgemäßen Siloxan-Polymere sowie das erfindungsgemäße Verfahren näher, ohne die Erfindung auf diese Beispiele zu beschränken.

### Beispiele:

### Analytik:

MALDI-TOF-MS: Ultraflex time of flight-Massenspektrometer, Bruker. 337 nm Stickstofflaser, Linearmodus oder Reflektormodus. Eingewogene Proben wurden in geeignetem Lösungsmittel gelöst. Als Matrix diente Dithranol (DIT) oder 2,5-Dihydroxy-benzoesäure (DHB).

FT-IR-Spektren: FT-IR-5SXB, Nicolet verwendet. Kalibrierung: mittels HeNe-Lasers. ATR-Messungen: specac golden-gate Diamant ATR-Einheit.

NMR-Spektroskopie: 300 MHz-NMR Spektrometer, Bruker, Modell Avance III - 300, Magnetfeldstärke 7.05 Tesla. Absorptionsfrequenz: 1 H-NMR bei 300 MHz, 13C{1H}-NMR bei 75 MHz. 200 bzw. 500 MHz-NMR-Spektren: FT-NMR-Spektrometer, Bruker DRX200 bzw. DRX500.

### Beispiel 1: Synthese Isocyanat-terminierter Polydimethylsiloxane

Die Synthese eines Isocyanat-terminierten PDMS erfolgte durch Umsetzung Hydroxy-terminierter PDMS verschiedener Kettenlängen (n= 30 bzw. 80) (**14** bzw. **15**) mit Isophorondiisiocyanat (**16**). Der Vorteil des IPDIs ist, dass die Diisocyanat-Komponente nicht zweifach mit den Hydroxylgruppen der PDMS reagiert. Des Weiteren konnte eine Vergelung des Reaktionsgemisches bedingt durch die Bildung hoher Molekülmassen verhindert werden. Isophorondiisocyanat (**16**) wird in einer sekurierten Apparatur vorgelegt und katalytische Mengen Triethylamin mit dem zugetropften α,ω-Bis(hydroxyhexyl)-polydimethylsiloxan zugegeben (PDMS-30, n = 30 bzw. PDMS-80, n = 80) (**14** bzw. **15**) in Substanz umgesetzt. Vorzugsweise kann die Reaktion der α,ω-Bis(hydroxyhexyl)-polydimethylsiloxane **14** und **15** mit Isophorondiisocyanat (**16**) zu den α,ω-Bis[hexyl(3-(isocyanatomethyl)-3,5,5-trimethylcyclohexyl)carbamyl]-polydimethyl-siloxanen **17** und **18** (PDMS-30-IPDI, n=30 bzw. PDMS-80-IPDI, n=80) in Dichlormethan erfolgen (60 °C, 2 h).

Analytik Isocyanat-terminierter PDMS: MS, 1H-NMR-, IR-Spektroskopie, Molmassen (MALDI-TOF) bestätigen Verbindungen **17** und **18;** IR: charakteristische C-H Valenz- und Deformationsschwingungen des Polydimethylsiloxans bei 2961, 1412 und 1257 cm-1, 2256 cm-1 freie Isocyanat-Gruppen, 1709 cm-1 C=O Valenzschwingung Urethaneinheit.

### Beispiel 2: Synthese Valin-terminierter Polydimethylsiloxane

Valin (Val)-terminierter Polydimethylsiloxane wird hergestellt durch Umsetzung von α,ω-Bis[hexyl(3-(isocyanatomethyl)-3,5,5-trimethylcyclohexyl)carbamyl]polydimethylsiloxan **17** oder **18** in Dichlormethan (CH₂Cl₂) unter Bildung der Valin-terminierten Polydimethylsiloxanen **20** und **21** (PDMS-30-Val bzw. PDMS-80-Val). In Dichlormethan sind die Edukte vollständig löslich. Die Produkte werden nach extraktiver Trennung aus der organischen Phase erhalten, getrocknet und analysiert.

IR-Spektren der Valin-terminierten PDMS (PDMS-30-Val bzw. PDMS-80-Val) (**20** bzw. **21**): Molekülschwingungen PDMS, C=O: Urethan-Gruppe bei 1726 bzw. 1729 cm-1, C=O: 1635 bzw. 1641 cm-1 Harnstoff-Gruppe, fehlt: NCO-Bande bei 2256 cm-1.

### MS-Messungen (MALDI-TOF): (m/z= 1433 für 20 und m/z= 3507 für 21).

Methylester-Schutzgruppen der Valin-Substituenten werden nur in Spuren nachgewiesen. 1 H-NMR Spektroskopie: zeigt Bildung Valin-terminierten PDMS-Derivate **20** (n= 30) und **21** (n= 80).

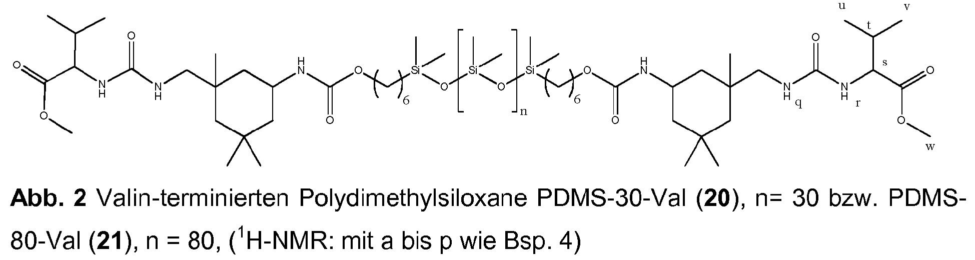

### Beispiel 3: Synthese Tryptophan-terminierter Polydimethylsiloxane

Tryptophan (Trp)-terminiertes PDMS wird durch Umsetzung von Isocyanatmodifiziertem PDMS unterschiedlicher Kettenlängen in Lösungsmittelgemisch aus Dichlormethan und DMF (1:1) hergestellt. Das Rohprodukt wird in wässriger Lösung umgefällt. Lösungsmittelrückstände des DMF werden so aus den Produkten entfernt. Tryptophan-terminierten PDMS werden unter Hochvakuum getrocknet.

IR- und 1 H-NMR Spektroskopie, MALDI-TOF Spektrometrie bestätigen die Bildung des Tryptophan-terminierten Polydimethylsiloxans.

Frei vorliegende Isocyanat-Endgruppen könne durch gezielte Hydrolyse vermieden werden. Die erzielte Reinheit der PDMS-Derivate ermöglicht deren Verwendung in kosmetischen Formulierungen für die Haut.

Die Herstellung der Produkte **23** und **24** kann wie folgt beschrieben werden: Umsetzung von PDMS-30-IPDI (**17**, n = 30) bzw. PDMS-80-IPDI (**18**, n = 80) mit Tryptophanmethylesterhydrochlorid (**22**) bspw. In Gegenwart von Dichlormethan, Triethylenamin, bei Raumtemperatur für etwa 24 Stunden zu Tryptophan-terminierten Polydimethylsiloxanen **23** und **24** (PDMS-30-Trp bzw. PDMS-80-Trp) optional Abtrennen des Hydrochlorids des Triethylenamins.

### Beispiel 4: Synthese kurzkettiges α,ω-Bis[hexyl(3-(isocyanatomethyl)-3,5,5-trimethylcyclohexyl)carbamyl]-polydimethylsiloxan (PDMS-30-IPDI) (17)

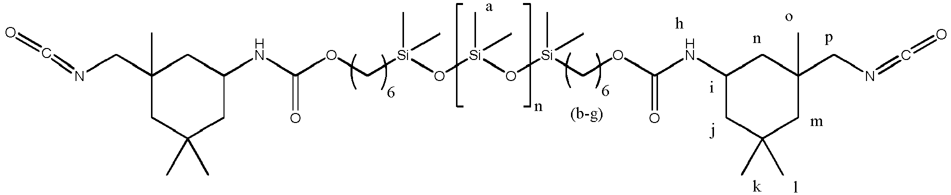

Isophorondiisocyanat (4,72 mL, 22,5 mmol) wird in einem sekurierten Rundkolben mit Rückflusskühler und Tropftrichter vorgelegt. Unter Schutzgasatmosphäre und Rühren wird auf 60 °C erhitzt. 0,1 Gew.-% Triethylamin (32 mg) werden hinzugefügt. Innerhalb von 2 h wird α,ω-Bis(hydroxyhexyl)-poly(dimethylsiloxan) (n=30) (26.4 g, 11.0 mmol) langsam zugetropft. Es wird solange gerührt, bis keine Trübung mehr auftritt. Das Produkt wird in quantitativer Ausbeute erhalten.

1 H-NMR (300 MHz, CDCl3, 24 °C): δ [ppm]= 3.98 (4H, m, H-g), 3.57 (2H, m, H-i), 2.98 (4H, m, H-p), 1.88-1.38 (8H, m, H-f/H-n), 1.26 (12H, m, H-c/H-d/H-e), 1.20-0.72 (26H, m, H-j/H-k/H-l/H-m/H-o), 0.46 (4H, m, H-b), 0.00 (168H, m, H-a); FT-IR (Diamant): ũ [cm-1]= 2961 (u R-CH3, Si-CH3, m-w), 2256 (u -NCO, Isocyanat, s), 1709 (u C=O, Urethan, s), 1412 (δ C-H, Si-CH3, w), 1257 (δ C-H, Siloxan, s-m), 1011 (u Si-O-Si, Siloxan, s-m); MALDI-TOF-MS m/z: 1542 [M+Na]+ (für n=10), disubstituiert, (¹H-NMR: mit a bis p wie Bsp. 4)

### Beispiel 5: Synthese langkettiges α,ω-Bis[hexyl(3-(isocyanatomethyl)-3,5,5-trimethylcyclohexyl)carbamyl]-polydimethylsiloxan (PDMS-80-IPDI) (18), Struktur analog Beispiel 4 mit n = 80

Isophorondiisocyanat (4,72 mL, 22,5 mmol) wird in einem sekurierten Rundkolben samt Rückflusskühler und Tropftrichter vorgelegt. Unter Schutzgasatmosphäre und Rühren wird auf 60°C erhitzt. 0,1 Gew. % Triethylamin (32 mg) werden hinzugegeben. α,ω-Bis(hydroxyhexyl)-poly(dimethylsiloxan) (n=80) (67.1 g, 11.0 mmol) wird innerhalb von 2 h langsam zugetropft. Es wird solange gerührt, bis keine Trübung mehr auftritt. Das Produkt wird in quantitativer Ausbeute erhalten.

1 H-NMR (300 MHz, CDCl3, 24 °C): δ [ppm]= 4.01 (4H, m, H-g), 3.77 (2H, m, H-i), 3.01 (4H, m, H-p), 1.89-1.42 (8H, m, H-f/H-n), 1.33 (12H, m, H-c/H-d/H-e), 1.24-0.76 (26H, m, H-j/H-k/H-l/H-m/H-o), 0.51 (4H, m, H-b), 0.00 (396H, m, H-a); FT-IR (Diamant): ũ [cm-1]= 2960 (u R-CH3, Si-CH3, m-w), 2256 (u -NCO, Isocyanat, s), 1711 (u C=O, Urethan, s), 1411 (δ C-H, Si-CH3, w), 1257 (δ C-H, Siloxan, s-m), 1010 (u Si-O-Si, Siloxan, s-m); MALDI-TOF-MS m/z: 2283 [M+Na]+ (für n=20), disubstituiert

### Beispiel 6: Synthese Valin-terminiertes, kurzkettiges Polydimethylsiloxans (PDMS-30-Val) (20), n = 30, Abb. 2

Valin-methylester-hydrochlorid (0.75 g, 4.5 mmol) wird in einem sekurierten Rundkolben samt Rückflusskühler und Tropftrichter in 20 mL Dichlormethan gelöst. Triethylamin (0.625 mL, 4.5 mmol) wird hinzugegeben. α,ω-Bis[hexyl(3-(isocyanatomethyl)-3,5,5-trimethylcyclohexyl)carbamyl]-poly(dimethylsiloxan) (n=30) (4.26 g, 1.5 mmol) wird in 20 mL Dichlormethan gelöst und per Tropftrichter unter Rühren innerhalb von 1 h zugetropft. Nach 24 h Rühren bei RT wird mit je 2x 20 mL dest. Wasser und 1 x 20 mL ges. wässr. Natriumhydrogencarbonat-Lösung gewaschen. Die organischen Phasen werden vereinigt, über Natriumsulfat getrocknet und das Lösungsmittel am Rotationsverdampfer entfernt. Das Produkt wird abschließend im Hochvakuum getrocknet. Ausbeute: PDMS-30-Val: 86%.

1 H-NMR (600 MHz, CDCl3, 24 °C): δ [ppm]= 4.39 (2H, m, H-s), 4.01 (4H, m, H-g), 3.87 (2H, m, H-i), 3.71 (6H, m, H-w), 2.89 (4H, m, H-p), 2.10 (2H, m, H-t), 1.75-1.45 (8H, m, H-f/H-n), 1.31 (12H, m, H-c/H-d/H-e), 1.21-0.73 (38H, m, H-j/H-k/H-l/H-m/H-o/H-u/H-v), 0.46 (4H, m, H-b), 0.00 (174H, m, H-a); FT-IR (Diamant): ũ [cm-1]= 2961 (u R-CH3, Si-CH3, m-w), 2904 (u CH3, Ester, w) (u C-H, freie Aminosäure, m), 1726 (u C=O, Ester, v) (u C=O, Urethan, m-w), 1635 (u C=O, N-CO-N, v), 1561 (u C=O, N-CO-N, v), 1412 (δ C-H, Si-CH3, w), 1257 (δ C-H, Siloxan, s-m), 1011 (u Si-O-Si, Siloxan, s-m); MALDI-TOF-MS m/z: 1433 [M+Na]+ (für n=5), disubstituiert

### Beispiel 7: Synthese Valin-terminiertes, langkettiges Polydimethylsiloxan (PDMS-80-Val) (21), n = 80, Abb. 2

Valin-methylester-hydrochlorid (0.75 g, 4.5 mmol) wird in einem sekurierten Rundkolben samt Rückflusskühler und Tropftrichter in 20 mL Dichlormethan gelöst. Triethylamin (0.625 mL, 4.5 mmol) wird hinzugegeben. α,ω-Bis[hexyl(3-(isocyanatomethyl)-3,5,5-trimethylcyclohexyl)carbamyl]-poly(dimethylsiloxan) (n=80) (9.81 g, 1.5 mmol) wird ebenfalls in 20 mL Dichlormethan gelöst und per Tropftrichter unter Rühren innerhalb von 1 h zugetropft. Nach 24 h Rühren bei RT wird mit je 2x 20 mL dest. Wasser und 1 x 20 mL ges. wässr. Natriumhydrogencarbonat-Lösung gewaschen. Die organischen Phasen werden vereinigt, über Natriumsulfat getrocknet und das Lösungsmittel am Rotationsverdampfer entfernt. Das Produkt wird abschließend im Hochvakuum getrocknet. Ausbeute: PDMS-80-Val: 78%.

1 H-NMR (600 MHz, CDCl3, 24 °C): δ [ppm]= 4.38 (2H, m, H-s), 4.01 (4H, m, H-g), 3.86 (2H, m, H-i), 3.71 (6H, m, H-w), 2.90 (4H, m, H-p), 2.10 (2H, m, H-t), 1.78-1.50 (8H, m, H-f/H-n), 1.31 (12H, m, H-c/H-d/H-e), 1.25-0.74 (38H, m, H-j/H-k/H-l/H-m/H-o/H-u/H-v), 0.46 (4H, m, H-b), 0.00 (444H, m, H-a); FT-IR (Diamant): ũ [cm-1]= 2962 (u R-CH3, Si-CH3, m-w), 2907 (u CH3, Ester, w) (u C-H, freie Aminosäure, m), 1729 (u C=O, Ester, v) (u C=O, Urethan, m-w), 1641 (u C=O, N-CO-N, v), 1562 (u C=O, N-CO-N, v), 1412 (δ C-H, Si-CH3, w), 1257 (δ C-H, Siloxan, s-m), 1009 (u Si-O-Si, Siloxan, s-m); MALDI-TOF-MS m/z: 3507 [M+Na]+ (für n=33), disubstituiert, (1 H-NMR: mit a bis p wie Bsp. 4)

### Beispiel 8: Synthese Tryptophan-terminiertes, kurzkettiges Polydimethylsiloxan (PDMS-30-IPDI-Trp) (23), n = 30

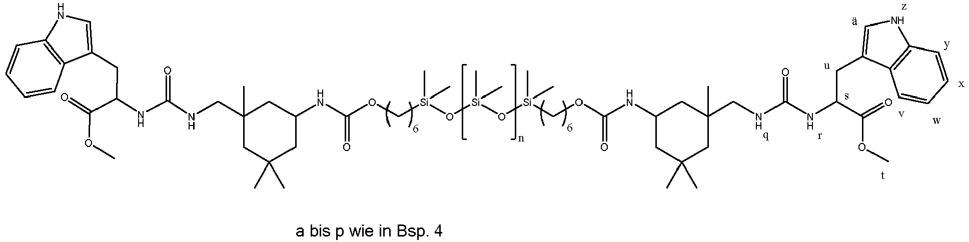

Tryptophan-methylester-hydrochlorid (1.15 g, 4.5 mmol) wird in einem sekurierten Rundkolben samt Rückflusskühler und Tropftrichter in einer Mischung aus 20 mL Dichlormethan und 20 mL Dimethylformamid gelöst. Es wird Triethylamin (0.625 mL, 4.5 mmol) hinzugegeben. α,ω-Bis[hexyl(3-(isocyanatomethyl)-3,5,5-trimethylcyclohexyl) carbamyl]-poly(dimethylsiloxan) (n=30) (4.26 g, 1.5 mmol) wird ebenfalls in einer Mischung aus 20 mL Dichlormethan und 20 mL Dimethylformamid gelöst und per Tropftrichter unter Rühren innerhalb von 1 h zugetropft. Nach 24 h Rühren bei RT wird mit je 2x 20 mL dest. Wasser und 1x 20 mL ges. wässr. Natriumhydrogencarbonat-Lösung gewaschen. Die organischen Phasen werden vereinigt, über Natriumsulfat getrocknet und das Lösungsmittel am Rotationsverdampfer entfernt. Das Rohprodukt wird in etwas Ethanol gelöst, in dest. Wasser wieder ausgefällt und zentrifugiert. Abschließend wird das Produkt im Hochvakuum getrocknet. Ausbeute: PDMS-30-Trp: 73%.

1 H-NMR (600 MHz, CDCl3, 24 °C): δ [ppm]= 7.46 (2H, m, H-v), 7.28 (4H, m, H-y), 7.04 (6H, m, H-w/H-x/H-ä), 4.72 (2H, m, H-s), 3.96 (4H, m, H-g), 3.63 (12H, m, H-i/H-t/H-u), 3.20 (4H, m, H-p), 1.67-1.39 (8H, m, H-f/H-n), 1.26 (12H, m, H-c/H-d/H-e), 1.17-0.59 (26H, m, H-j/H-k/H-l/H-m/H-o), 0.46 (4H, m, H-b), 0.00 (144H, m, H-a) FT-IR (Diamant): ũ [cm-1]= 2961 (u R-CH3, Si-CH3, m-w), 2600 (u C-H, freie Aminosäure, m), 2495 (u C-H, freie Aminosäure, m), 1667 (u C=O, N-CO-N, v) (u C=O, Urethan, m-w), 1439 (δ C-H, Si-CH3, w), 1258 (δ C-H, Siloxan, s-m), 1015 (u Si-O-Si, Siloxan, s-m); MALDI-TOF-MS m/z: 2126 [M+Na]+ (für n=12), disubstituiert

### Beispiel 9: Synthese Tryptophan-terminiertes, langkettiges Polydimethylsiloxan (PDMS-80-IPDI-Trp) (24), Struktur analog Beispiel 8, n = 80

Tryptophan-methylester-hydrochlorid (1.15 g, 4.5 mmol) wird in einem sekurierten Rundkolben mit Rückflusskühler und Tropftrichter in Mischung aus 20 mL Dichlormethan und 20 mL Dimethylformamid gelöst. Triethylamin (0.625 mL, 4.5 mmol) wird zugegeben. α,ω-Bis[hexyl(3-(isocyanatomethyl)-3,5,5-trimethylcyclohexyl)-carbamyl]-poly(dimethylsiloxan) (n=80) (9.81 g, 1.5 mmol) wird in Mischung aus 20 mL Dichlormethan und 20 mL Dimethylformamid gelöst und unter Rühren innerhalb von 1 h zugetropft. Nach 24 h Rühren bei RT wird mit je 2x 20 mL dest. Wasser und 1 x 20 mL ges. wässr. Natriumhydrogencarbonat-Lösung gewaschen. Die organischen Phasen werden vereinigt, über Natriumsulfat getrocknet und das Lösungsmittel am Rotationsverdampfer entfernt. Das Rohprodukt wird in etwas Ethanol gelöst, in dest. Wasser wieder ausgefällt und zentrifugiert. Das Produkt wird im Hochvakuum getrocknet. Ausbeute: PDMS-80-Trp: 75%.

1 H-NMR (600 MHz, CDCl3, 24 °C): δ [ppm]= 7.51 (2H, m, H-v), 7.33 (4H, m, H-y), 7.05 (6H, m, H-w/H-x/H-ä), 4.78 (2H, m, H-s), 4.01 (4H, m, H-g), 3.68 (12H, m, H-i/H-t/H-u), 3.24 (4H, m, H-p), 1.81-1.41 (8H, m, H-f/H-n), 1.31 (12H, m, H-c/H-d/H-e), 1.16-0.62 (26H, m, H-j/H-k/H-l/H-m/H-o), 0.51 (4H, m, H-b), 0.00 (366H, m, H-a); FT-IR (Diamant): ũ [cm-1]= 2959 (u R-CH3, Si-CH3, m-w), 2600 (u C-H, freie Aminosäure, m), 2495 (u C-H, freie Aminosäure, m), 1666 (u C=O, N-CO-N, v) (u C=O, Urethan, m-w), 1439 (δ C-H, Si-CH3, w), 1258 (δ C-H, Siloxan, s-m), 1087 (u Si-O-Si, Siloxan, s-m); MALDI-TOF-MS m/z: 3165 [M+Na]+ (für n=26), disubstituiert

### Beispiel 10: Synthese Histidin-terminiertes, langkettiges Polydimethylsiloxan (PDMS-80-IPDI-His)

Die Herstellung erfolgt entsprechen der Versuchsvorschrift des Beispiels 9
Unter Verwendung von Histidin-methylester-hydrochlorid (1.15 g, 4.5 mmol).

### Beispiel 11: Synthese Histidin-terminiertes, kurzkettiges Polydimethylsiloxan (PDMS-30-IPDI-His)

Die Herstellung erfolgt entsprechen der Versuchsvorschrift des Beispiels 8 unter Verwendung von Histidin-methylester-hydrochlorid (1.15 g, 4.5 mmol).

### Beispiel 12: Synthese von α,ω-Bis[hexyl(6-isocyanatohexyl)carbamyl]poly-(dimethylsiloxan)

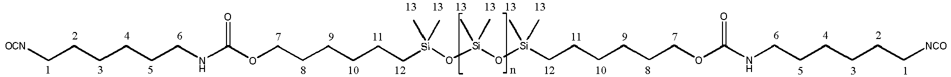

2,00 g (11.89 mmol) 1,6-Hexamethylendiisocyanat, gelöst in 20 mL Ethylacetat, werden in einer ausgeheizten, mit Argon gespülten und sekurierten Apparatur mit Rückflusskühler und Tropftrichter vorgelegt. Man erhitzt unter Rühren auf 75 °C und gibt anschließend 0,1 Gew. % (16 mg) Triethylamin hinzu. Per Tropftrichter werden anschließend 14 g (5.8 mmol) α,ω-Bis(hydroxyhexyl)-poly(dimethylsiloxan), gelöst in 30 mL Ethylacetat, zur Diisocyanat-Komponente langsam zugegeben (Tropfzeit 2 Std.). Dabei wird stets gerührt. Anschließend wird für weitere 16 Std. bei 75 °C gerührt. Ausbeute: 15,90 g

¹H-NMR: (300 MHz, CDCl₃) δ = 4.01 (m, 4H, 7), 3.27 (q, 4H, 1), 3.13 (m, 4H, 6), 1.68-1.23 (m, 32H, 2, 3, 4, 5, 8, 9, 10, 11), 0.50 (m, 4H, 12), 0.04 (m, 180H, 13) ppm. FT-IR (Diamant): *ṽ* = 2964 v(C-H), 2267 v(NCO), 1708 v(C=O)_{Urethan}, 1523 δ(N-H), 1410 δₐₛ(C-H), 1254 δ_{sym}(C-H), 1011 v(Si-O-C), 853 + 789 v(SI-C) cm⁻¹.

### Beispiel 13: Synthese von α,ω-Bis[hexyl(6-(valin-methylester)ureylhexyl)-carbamyl]-poly(dimethylsiloxan)

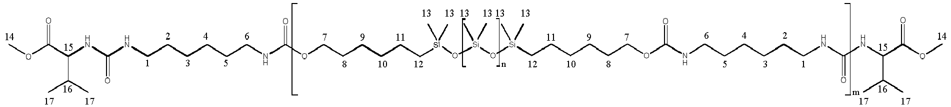

168 mg (1 mmol) Valin-methylester-hydrochlorid werden in 15 mL Dichlormethan gelöst und in einer ausgeheizten, mit Argon gespülten und sekurierten Apparatur mit Rückflusskühler und Tropftrichter vorgelegt. Es werden 101,2 mg (0,139 mL, 1 mmol) Triethylamin zugegeben. 1,033 g (0,378 mmol) α,ω-Bis[hexyl(6-isocyanatohexyl)carbamyl]-poly(dimethylsiloxan) werden in 20 mL Dichlormethan gelöst und per Tropftrichter unter Rühren innerhalb von einer Stunde zur Aminosäure zugegeben. Nach zwei Stunden Rühren bei Raumtemperatur wird die organische Phase 3x mit je 15 mL dH₂O, 1 x mit 15 mL ges. wässr. NaHCO₃-Lsg. und 1 x mit 15 mL ges. NaCl-Lsg. gewaschen. Die organischen Phasen werden vereinigt, über Na₂SO₄ getrocknet und das Lösemittel am Rotationsverdampfer entfernt. Anschließend wird im Hochvakuum getrocknet. Ausbeute: 0,92 g (79 %)

¹H-NMR: (300 MHz, CDCl₃) δ = 4.36 (m, 0.85H, 15), 4.01 (m, 4H, 7), 3.71 (m, 2.71 H, 14) 3.31-3.30 (m, 8H, 1, 6), 2.10 (m, 0.89H, 16), 1.68-1.23 (m, 32H, 2, 3, 4, 5, 8, 9, 10, 11), 0.98-0.82 (m, 5.52H, 17), 0.50 (m, 4H, 12), 0.04 (m, 180H, 13) ppm.

FT-IR (Diamant): *ṽ* = 2962 v(C-H), 1720 v(C=O)_{Urethan}, 1658 v(C=O)_{Harnstoff}, 1563 δ(N-H), 1404 δₐₛ(C-H), 1257 δ_{sym}(C-H), 1011 v(Si-O-C), 863 + 789 v(SI-C) cm⁻¹.

### Beispiel 14: Synthese von Isocyanat-funktionalisierten Aminosäure-Derivaten (A-IPDI, IXa*, IXb*)

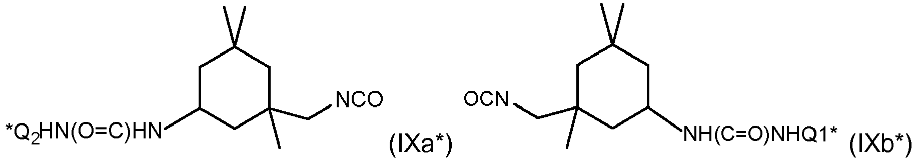

sowie ggf. der allgemeinen Formeln IXc* und/oder IXd* oder Mischungen davon.

### Beispiel 15: Synthese eines Isocyanat-funktionalisierten Aminosäure-Derivat (A-IPDI), Valin-methylester-IPDI

Isophorondiisocyanat (4.72 mL, 22.5 mmol) wird in einer zuvor sekurierten Apparatur bei RT vorgelegt. Es werden 1-2 Tropfen Triethylamin unter Argongegenstrom hinzugefügt. Über einen Tropftrichter wird Valin-methylester-hydrochlorid (22.5 mmol) in 10 mL Dichlormethan und 10 mL Dimtehylformamid gelöst, innerhalb von 2 h tropfenweise zu der Reaktionsmischung gegeben. Nach 24 h Rühren bei RT werden die Lösungsmittel am Rotationsverdampfer entfernt und das Produkt abschließend im Hochvakuum getrocknet. Erhalten wird der Valin-methylester-IPDI.

### Beispiel 16: Synthese eines Isocyanat-funktionalisierten Aminosäure-Derivat (A-IPDI), Histidin-methylester-IPDI

Isophorondiisocyanat (4.72 mL, 22.5 mmol) wird in einer zuvor sekurierten Apparatur bei RT vorgelegt. Es werden 1-2 Tropfen Triethylamin unter Argongegenstrom hinzugefügt. Über einen Tropftrichter wird Histidin-methylester-hydrochlorid (22.5 mmol) in 10 mL Dichlormethan und 10 mL Dimethylformamid gelöst, innerhalb von 2 h tropfenweise zu der Reaktionsmischung gegeben. Nach 24 h Rühren bei RT werden die Lösungsmittel am Rotationsverdampfer entfernt und das Produkt abschließend im Hochvakuum getrocknet. Erhalten wird der Histidin-methylester-IPDI.

### Anwendungstest - Kämmkraft

Für die anwendungstechnische Beurteilung der Konditionierung von Haar wurden die nachstehend genannten erfindungsgemäßen Verbindungen sowie das kommerziell erhältliche Produkt ABIL^{®} Quat 3272 in einer einfachen kosmetischen Haarspülungs-Formulierung eingesetzt und mittels Kämmkraftmessungen untersucht.
Die Formulierungsbestandteile sind in den Zusammensetzungen in Form der allgemein anerkannten INCI-Nomenklatur unter Verwendung der englischen Begriffe benannt. Alle Konzentrationen in den Anwendungsbeispielen sind in Gewichtsprozent angegeben.

Für die anwendungstechnische Beurteilung wurden Haartressen (braune kaukasische Flachtressen, unbehandelt, Firma Kerling Deutschland) durch eine Dauerwellbehandlung standardisiert vorgeschädigt ("Universal-Dauerwelle" mit "Schaumfixierung-Konzentrat", Basler). 4 g Dauerwellenmittel / g Haar, Einwirkzeit 15 min, Ausspülzeit 2 min unter fließendem Wasser (T=38 °C). Dann 4 g Neutralisierlösung (1 Teil Schaumfixierer + 3 Teile Wasser)/g Haar, Einwirkzeit 10 min, Ausspülzeit 2 min unter fließendem Wasser. Dazu wurden friseurübliche Produkte verwendet.

Die Kämmkraftmessung erfolgten mit dem Gerät Diastron MTT 175 (Geschwindigkeit: 2000 mm/min) bei standardisierten Bedingungen (22 °C, 50% relative Luftfeuchte).
Die Vorbehandlung der Haare erfolgte durch ein Shampoo, welches keine Konditioniermittel enthält.

Standardisierte Behandlung und Messung der Haartressen: Die Haartressen wurden bei kontrollierten Bedingungen (22 °C, 50% relative Luftfeuchte) für mindestens 12 h gelagert. Die Haartressen wurden dann für 1 min in eine hoch-verdünnte Puffer (Na-Citrat, pH-6)-Lösung getaucht. Die Haare wurden daraufhin per Hand vorgekämmt, bis keine spürbare Verbesserung der Kämmbarkeit erzielt werden kann. Das Haar wird so lang aufgehängt bis die Restfeuchte am Haar ca. 60% (+-5%) betrug (2 g Haar + 1,2 g Restfeuchte). Die Haartresse wurde dann am Messgerät fixiert und die erste Kämmkraftmessung gestartet. The Messung wurde 10-Mal wiederholt. Vor jeder Messung wurde die Haartresse mittels 2-maligem Besprühen aus einer Sprühflasche mit der Citrat-Puffer-Lösung befeuchtet. Der Mittelwert der Kämmkraftmessungen wurde über die MTT175-software bestimmt. Aus statistischen Gründen wurden für jede Formulierung 4 Haartressen gemessen. Die gleiche Vorgehensweise und Messung wurde vor und nach Behandlung der Haare mit der Haarspülung durchgeführt.

Für die standardisierte Behandlung der Haartressen mit der Haarspülung wurden die vorgeschädigten Haartressen wie folgt mit der nachstehend beschriebenen konditionierenden Spülung behandelt:
Die Haartressen wurden unter fließendem, 38 °C warmem Wasser benetzt. Das überschüssige Wasser wurde leicht von Hand ausgedrückt, dann wurde die Spülung aufgebracht und sanft für 1 min im Haar eingearbeitet (0,5 g/Haartresse (2 g)). Nach einer Verweilzeit von 5 min wurde das Haar für 1 min gespült.

Für die Auswertung der Messungen wurde der Unterschied der nötigen Kämmkräfte vor Behandlung im Vergleich zu nach Behandlung mit der Haarspülung berechnet.

Die erfindungsgemäßen Siloxane werden wie vorstehend erläutert in einer Haarspülung der nachstehend genannten Rezeptur auf die Haare aufgebracht und eine Minute aus den behandelten Haaren ausgespült. Gemessen wird anschließend die Verminderung der aufzubringenden Kraft zum Kämmen einer definierten Haarprobe. Die erfindungsgemäßen Siloxane vermindern die Kraft um etwa 51 bis 56 %, wobei das Siloxan mit n = 80, IPDI + Methyltryptophan die besten Ergebnisse aufzeigt. Die Matrixformulierung (CTAC) umfasst 0.5 Gew.-%, Ceterareth-25, 5.0 Gew.-% Cetyl-Alkohol, 1.0 Gew-% Cetrimonium-Chlorid, ad 100 % Wasser, der pH-Wert beträgt etwa 4.3. Die erfindungsgemäßen Formulierungen umfassen in 100 Gew.-% zudem 0.5 Gew.-% der erfindungsgemäß mit Aminosäuren funktionalisierten Siloxane, der pH-Wert beträgt etwa 4.3. Ebenso zeigen das Siloxan mit n = 80 + IPDI + Histidin sowie das Siloxan mit n = 30, + IPDI + Methyltryptophan eine Reduktion der Kämmkraft um etwa 51 %. Der pH-Wert kann mit Zitronensäure reguliert werden. Als Vergleich wurde in der Matrixformulierung 1,0 Gew% ABIL® Quat 3272 (Silikonquat der Firma Evonik Industries, 50%ig in Propylenglycol) eingesetzt.
In der folgenden Tabelle 1 werden die Ergebnisse der Kämmkraftmessungen als Erniedrigung der aufzuwendenden Kämmkraft für die vorstehend Matrixformulierung mit jeweils 0,5 Gew.-% der erfindungsgemäßen funktionalisierten PDMS der allgemeinen Formel I dargestellt.

**Tabelle 1: Kämmkraftreduktion**

| | Kämmkrafterniedrigung (Vergleich vorher zu nach einer Behandlung mit der Haarspülung) |
|---|---|
| CTAC (Matrixfomulierung) | 35,3 % |
| CTAC + ABIL QUAT 3272 | 49 % |
| CTAC + (PDMS-80-Val) (21) | 51,3 % |
| CTAC + PDMS-80-IPDI-His) | 54,1 % |
| CTAC + (PDMS-30-IPDI-Trp) (23) | 51,7 % |
| CTAC + PDMS-80-IPDI-Trp) (24) | 56,7 % |

## Patentansprüche

1. Siloxan-Polymer der allgemeinen Formel I umfassend einen zentralen Polysiloxan-Polymerblock B,
(i) der mit organofunktionellen Resten substituiert ist,
(ii) der Polymerblock B weist lineare und/oder verzweigte Strukturen mit mindestens zwei difunktionellen Siloxan-Einheiten auf,
(iii) der Polymerblock B weist an mindestens zwei terminalen Silizium-Atomen oder mindestens einem terminalen und mindestens einem seitenkettenständigen Silizium-Atom der Siloxan-Einheiten des Polymerblocks B die organofunktionellen Reste -Q1 und -Q2 auf, wobei die Reste gleich oder verschieden sind,
Q2-B-Q1 (I)
wobei -Q1 der allgemeinen Formel IIa und -Q2 der Formel IIb entspricht,
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- mit A gleich -NH-, -O- oder -S- und D gleich -NH- jeweils unabhängig in Formeln IIa und IIb,
- mit A' gleich -NH- und D' gleich -NH- -O- oder -S- jeweils unabhängig in Formeln IIa und IIb, wobei jeder Rest Q1 und Q2 der Formeln IIa oder IIb mindestens eine bivalente Harnstoff-Gruppe und eine weitere bivalente Harnstoff- oder Carbamat-Gruppe aufweist,
- mit Q1' und Q2' jeweils unabhängig umfassend einen bivalenten Kohlenwasserstoff-Rest mit 6 bis 200 C-Atomen optional umfassend mindestens ein Heteroatom ausgewählt aus O, N und S, einen bivalenten Rest umfassend Aryl-, Arylalkyl-Gruppen optional umfassend mindestens ein Heteroatom O, N oder S oder Alkyl-, Aryl- oder Alkyl- und Aryl-Gruppen enthaltende Polyether-Reste,
- mit Q1" und Q2" jeweils unabhängig umfassend einen bivalenten linearen, verzweigten und/oder cyclischen Alkyl-Rest mit 4 bis 200 C-Atomen oder einen bivalenten Rest umfassend einen Aryl- und/oder Arylalkyl-Rest mit 6 bis 200 C-Atomen, und
- mit -D'-Q1* und -D'-Q2* mit D', wie vorstehend definiert, und wobei -D'-Q1* und -D'-Q2* jeweils unabhängig Reste umfassen, die aus einer Aminosäure, einem Aminosäure-Derivat oder deren Salzen abgeleitet sind.

2. Siloxan-Polymer nach Anspruch 1,
**dadurch gekennzeichnet,**
**dass** in dem Siloxan-Polymer der allgemeinen Formeln I, IIa und IIb -D'-Q1* und -D'-Q2* jeweils unabhängig Reste bilden, die aus einer Aminosäure, einem Aminosäure-Derivat oder deren Salzen abgeleitet sind und D' gleich -NH jeweils unabhängig in -D'-Q1* und -D'-Q2* in alpha-Stellung zu einer Ester- oder einer Carboxy-Gruppe der Aminosäure, des Aminosäure-Derivates oder Salzes steht, wobei der Ester Alkyl-Ester mit 1 bis 25 C-Atome oder Aryl-Ester umfassen.

3. Siloxan-Polymer nach Anspruch 1 oder 2,
**dadurch gekennzeichnet,**
**dass** in dem Siloxan-Polymer der allgemeinen Formel I der Polymerblock B der allgemeinen Formel IIIa oder IIIb entspricht, mit B gleich 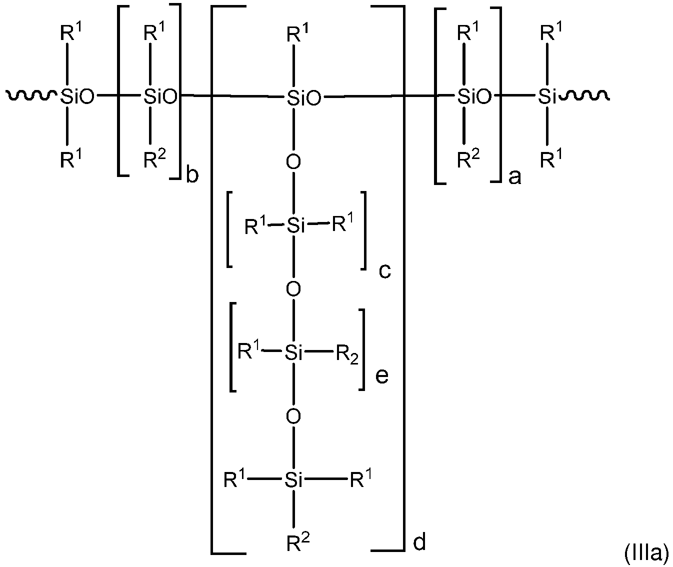 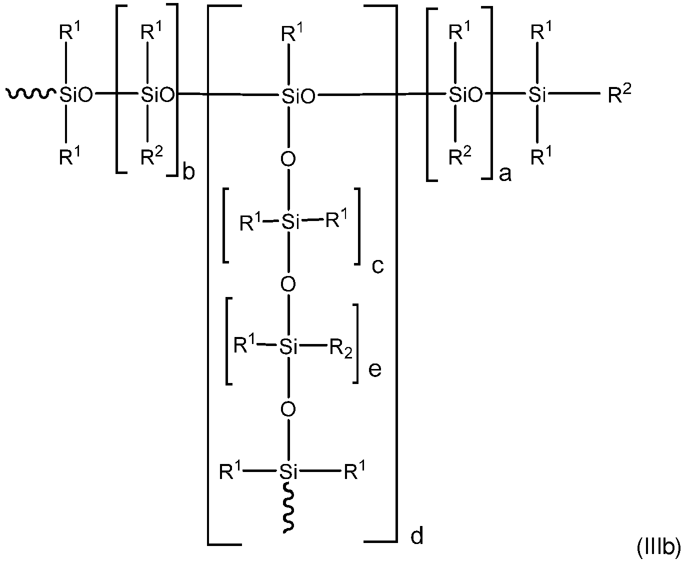 mit a, b, c, d und e in Formeln IIIa und IIIb jeweils unabhängig eine ganze Zahl mit a von 1 bis 200, mit b von 0 bis 200, mit c von 0 bis 200, mit d von 0 bis 200, mit e von 0 bis 200 und mit R¹ in Formeln IIIa oder IIIb jeweils unabhängig gleich oder verschieden, wobei R¹ umfasst Alkyl-Reste mit 1 bis 22 C-Atomen, vorzugsweise 1 bis 4 C-Atomen oder Phenyl-Reste, mit R² in Formeln IIIa oder IIIb gleich Alkyl-Rest mit 1 bis 22 C-Atomen, vorzugsweise 1 bis 4 C-Atomen, ein Alkyl-Rest mit mindestens einem Heteroatom ausgewählt aus N, O S oder Phenyl-Rest.

4. Siloxan-Polymer nach Anspruch 3,
**dadurch gekennzeichnet,**
**dass** b, c, d und e gleich 0 sind und a gleich 20 bis 100 ist, insbesondere 30 oder 80 mit einer Schwankungsbreite von plus/minus 5.

5. Siloxan-Polymer nach einem der Ansprüche 3 oder 4,
**dadurch gekennzeichnet,**
**dass** R¹ und R² ausgewählt sind aus Alkyl-Gruppen mit 1, 2, 3 oder 4 C-Atomen, insbesondere aus Methyl-Gruppen oder mindestens ein R² eine Aminoalkyl-Gruppe oder ein quartäres Alkylamin ist.

6. Siloxan-Polymer nach einem der Ansprüche 1 bis 5,
**dadurch gekennzeichnet,**
**dass** die Reste -Q1 und -Q2 in der allgemeinen Formel I unabhängig ausgewählt sind aus
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
a) mit A gleich -O-, D gleich -NH-, A' gleich -NH- und D' gleich -NH-,
b) mit A gleich -NH-, D gleich -NH-, A' gleich -NH- und D' gleich -NH-,
c) mit A gleich -S-, D gleich -NH-, mit A' gleich -NH- und D' gleich -NH-,
d) mit A gleich -O-, D gleich -NH-, A' gleich -NH- und D' gleich -O-,
e) mit A gleich -NH-, D gleich -NH-, A' gleich -NH- und D' gleich -O-,
f) mit A gleich -S-, D gleich -NH-, A' gleich -NH- und D' gleich -O-,
g) mit A gleich -O-, D gleich -NH-, A' gleich -NH- und D' gleich -S-,
h) mit A gleich -NH-, D gleich -NH-, A' gleich -NH- und D' gleich -S- oder
i) mit A gleich -S-, D gleich -NH-, A' gleich -NH- und D' gleich -S-.

7. Siloxan-Polymer nach einem der Ansprüche 1 bis 6,
**dadurch gekennzeichnet,**
**dass** in den Resten -Q1 und -Q2 der Formel I die Reste -D'-Q1 * und -D'-Q2* jeweils unabhängig abgeleitet sind aus Aminosäuren, Aminosäure-Derivaten oder deren Salzen, umfassend
- unpolare Aminosäuren umfassend Alanin, Valin, Methionin, Leucin, Isoleucin, Prolin, Tryptophan, Phenylalanin,
- basische Aminosäuren umfassend Lysin, Arginin, Histidin,
- polare und neutrale Aminosäuren Tyrosin, Threonin, Glutamin, Glycin, Serin, Cystein, Asparagin und/oder
- saure Aminosäuren ausgewählt aus Glutaminsäure und Asparaginsäure,
sowie deren Mono-, Dicarbonsäureester, Amide mit primären Amino-Gruppen der Aminosäuren, Amide der Carbonsäure-Gruppen der Aminosäuren und/oder Ester mit den primären Hydroxy-Gruppen, Thioester der HS-Gruppe oder Amide mit sekundären Amino-Gruppen der Aminosäuren.

8. Siloxan-Polymer nach einem der Ansprüche 1 bis 7,
**dadurch gekennzeichnet,**
**dass** in den Resten -Q1 und -Q2 der Formel I
A gleich -O-, D gleich -NH-, A' gleich -NH- und D' gleich -NH-, -O- oder -S- sind, und die Reste -D'-Q1* und -D'-Q2* jeweils unabhängig abgeleitet sind aus Aminosäuren, Aminosäure-Derivaten oder deren Salzen, insbesondere umfassend unpolare Aminosäuren umfassend Alanin, Valin, Methionin, Leucin, Isoleucin, Prolin, Tryptophan, Phenylalanin, basische Aminosäuren umfassend Lysin, Arginin, Histidin, polare und neutrale Aminosäuren umfassend Tyrosin, Threonin, Glutamin, Glycin, Serin, Cystein, Asparagin und/oder die sauren Aminosäuren ausgewählt aus Glutaminsäure und Asparaginsäure, sowie deren Mono-, Dicarbonsäureester, Amide mit primären Amino-Gruppen der Aminosäuren, Amide der Carbonsäure-Gruppen der Aminosäuren und/oder Ester mit den primären Hydroxy-Gruppen oder Thioester der HS-Gruppe sowie deren Salze.

9. Siloxan-Polymer nach einem der Ansprüche 1 bis 8,
**dadurch gekennzeichnet,**
**dass** in den Resten -Q1 und -Q2 der Formel I die bivalenten Reste -Q1"- und -Q2"- unabhängig ausgewählt sind aus bivalenten, linearen, verzweigten oder cyclischen Alkylen-Resten mit 4 bis 25 C-Atomen, insbesondere mit 6, 7, 8, 9, 10, 11, 12, 13 oder 14 C-Atomen, vorzugsweise Hexylen (-CH₂)₆, Heptylen, bivalentes 2,4-Toluolyl, Diphenylmethan, polymeres Diphenylmethan, 3,5,5-Trimethyl-1-methylen-3-ethylen-cyclohexan oder 4,4'-Dicyclohexylen.

10. Siloxan-Polymer nach einem der Ansprüche 1 bis 9,
**dadurch gekennzeichnet,**
**dass** in den Resten -Q1 und -Q2 der Formel I die bivalenten Reste -Q1'- und -Q2'-ausgewählt sind aus Alkylen-Resten mit 3 bis 22 C-Atomen optional mit mindestens einem Heteroatom umfassend N, O oder S, insbesondere -(CH₂)ₙ- mit n von 3 bis 22 C-Atomen, vorzugsweise Hexylen (-CH₂)₆-, Heptylen (-CH₂)₇-, Octylen (-CH₂)₈-, Nonylen (-CH₂)₉-, Decylen (-CH₂)₁₀-, Undecylen (-CH₂)₁₁-, Dodecylen (-CH₂)₁₂-, oder mit Heteroatomen, Alkylen-(C=O)-, Alkylen-O(CO)-Alkylen, Alkylen-(CO)O-Alkylen, Alkylen-NH(CO)-Alkylen, Alkylen-(CO)NH-Alkylen, Alkylen-NH(CO)NH-Alkylen, Alkylen-NH(CO)O-Alkylen, oder aus Alkyl-, Aryl- oder Alkyl- und Aryl-Gruppen enthaltenden Polyether-Resten den Formeln IVa oder IVb mit Q1' und Q2' jeweils unabhängig gleich
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb),
mit T = bivalenter Kohlenwasserstoffrest mit 2 bis 4 C-Atomen, mit x = 0 bis 200,
y = 0 bis 200, wobei x und y ganze Zahlen sind, mit der Maßgabe, dass x oder y mindestens 1 ist, mit R^{#} Wasserstoff oder Methyl, R" lineares oder verzweigtes Alkylen, vorzugsweise mit T = -(CH₂)₂- oder -(CH₂)₃-.

11. Siloxan-Polymer nach einem der Ansprüche 1 bis 10,
**dadurch gekennzeichnet,**
**dass** in den Resten -Q1 und -Q2 der Formel (I) mindestens einer der bivalenten Reste -Q1 "- und -Q2"- unabhängig ein bivalenter Cyclohexan enthaltender-Rest ausgewählt aus den Formeln Va und Vb 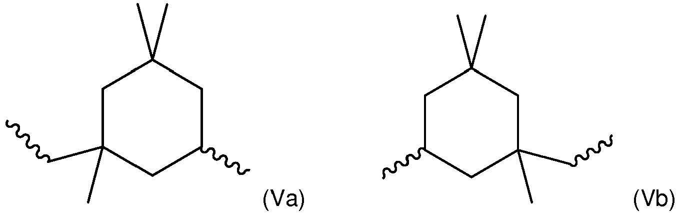 ist, insbesondere ist Q2"- ein bivalenter Cyclohexan enthaltender-Rest der Formel Va und -Q1" der Formel Vb.

12. Siloxan-Polymer nach einem der Ansprüche 1 oder 2 und 6 bis 11, **dadurch gekennzeichnet,**
**dass** Siloxan der allgemeinen Formel I dem Siloxan-Polymer der allgemeinen Formel XI entspricht mit
Mₐ₁M^{A}ₐ₂M^{B}ₐ₃D_{b1}D^{A}_{b2}D^{B}_{b3}T_{c1}T^{A}_{c2}T^{B}_{c3}Q_{d1} (XI)
mit M = [R¹⁶₃SiO_{1/2}], M^{A} = [R¹⁷R¹⁶₂SiO_{1/2}], M^{B} = [R¹⁸R¹⁶₂SiO_{1/2}],
D = [R¹6₂SiO_{2/2}], D^{A} = [R¹⁷₁R¹⁶₁SiO_{2/2}], D^{B} = [R¹⁸1R¹⁶1SiO_{2/2}],
T = [R¹⁶SiO_{3/2}], T^{A} = [R¹⁷SiO_{3/2}], T^{B} = [R¹⁸SiO_{3/2}], Q = [SiO_{4/2}],
wobei gilt
R¹⁶ unabhängig voneinander gleiche oder verschiedene lineare oder verzweigte, gesättigte oder ungesättigte Kohlenwasserstoffreste mit 1 bis 30 Kohlenstoffatomen oder auch aromatische Kohlenwasserstoffreste mit 6 bis 30 Kohlenstoffatomen bevorzugt Methyl oder Phenyl, insbesondere Methyl,
mit R¹⁷ ist jeweils unabhängig -Q1 oder -Q2,
R¹⁸ unabhängig voneinander gleiche oder verschiedene lineare oder verzweigte, gesättigte oder olefinisch ungesättigte Kohlenwasserstoffreste mit 8 bis 30 Kohlenstoffatomen, aromatischer Kohlenwasserstoffrest mit 6 bis 40 Kohlenstoffatomen, Alkylarylrest mit 7 bis 40 Kohlenstoffatomen, ein durch ein oder mehrere Heteroatome, wie Sauerstoff, NH, NR' mit R' gleich einem gegebenenfalls Doppelbindungen enthaltenden C1 bis C30-Alkylrest unterbrochenen linearen oder verzweigten gegebenenfalls Doppelbindungen enthaltenden aliphatischen Kohlenwasserstoffrest mit 2 bis 30 Kohlenstoffatomen, ein durch eine oder mehrere Funktionalitäten, ausgewählt aus der Gruppe -OH, -O-C(O)-, -(O)C-O-, -NH-C(O)-, -(O)C-NH, - (CH₃)N-C (O)-, -(O)C-N(CH₃)-, -S(O₂)-O-, -O-S(O₂)-, -S(O₂)-NH-, -NH-S(O₂)-, -S(O₂)-N(CH₃)-, -N(CH₃)-S(O₂)-, unterbrochenen linearen oder verzweigten gegebenenfalls Doppelbindungen enthaltenden aliphatischen Kohlenwasserstoffrest mit 2 bis 30 Kohlenstoffatomen,
ein endständig OH, OR', NH², N(H)R', N(R')₂ mit R' gleich einem gegebenenfalls Doppelbindungen enthaltenden C1- bis C30-Alkylrest, funktionalisierten linearen oder verzweigten gegebenenfalls Doppelbindungen enthaltenden aliphatischen oder cycloaliphatischen Kohlenwasserstoffrest mit 1 bis 30 Kohlenstoffatomen oder einen blockweise oder statistisch aufgebauten Polyether gemäß -(R⁵-O)ₙ-R⁶, wobei R⁵ ein 2 bis 4 Kohlenstoffatome enthaltender linearer oder verzweigter Kohlenwasserstoffrest, n 1 bis 100, vorzugsweise 2 bis 60, ist und R⁶ Wasserstoff, einen linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einen aromatischen Kohlenwasserstoffrest mit 6 bis 40 Kohlenstoffatomen, einen Alkylarylrest mit 7 bis 40 Kohlenstoffatomen, oder ein Rest -C(O)-R⁷ mit R⁷ gleich einem linearen oder verzweigten gegebenenfalls Doppelbindungen enthaltenden aliphatischen Kohlenwasserstoffrest mit 1 bis 30 Kohlenstoffatomen, einen gegebenenfalls Doppelbindungen enthaltenden cycloaliphatischen Kohlenwasserstoffrest mit 5 bis 40 Kohlenstoffatomen, einem aromatischen Kohlenwasserstoffrest mit 6 bis 40 Kohlenstoffatomen, einem Alkylarylrest mit 7 bis 40 Kohlenstoffatomen mit den Indices
a1 = 0-200, bevorzugt 1-60, insbesondere 0,
a2 = 0-30, bevorzugt 1-20, insbesondere 2-10,
a3= 0-30, bevorzugt 1-20,
b1 = 2 bis 5000, bevorzugt 10 bis 1000,
b2 = 0 bis 100, bevorzugt 1 bis 30, insbesondere 1 bis10 oder 0,
b3 = 0 bis 100, bevorzugt 0 bis 30, insbesondere 1 bis 10 oder 0,
c1 = 0 bis 30, bevorzugt 1 bis 30,
c2 = 0 bis 30, bevorzugt 0 bis 5, insbesondere 0,
c3 = 0 bis 30, bevorzugt 0 bis 5, insbesondere 0,
d1 = 0 bis 30, bevorzugt 0 bis 5, vorzugsweise 0
mit der Maßgabe, dass mindestens einer der Indices ausgewählt aus a2 und a3 ungleich 0 ist, vorzugsweise ist a2 eine ganze Zahl zwischen 2 und 10.

13. Siloxan-Polymer nach einem der Ansprüche 1 bis 12,
**dadurch gekennzeichnet,**
**dass** es ausgewählt ist aus Siloxan-Polymeren der Formeln la und Ib oder Mischungen dieser 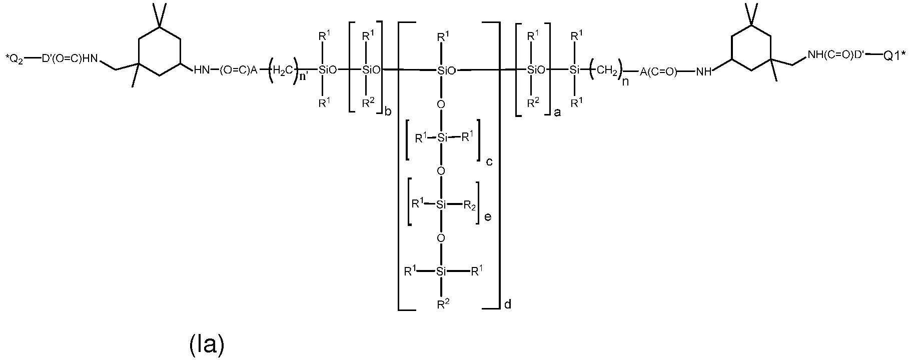 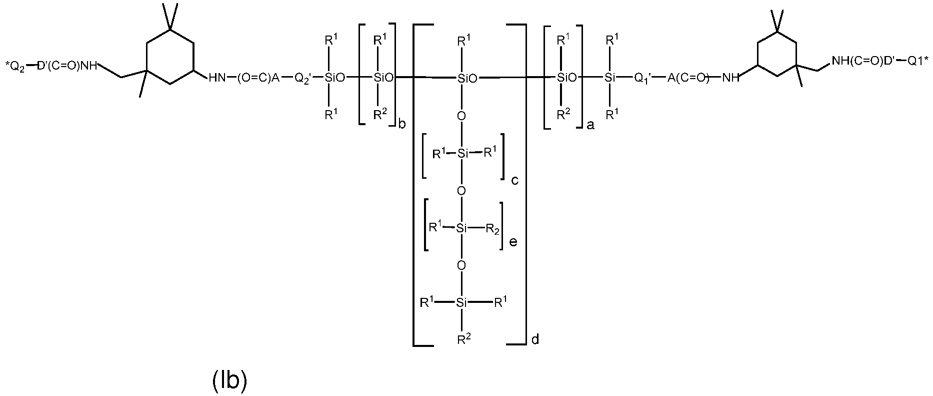 mit n oder n' jeweils unabhängig ausgewählt aus einer ganzen Zahl von 3 bis 22, mit a von 1 bis 200, mit b von 0 bis 200, mit c von 0 bis 200, mit d von 0 bis 200, mit e von 0 bis 200 und mit R¹ in Formeln la und Ib jeweils unabhängig gleich oder verschieden, wobei R¹ umfasst Alkyl-Reste mit 1 bis 4 C-Atomen oder Phenyl-Reste, mit R² Alkyl-Rest mit 1 bis 22 C-Atomen, vorzugsweise 1 bis 4 C-Atomen, ein Alkyl-Rest mit mindestens einem Heteroatom ausgewählt aus N, O S oder Phenyl-Rest und mit -D'-Q1 * und -D'-Q2* jeweils unabhängig abgeleitet aus Aminosäuren, Aminosäure-Derivaten oder deren Salzen, und in Formel Ib mit Q1' und Q2' jeweils unabhängig gleich Alkylen mit 2 bis 40 C-Atomen oder einem Polyether der Formeln IVa oder IVb
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb)
ist, mit T = bivalenter Kohlenwasserstoffrest mit 2 bis 4 C-Atomen, mit x = 0 bis 200,
y = 0 bis 200, wobei x und y ganze Zahlen sind mit der Maßgabe, dass x oder y mindestens 1 ist, mit R^{#} Wasserstoff oder Methyl, R" Wasserstoff oder lineares oder verzweigtes Alkylen, insbesondere mit T = -(CH₂)₂- oder -(CH₂)₃-.

14. Siloxan-Polymer nach einem der Ansprüche 1 bis 13,
**dadurch gekennzeichnet,**
**dass** sie ausgewählt sind aus Siloxan-Polymeren der Formel Ia* und Ib* 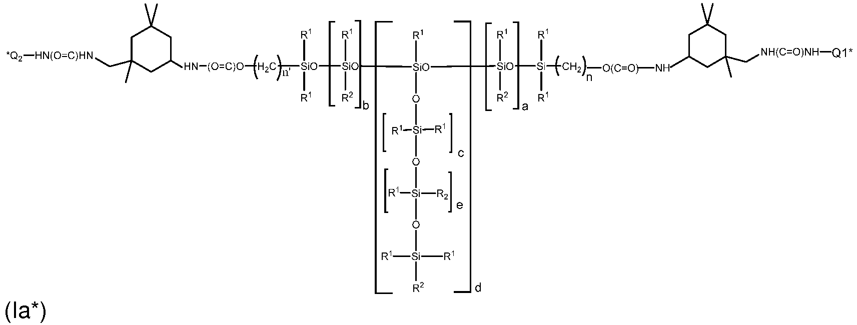 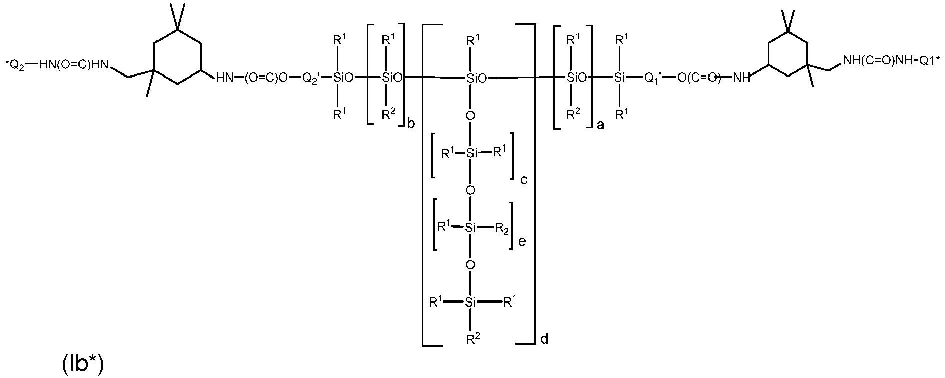
mit n oder n' jeweils unabhängig ausgewählt aus einer ganzen Zahl von 3 bis 22, mit a von 1 bis 200, mit b von 0 bis 200, mit c von 0 bis 200, mit d von 0 bis 200, mit e von 0 bis 200 und
mit R¹ in Formeln Ia* und Ib* jeweils unabhängig gleich oder verschieden, wobei R¹ umfasst Alkyl-Reste mit 1 bis 4 C-Atomen oder Phenyl-Reste, mit R² Alkyl-Rest mit 1 bis 22 C-Atomen, vorzugsweise 1 bis 4 C-Atomen, ein Alkyl-Rest mit mindestens einem Heteroatom umfassend N, O, S oder Phenyl-Rest und mit Q1* und Q2* jeweils unabhängig ausgewählt aus Aminosäuren, Aminosäure-Derivaten oder deren Salzen, wobei das Fragment-NH-Q1* und -NH-Q2* durch Reaktion der sekundären alpha-Amino-Gruppen der Aminosäuren, deren Derivate oder der Salze mit dem Isocyanat eine Harnstoff-Gruppe bildet, und wobei die Aminosäuren umfassend unpolare Aminosäuren umfassend Alanin, Valin, Methionin, Leucin, Isoleucin, Prolin, Tryptophan, Phenylalanin, basische Aminosäuren umfassend Lysin, Arginin, Histidin, polare und neutrale Aminosäuren umfassend Tyrosin, Threonin, Glutamin, Glycin, Serin, Cystein, Asparagin und/oder die sauren Aminosäuren ausgewählt aus Glutaminsäure und Asparaginsäure, sowie deren Mono-, Dicarbonsäureester, Amide mit primären Amino-Gruppen der Aminosäuren, Amide der Carbonsäure-Gruppen der Aminosäuren und/oder Ester mit den primären Hydroxy-Gruppen oder Thioester der HS-Gruppe oder deren Salzen, und in Formel Ib* mit Q1' und Q2' jeweils unabhängig gleich
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb),
mit T = bivalenter Kohlenwasserstoffrest mit 2 bis 4 C-Atomen, mit x = 0 bis 200, y = 0 bis 200, wobei x und y ganze Zahlen sind mit der Maßgabe, dass x oder y mindestens 1 ist, mit R^{#} Wasserstoff oder Methyl, R" Wasserstoff oder lineares oder verzweigtes Alkylen, insbesondere Ethylen, insbesondere mit T = -(CH₂)₂- oder - (CH₂)₃-.

15. Verfahren zur Herstellung eines Siloxan-Polymers sowie von Zusammensetzungen enthaltend diese Siloxan-Polymere oder Mischungen der Siloxan-Polymere mit einem zentralen Polysiloxan-Polymerblock B, indem
a) ein Polysiloxan-Diisocyanat der Formel VII,
OCN-Q2"-D-(O=C)-A-Q2'-B-Q1'-A-(C=O)-D-Q1 "-NCO (VII)
mit mindestens einer Aminosäure, einem Aminosäure-Derivat, deren Salz oder Mischungen dieser umgesetzt wird und ein Siloxan-Polymer der allgemeinen Formel I
Q2-B-Q1 (I)
erhalten wird, wobei -Q1 der allgemeinen Formel IIa und -Q2 der Formel IIb entsprechen,
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- mit A gleich -NH-, -O- oder -S- und D gleich -NH- jeweils unabhängig in Formeln IIa und IIb,
- mit A' gleich -NH- und D' gleich -NH-, -O- oder -S- jeweils unabhängig in Formeln IIa und IIb, wobei jeder Rest Q1 und Q2 der Formel IIa oder IIb jeweils unabhängig mindestens eine bivalente Harnstoff-Gruppe und eine weitere bivalente Harnstoff- oder eine Carbamat-Gruppe aufweist, oder
b) ein Polysiloxan der Formel VI
HA-Q2'-B-Q1'-AH (VI)
mit einem Aminosäure-Isocyanat ausgewählt aus den Formeln IXa, IXb, IXc, IXd oder Gemischen enthaltend diese
Q2*-NH(CO)NH-"2Q-NCO (IXa)
Q1*-NH(CO)NH-"1-NCO (IXb)
Q2*-NH(CO)NH-Q2"-NCO (IXc)
Q1*-NH(CO)NH-Q1"-NCO (IXd)
umgesetzt wird,
- mit A gleich -NH-, -O- oder -S- und D gleich -NH-jeweils unabhängig in Formeln VII, I und VI,
- mit Q1' und Q2' jeweils unabhängig umfassend einen bivalenten Kohlenwasserstoff-Rest mit 6 bis 200 C-Atomen optional umfassend mindestens ein Heteroatom O, N oder S, einen bivalenten Rest umfassend Aryl-, Arylalkyl-Gruppen oder einen bivalenten Rest umfassend Aryl-, Arylalkyl-Gruppen optional umfassend mindestens ein Heteroatom O, N oder S oder Alkyl-, Aryl- oder Alkyl- und Aryl-Gruppen enthaltende Polyether-Reste, jeweils unabhängig in Formeln VII, I und VI,
- mit Q1" und Q2" jeweils unabhängig umfassend einen bivalenten linearen, verzweigten und/oder cyclischen Alkyl-Rest mit 4 bis 200 C-Atomen, oder einen bivalenten Rest umfassend einen Aryl- und/oder Arylalkyl-Rest mit 6 bis 200 C-Atomen, jeweils unabhängig in Formeln VII, I, IXa, IXb, IXc und IXd
- mit -NH-Q1* und -NH-Q2* umfassend jeweils unabhängige Reste, die aus einer Aminosäure, Aminosäure-Derivat oder deren Salz abgeleitet sind.

16. Verfahren nach Anspruch 15,
**dadurch gekennzeichnet,**
**dass** in Formel IXa, IXb, IXc und/oder IXd und I -NH-Q1* und -NH-Q2* jeweils unabhängig Reste umfassen, die aus einer Aminosäure, Aminosäure-Derivat oder deren Salz abgeleitet sind und -NH in -NH-Q1* und -NH-Q2* jeweils in alphaStellung zu einer Ester- oder einer Carboxy-Gruppe der Aminosäure, dem Aminosäure-Derivat oder deren Salz steht, wobei der Ester Alkyl-Ester mit 1 bis 25 C-Atome oder Aryl-Ester umfasst.

17. Verfahren nach Anspruch 15 oder 16,
**dadurch gekennzeichnet,**
**dass** ein Polysiloxan der Formel VI
HA-Q2'-B-Q1'-AH (VI)
eingesetzt wird, mit A ausgewählt aus -O, -NH, -S- bzw. -AH ausgewählt aus -OH, -NH₂, und -SH mit -Q2'- und -Q1'-, wie vorstehend definiert, und umgesetzt wird mit einem Diisocyanat zu einem Polysiloxan-Diisocyanat der Formel VII,
OCN-Q2"-D-(O=C)-A-Q2'-B-Q1'-A-(C=O)-D-Q1"-NCO (VII)
mit -Q2"- und/oder -Q1 "- unabhängig ausgewählt aus einem bivalenten, linearen, verzweigten und/oder cyclischen Alkyl-Rest mit 4 bis 200 C-Atomen, oder einem bivalenten Rest umfassend einen Aryl- und/oder Arylalkyl-Rest mit 6 bis 200 C-Atomen, wobei das molare Verhältnis von HA-Gruppen im Polysiloxan zu Isocyanat-Gruppen in Formel VII mindestens 1 zu 1 beträgt, insbesondere ist das Verhältnis 1 : 100 bis 1 : 1, vorzugsweise 1 : 10 bis 1 : 1.

18. Verfahren nach einem der Ansprüche 15 bis 17,
**dadurch gekennzeichnet,**
**dass**
(i) a) ein Polysiloxan-Gruppen enthaltender linearer und/oder verzweigter Polymerblock B, insbesondere der Formeln IIIa und/oder IIIb, mit mindestens zwei terminalen Si-H-Gruppen oder mindestens einer terminalen Si-H-Gruppen und mindestens einer seitenkettenständigen Si-H-Gruppe, oder b) ein Polysiloxan-Gruppen der Formel XI mit mindestens ein R¹⁷ Wasserstoff, vorzugsweise zwei R¹⁷ sind Wasserstoff,
(ii) mit einer olefinischen Verbindung umfassend Alkylen und optional umfassend mindestens ein Heteroatom N und/oder O, insbesondere Alkenylenol, Alkylenamin, quartäres Alkylamin, Alkylencarbonsäure, Alkylenester, Alkylenamid, oder einen olefinischen Polyether umgesetzt wird,
wobei die olefinische Verbindung jeweils unabhängig eine Allyl- oder Vinyl-Gruppe aufweist und den Formeln VIIIa und/oder Vlllb entspricht
Q1'-AH (VIIIa)
Q2'-AH (Vlllb)
(iii) und die Umsetzung in Gegenwart eines Katalysators zu einem Polysiloxan der Formel VI
HA-Q2'-B-Q1'-AH (VI)
erfolgt, wobei jeweils unabhängig in Formeln VIIIa, Vlllb und VI mit AH unabhängig ausgewählt aus -OH und -NH₂ und mit -Q2'- und -Q1'- jeweils unabhängig in Formeln VIIIa, Vlllb und VI umfassend einen bivalenten Kohlenwasserstoff-Rest mit 6 bis 200 C-Atomen optional umfassend mindestens ein Heteroatom O oder N einen bivalenten Rest umfassend Aryl-, Arylalkyl-Gruppen optional umfassend mindestens ein Heteroatom O oder N olefinischen Polyether.

19. Verfahren nach Anspruch 15,
**dadurch gekennzeichnet,**
**dass** ein Polysiloxan-Diisocyanat der Formel VII,
OCN-Q2"-D-(O=C)-A-Q2'-B-Q1'-A-(C=O)-D-Q1"-NCO (VII)
mit B einem linearen und/oder verzweigten Polysiloxan-Polymerblock B, mit -Q2'- und -Q1'- jeweils unabhängig umfassend einen bivalenten Kohlenwasserstoff-Rest mit 6 bis 200 C-Atomen optional umfassend mindestens ein Heteroatom O, N oder S, einen bivalenten Rest umfassend Aryl-, Arylalkyl-Gruppen optional umfassend mindestens ein Heteroatom O, N oder S, oder Alkyl-, Aryl- oder Alkyl- und Aryl-Gruppen enthaltende Polyether-Reste, mit A jeweils unabhängig gleich -NH-, -O- oder -S- und D gleich -NH- jeweils unabhängig in Formel VII, und mit -Q2"- und/oder -Q1 "- unabhängig ausgewählt aus einem bivalenten, linearen, verzweigten und/oder cyclischen Alkyl-Rest mit 4 bis 200 C-Atomen, oder einem bivalenten Rest umfassend einen Aryl- und/oder Arylalkyl-Rest mit 6 bis 200 C-Atomen,
umgesetzt wird mit einer Aminosäure, Aminosäure-Derivat, deren Salzen oder Mischungen enthaltend diese, insbesondere mit einer sekundären Amino-Gruppe der Aminosäure oder des Aminosäure-Derivats.

20. Verfahren nach Anspruch 15 oder 19,
**dadurch gekennzeichnet,**
**dass** die Aminosäure, das Aminosäure-Derivat oder das Salz eine sekundäre Amino-Gruppe aufweist, und insbesondere die sekundäre Aminosäure-Gruppe mit einer Isocyanat-Gruppe umgesetzt wird, wobei die Aminosäure, das AminosäureDerivate oder deren Salze ausgewählt sind aus unpolaren Aminosäuren ausgewählt aus Alanin, Valin, Methionin, Leucin, Isoleucin, Prolin, Tryptophan, Phenylalanin, basischen Aminosäuren umfassend Arginin, Histidin, polaren und neutralen Aminosäuren umfassend Tyrosin, Threonin, Glutamin, Glycin, Serin, Cystein, Asparagin und/oder sauren Aminosäuren ausgewählt aus Glutaminsäure und Asparaginsäure, wobei die Derivate der Aminosäuren umfassen die Mono-, Dicarbonsäureester, Amide der primären Amino-Gruppen der Aminosäuren, Amide der Carbonsäure-Gruppen der Aminosäuren und/oder Ester mit den primären Hydroxy-Gruppen oder Thioester der HS-Gruppe der Aminosäuren, insbesondere die Alkylester der Aminosäuren, bevorzugt die Methyl-, Ethyl-, Phenyl-Ester der Aminosäuren oder deren Salzen.

21. Verfahren nach Anspruch 15,
**dadurch gekennzeichnet,**
**dass** ein Diisocyanat mit einem Aminosäure-Derivat oder dessen Salz zu einem Aminosäure-Isocyanat umgesetzt wird, insbesondere zu einem Aminosäure-Isocyanat ausgewählt aus den Formeln IXa und IXb, wie vorstehend definiert.

22. Zusammensetzung erhältlich nach einem Verfahren nach einem der Ansprüche 15 bis 21.

23. Zusammensetzungen enthaltend Siloxan-Polymere nach einem der Ansprüche 1 bis 14 sowie Mischungen dieser umfassend a) Siloxan-Polymere der allgemeinen Formel XI sowie Mischungen dieser oder b) Siloxan-Polymere mit einem zentralen Polysiloxan-Polymerblock B ausgewählt aus
(i) mindestens ein Siloxan-Polymer der allgemeinen Formel I sowie Mischungen umfassend dieses Polymer,
(ii) mindestens ein Siloxan-Polymer der allgemeinen Formel Ia sowie Mischungen umfassend dieses Polymer oder
(iii) mindestens ein Siloxan-Polymer der allgemeinen Formel Ib sowie Mischungen umfassend dieses Polymer.

24. Zwischenprodukt zur Herstellung von Siloxan-Polymeren der Formel I nach einem der Ansprüche 1 bis 14, ausgewählt aus Aminosäure-Isocyanaten ausgewählt aus den Formeln IXa, IXb, IXc und IXd, deren Salzen oder Mischungen davon
Q2*-NH(CO)NH-"2Q-NCO (IXa) und/oder Q1*-NH(CO)NH-"1Q-NCO (IXb)
Q2*-NH(CO)NH-Q2"-NCO (IXc) und/oder Q1*-NH(CO)NH-Q1"-NCO (IXd)
mit Q2*, Q2", Q1* und Q1" wie vorstehend definiert, insbesondere mit Aminosäure-Isocyanaten der Formeln IXa*, IXb*, IXc*, IXd* oder deren Salzen

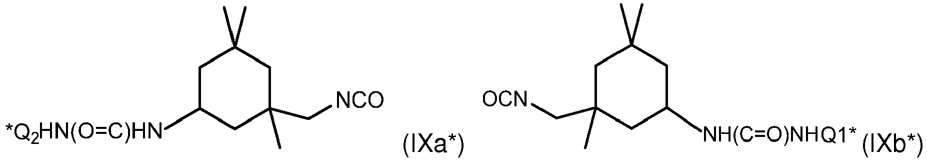

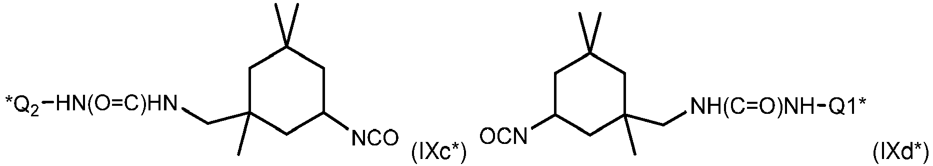

25. Formulierung enthaltend mindestens ein Siloxan-Polymere nach einem der Ansprüche 1 bis 14, 22, 23 oder hergestellt nach einem der Ansprüche 15 bis 20 oder hergestellt über das Zwischenprodukt nach Anspruch 24 und mindestens einen Hilfsstoff.

26. Verwendung der Siloxan-Polymere nach einem der Ansprüche 1 bis 14 oder der erhaltenen Zusammensetzungen umfassend Siloxan-Polymere nach einem der Ansprüche 15 bis 21 oder der Zusammensetzungen nach Anspruch 21 oder 22 als Additiv in kosmetischen Formulierungen, als Additiv in pharmazeutischen Formulierungen, in Lacken, Pasten, als Schaumstabilisator oder Schaumadditiv für Polyurethanschäume, insbesondere Polyurethanhartschäume und Polyurethanweichschäume, als Griffverbessere oder Imprägniermittel bei der bei der Herstellung von Fasern, Textilien, in kosmetischen Formulierungen zur Behandlung, Nachbehandlung und Schutz von keratinischen Fasern sowie Haut und Hautanhangsgebilden, als Additiv in Waschmittel-, Weichspülerformulierungen, in kosmetischen Formulierungen umfassend Cremes, Spülungen, Haarwachsmittel, Waschmittel, Festiger, Pflegespülungen, pflegende Pasten, Sprays, Haarsprays, zur Verbesserung der Kämmbarkeit von keratinischen oder textilen Fasern natürlichen oder synthetischen Ursprungs.

## Claims

1. Siloxane polymer of the general formula I comprising a central polysiloxane polymer block B,
(i) which is substituted with organofunctional radicals,
(ii) the polymer block B has linear and/or branched structures with at least two difunctional siloxane units,
(iii) the polymer block B has on at least two terminal silicon atoms or at least one terminal and at least one lateral silicon atom on the siloxane units of polymer block B, the organofunctional radicals -Q1 and -Q2, where the radicals are identical or different,
Q2-B-Q1 (I)
where -Q1 corresponds to the general formula IIa and-Q2 corresponds to the formula IIb,
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- where A is -NH-, -0- or -S- and D is -NH-, in each case independently in formulae IIa and IIb,
- where A' is -NH- and D' is -NH-, -0- or -S-, in each case independently
in formulae IIa and IIb, where each radical Q1 and Q2 of the formulae IIa or IIb
has at least one bivalent urea group and one further bivalent urea or carbamate group
- with Q1' and Q2' in each case independently comprising a bivalent hydrocarbon radical with 6 to 200 carbon atoms optionally comprising at least one heteroatom selected from 0, N and S, a bivalent radical comprising aryl, arylalkyl groups optionally comprising at least one
heteroatom 0, N or S or polyether radicals containing alkyl, aryl or alkyl and aryl groups,
- with Q1" and Q2" in each case independently comprising a bivalent linear, branched and/or cyclic alkyl radical having 4 to 200 carbon atoms or a bivalent radical comprising an aryl and/or arylalkyl radical with 6 to 200
carbon atoms, and
- with -D'-Q1* and -D'-Q2* with D', as defined above, and where -D'-Q1*
and -D'-Q2* in each case independently comprise radicals which are derived from an amino acid, an amino acid derivative or salts thereof.

2. Siloxane polymer according to Claim 1,
**characterized**
**in that** in the siloxane polymer of the general formulae I, IIa and IIb -D'-Q1* and -D'-Q2* in each case independently form radicals which are derived from an amino acid, an amino acid derivative or salts thereof and D' is -NH in each case independently in -D'-Q1* and -D'-Q2* is in the alpha position relative to an ester or a carboxyl group of the amino acid, of the amino acid derivative or salt, where the ester include alkyl esters with 1 to 25 carbon atoms or aryl esters.

3. Siloxane polymer according to Claim 1 or 2,
**characterized**
**in that** in the siloxane polymer of the general formula I the polymer block B corresponds to the general formula IIIa or IIIb, where B is 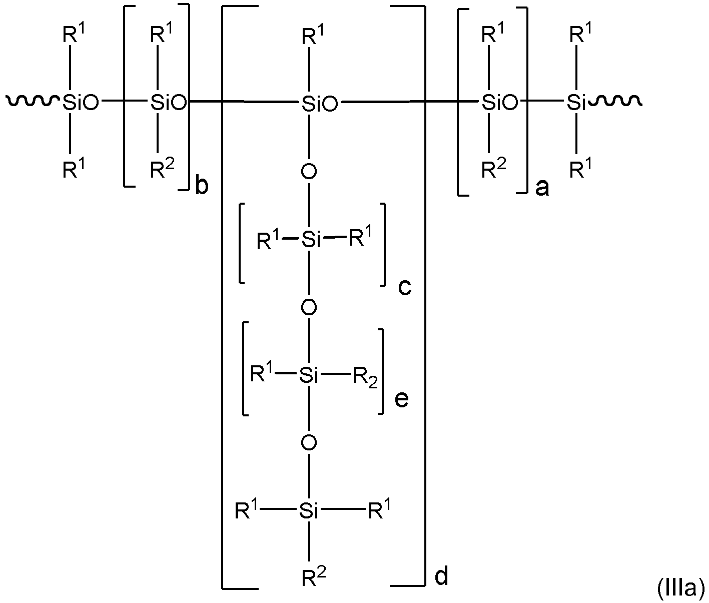 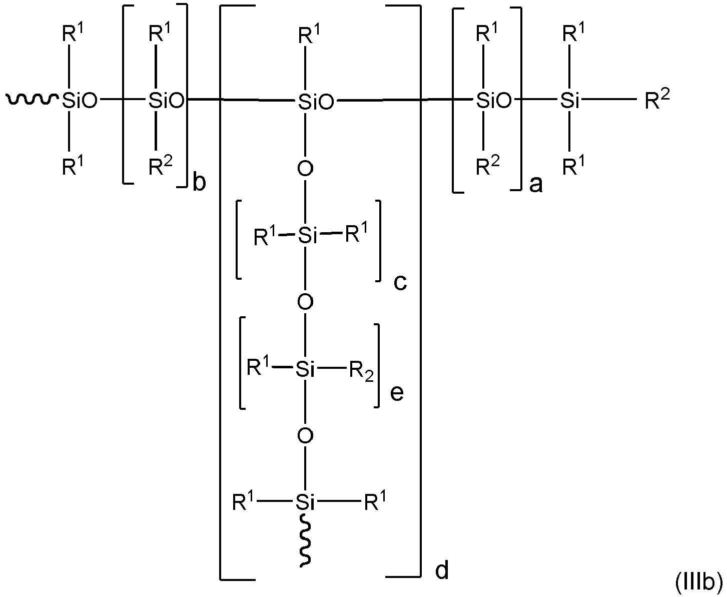 where a, b, c, d and e in formulae IIIa and IIIb are in each case independently an integer where a is from 1 to 200, where b is from 0 to 200, where c is from 0 to 200, where d is from 0 to 200, where e is from 0 to 200 and where R¹ in formulae IIIa or IIIb are in each case independently identical or different, where R¹ comprises alkyl radicals with 1 to 22 carbon atoms, preferably 1 to 4 carbon atoms, or phenyl radicals, where R² in formulae IIIa or IIIb is alkyl radical with 1 to 22 carbon atoms, preferably 1 to 4 carbon atoms, an alkyl radical with at least one heteroatom selected from N, 0, S or phenyl radical.

4. Siloxane polymer according to Claim 3,
**characterized**
**in that** b, c, d and e are 0 and a is 20 to 100, in particular 30 or 80 with a variation of plus/minus 5.

5. Siloxane polymer according to either of Claims 3 and 4,
**characterized**
**in that** R¹ and R² are selected from alkyl groups with 1, 2, 3 or 4 carbon atoms, in particular from methyl groups or at least one R² is an aminoalkyl group or a quartenary alkylamine.

6. Siloxane polymer according to one of Claims 1 to 5,
**characterized**
**in that** the radicals -Q1 and -Q2 in the general formula I are independently selected from
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
a) where A is -0-, D is -NH-, A' is -NH- and D' is -NH- ,
b) where A is -NH-, D is -NH- , A' is -NH- and D' is - NH-,
c) where A is -S-, D is -NH-, where A' is -NH- and D' is -NH-,
d) where A is -0-, D is -NH-, A' is -NH- and D' is -0-,
e) where A is -NH-, D is -NH-, A' is -NH- and D' is -O- ,
f) where A is -S-, D is -NH-, A' is -NH- and D' is -0-,
g) where A is -0-, D is -NH-, A' is -NH- and D' is -S-,
h) where A is -NH-, D is -NH-, A' is -NH- und D' is -S- or
i) where A is -S-, D is -NH-, A' is -NH- and D' is -S-.

7. Siloxane polymer according to one of Claims 1 to 6,
**characterized**
**in that** in the radicals -Q1 and -Q2 of the formula I the radicals -D'-Q1* and -D'-Q2* are in each case independently derived from amino acids, amino acid derivatives or salts thereof, comprising
- nonpolar amino acids comprising alanine, valine, methionine, leucine, isoleucine, proline, tryptophan, phenylalanine,
- basic amino acids comprising lysine, arginine, histidine,
- polar and neutral amino acids tyrosine, threonine, glutamine, glycine, serine, cysteine, asparagine and/or
- acidic amino acids selected from glutamic acid and aspartic acid,
and their mono-, dicarboxylic acid esters, amides with primary amino groups of the amino acids, amides of the carboxylic acid groups of the amino acids and/or esters with the primary hydroxyl groups, thioesters of the HS groups or amides with secondary amino groups of the amino acids.

8. Siloxane polymer according to one of Claims 1 to 7,
**characterized**
**in that** in the radicals -Q1 and -Q2 of the formula I A is -0-, D is -NH-, A' is -NH- and D' is -NH-, -0- or -S- and the radicals -D'-Q1* and -D'-Q2* are in each case independently derived from amino acids, amino acid derivatives or salts thereof, in particular comprising nonpolar amino acids comprising alanine, valine, methionine, leucine, isoleucine, proline, tryptophan, phenylalanine, basic amino acids comprising lysine, arginine, histidine, polar and neutral amino acids comprising tyrosine, threonine, glutamine, glycine, serine, cysteine, asparagine and/or the acidic amino acids selected from glutamic acid and aspartic acid, and their mono-, dicarboxylic acid esters, amides with primary amino groups of the amino acids, amides of the carboxylic acid groups of the amino acids and/or esters with the primary hydroxyl groups or thioesters of the HS group, and salts thereof.

9. Siloxane polymer according to one of Claims 1 to 8,
**characterized**
**in that** in the radicals -Q1 and -Q2 of the formula I the bivalent radicals -Q1"- and -Q2"- are independently selected from bivalent, linear, branched or cyclic alkylene radicals with 4 to 25 carbon atoms, in particular with 6, 7, 8, 9, 10, 11, 12, 13 or 14 carbon atoms, preferably hexylene (-CH₂)₆, heptylene, bivalent 2,4-toluolyl, diphenylmethane, polymeric diphenylmethane, 3,5,5-trimethyl-1-methylene-3-ethylene-cyclohexane or 4,4'-dicyclohexylene.

10. Siloxane polymer according to one of Claims 1 to 9,
**characterized**
**in that** in the radicals -Q1 and -Q2 of the formula I the bivalent radicals -Q1'- and -Q2'- are selected from alkylene radicals with 3 to 22 carbon atoms optionally with at least one heteroatom comprising N, 0 or S, in particular -(CH₂)ₙ- where n is from 3 to 22 carbon atoms, preferably hexylene (-CH₂)₆-, heptylene (-CH₂)₇-, octylene (-CH₂)₈-, nonylene (-CH₂)₉-, decylene (-CH₂)₁₀-, undecylene (-CH₂)₁₁-, dodecylene (-CH₂)₁₂-, or with heteroatoms, alkylene-(C=O)-, alkylene-O(CO)-alkylene, alkylene-(CO)O-alkylene, alkylene-NH(CO)-alkylene, alkylene-(CO)NH-alkylene, alkylene-NH(CO)NH-alkylene, alkylene-NH(CO)O-alkylene, or from polyether radicals containing alkyl, aryl or alkyl and aryl groups the formulae IVa or IVb where Q1' and Q2' are in each case independently
-T-O-(CH₂-CH₂-O-)ₓ-(CH₂-CH(R^{#})O-)_{y}-(SO) -R" (IVa)
-T-O-(CH₂-CH₂-O-)ₓ-(CH₂-CH(R^{#}) O-)_{y}-R" (IVb),
where T = bivalent hydrocarbon radical with 2 to 4 carbon atoms, where x = 0 to 200,
y = 0 to 200, where x and y are integers, with the proviso that x or y is at least 1, where R^{#} is hydrogen or methyl, R" is linear or branched alkylene, preferably where T = -(CH₂)₂- or -(CH₂)₃-.

11. Siloxane polymer according to one of Claims 1 to 10
**characterized**
**in that** in the radicals -Q1 and -Q2 of the formula (I) at least one of the bivalent
radicals -Q1 " - and -Q2"- is independently a bivalent cyclohexane-containing radical selected from the formulae Va and Vb 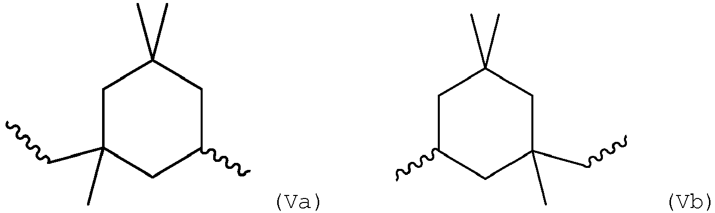 in particular Q2"- is a bivalent cyclohexane-containing radical of the formula Va and -Q1" is of the formula Vb.

12. Siloxane polymer according to one of Claims 1 or 2 and 6 to 11, **characterized in that**
siloxane of the general formula I corresponds to the siloxane polymer of the general
formula XI in
Mₐ₁M^{A}ₐ₂M^{B}ₐ₃D_{b1}D^{A}_{b2}D^{B}_{b3}T_{c1}T^{A}_{c2}T^{B}_{c3}Q_{d1} (XI)
where M = [R¹⁶3SiO_{1/2}], M^{A} = [R¹⁷R¹⁶₂SiO_{1/2}], M^{B} = [R¹⁸R¹⁶₂SiO_{1/2}],
D = [R¹⁶₂SiO_{2/2}], D^{A} = [R¹⁷₁R¹⁶₁SiO_{2/2}], D^{B} = [R¹⁸1R¹⁶1SiO_{2/2}],
T = [R¹⁶SiO_{3/2}], T^{A} = [R¹⁷SiO_{3/2}], T^{B} = [R¹⁸SiO_{3/2}], Q = [SiO_{4/2}],
where
R¹⁶, independently of one another, are identical or different linear or branched, saturated or unsaturated hydrocarbon radicals having 1 to 30 carbon atoms or else aromatic hydrocarbon radicals having 6 to 30 carbon atoms, preferably methyl or phenyl, in particular methyl,
where R¹⁷ is in each case independently -Q1 or -Q2,
R¹⁸ independently of one another are identical or different linear or branched, saturated or olefinically unsaturated hydrocarbon radicals with 8 to 30 carbon atoms, aromatic hydrocarbon radical with 6 to 40 carbon atoms, alkylaryl radical with 7 to 40 carbon atoms, a linear or branched optionally double-bond-containing aliphatic hydrocarbon radical with 2 to 30 carbon atoms that is interrupted by one or more heteroatoms, such as oxygen, NH, NR' where R' is an optionally double-bond-containing C1 to C30-alkyl radical, a linear or branched optionally double-bond-containing aliphatic hydrocarbon radical with 2 to 30 carbon atoms interrupted by one or more functionalities selected from the group -OH, -O-C(O)-, -(O)C-O-, -NH-C(O)-, -(O)C-NH, -(CH₃)N-C(O)-, -(O)C-N(CH₃)-, -S(O₂)-O-, -O-S(O₂)-, -S(O₂)-NH-,
-NH-S(O₂)-, -S(O₂)-N(CH₃)-, -N(CH₃)-S(O₂)-,
a linear or branched optionally double-bond-containing aliphatic or cycloaliphatic hydrocarbon radical with 1 to 30 carbon atoms terminally functionalized with OH, OR', NH², N(H)R', N(R')₂ where R' is an optionally double-bond-containing C1 to C30-alkyl radical, or
a blockwise or randomly constructed polyether according to (R⁵-O)ₙ-R⁶, where R⁵ is a linear or branched hydrocarbon radical containing 2 to 4 carbon atoms, n is 1 to 100, preferably 2 to 60, and R⁶ is hydrogen, a linear or branched optionally double-bond-containing aliphatic hydrocarbon radical with 1 to 30 carbon atoms, an optionally double-bond-containing cycloaliphatic hydrocarbon radical with 5 to 40 carbon atoms, an aromatic hydrocarbon radical with 6 to 40 carbon atoms, an alkylaryl radical with 7 to 40 carbon atoms, or a radical -C(O)-R⁷ where R⁷ is a linear or branched optionally double-bond-containing aliphatic hydrocarbon radical with 1 to 30 carbon atoms, an optionally double-bond-containing cycloaliphatic hydrocarbon radical with 5 to 40 carbon atoms, an aromatic hydrocarbon radical with 6 to 40 carbon atoms, an alkylaryl radical with 7 to 40 carbon atoms with the indices
a1 = 0-200, preferably 1-60, in particular 0,
a2 = 0-30, preferably 1-20, in particular 2-10,
a3= 0-30, preferably 1-20,
b1 = 2 to 5000, preferably 10 to 1000,
b2 = 0 to 100, preferably 1 to 30, in particular 1 to 10 or 0,
b3 = 0 to 100, preferably 0 to 30, in particular 1 to 10 or 0,
c1 = 0 to 30, preferably 1 to 30,
c2 = 0 to 30, preferably 0 to 5, in particular 0,
c3 = 0 to 30, preferably 0 to 5, in particular 0,
d1 = 0 to 30, preferably 0 to 5, in particular 0,
with the proviso that at least one of the indices selected from a2 and a3 is not equal to 0, preferably a2 is an integer between 2 and 10.

13. Siloxane polymer according to one of Claims 1 to 12,
**characterized**
**in that** it is selected from siloxane polymers of the formulae Ia and Ib or mixtures of these 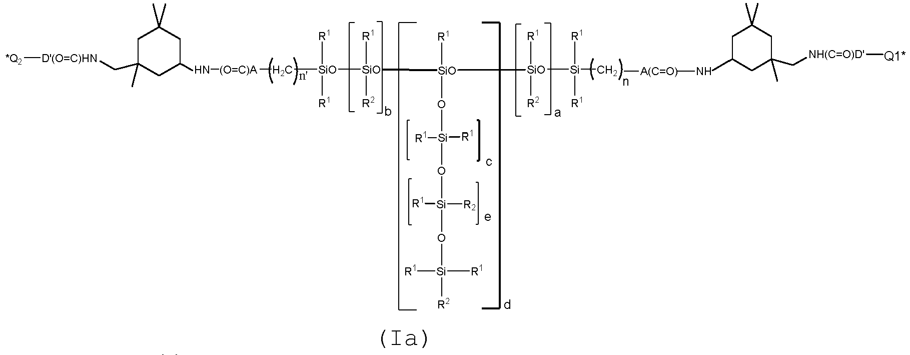 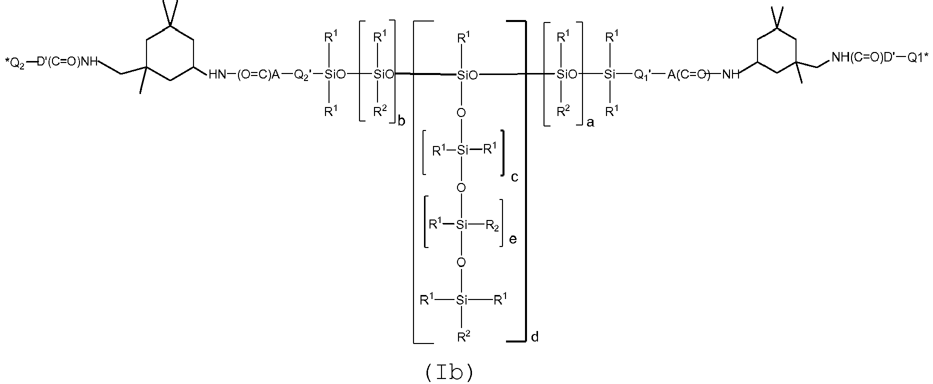
where n or n' is in each case independently selected from an integer from 3 to 22,
where a is from 1 to 200, where b is from 0 to 200,
where c is from 0 to 200, where d is from 0 to 200,
where e is from 0 to 200 and where R¹ in formulae Ia and Ib are in each case independently identical or different, where R¹ comprises alkyl radicals with 1 to 4 carbon atoms or phenyl radicals, where R² is alkyl radical with 1 to 22 carbon atoms, preferably 1 to 4 carbon atoms, an alkyl radical with at least one heteroatom selected from N, 0, S or phenyl radical and where -D'-Q1* and -D'-Q2* are in each case independently derived from amino acids, amino acid derivatives or salts thereof, and in formula Ib where Q1' and Q2' are in each case independently alkylene with 2 to 40 carbon atoms or a polyether of the formulae IVa or IVb
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb)
where T = bivalent hydrocarbon radical with 2 to 4 carbon atoms, where x = 0 to 200,
y = 0 to 200, where x and y are integers, with the proviso that x or y is at least 1, where R^{#} is hydrogen or methyl, R" is hydrogen or linear or branched alkylene, in particular where T = (CH₂)₂- or -(CH₂)₃-.

14. Siloxane polymer according to one of Claims 1 to 13,
**characterized**
**in that** they are selected from siloxane polymers of the formula Ia* and Ib* 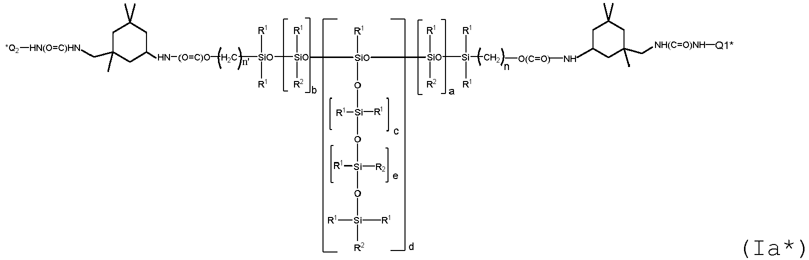 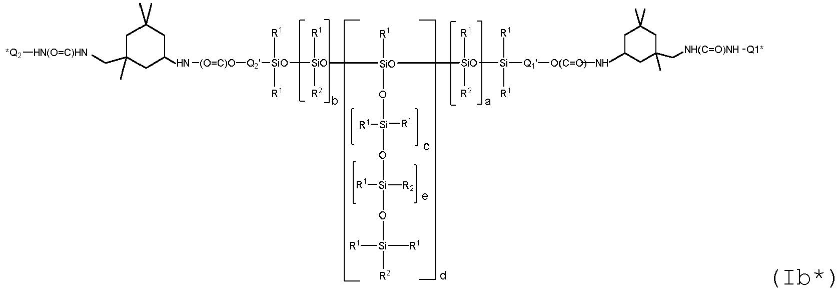
where n or n' in each case independently is selected from an integer from 3 to 22,
where a is from 1 to 200, where b is from 0 to 200,
where c is from 0 to 200, where d is from 0 to 200,
where e is from 0 to 200 and
where R¹ in formulae Ia* and Ib* is in each case independently identical or different, where R¹ comprises alkyl radicals with 1 to 4 carbon atoms or phenyl radicals, where R² is alkyl radical with 1 to 22 carbon atoms, preferably 1 to 4 carbon atoms, an alkyl radical with at least one heteroatom comprising N, 0, S or phenyl radical and where Q1* and Q2* are in each case independently selected from amino acids, amino acid derivatives or salts thereof, where the fragment -NH-Q1* and -NH-Q2*, as a result of reaction of the secondary alpha-amino groups of the aminoacids, derivatives thereof or the salts with the isocyanate, forms a urea group, and where the amino acids comprising nonpolar amino acids comprising alanine, valine, methionine, leucine, isoleucine, proline, tryptophan, phenylalanine, basic amino acids comprising lysine, arginine, histidine, polar and neutral amino acids comprising tyrosine, threonine, glutamine, glycine, serine, cysteine, asparagine and/or the acidic amino acids selected from glutamic acid and aspartic acid, and their mono-, dicarboxylic acid esters, amides with primary amino groups of the amino acids, amides of the carboxylic acid groups of the amino acids and/or esters with the primary hydroxyl groups or thioesters of the HS group or salts thereof,
and in formula Ib* where Q1' and Q2' are in each case independently
-T-O-(CH₂-CH₂-O-)ₓ-(CH₂-CH(R^{#})O-)_{y}-(SO) -R" (IVa)
-T-O-(CH₂-CH₂-O-)ₓ-(CH₂-CH(R^{#})O-)_{y}-R" (IVb),
where T = bivalent hydrocarbon radical with 2 to 4 carbon atoms, where x = 0 to 200, y = 0 to 200, where x and y are integers with the proviso that x or y is at least 1, where R^{#} is hydrogen or methyl, R" is hydrogen or linear or branched alkylene, in particular ethylene, in particular with T = -(CH₂)₂- or -(CH₂)₃-.

15. Process for the preparation of a siloxane polymer and of compositions comprising these siloxane polymers or mixtures of the siloxane polymers with a central polysiloxane polymer block B, by reacting
a) a polysiloxane diisocyanate of the formula VII,
OCN-Q2"-D-(O=C)-A-Q2'-B-Q1'-A-(C=O)-D-Q1"-NCO (VII)
with at least one amino acid, an amino acid derivative, salt thereof or mixtures of these, giving a siloxane polymer of the general formula I
Q2-B-Q1 (I)
where -Q1 corresponds to the general formula IIa and-Q2 corresponds to the formula IIb,
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- where A is -NH-, -0- or -S- and D is -NH- in each case independently in formulae IIa and IIb,
- where A' is -NH- and D' is -NH-, -0- or -S- in each case independently in formulae IIa and IIb, where each radical Q1 and Q2 of the formula IIa or IIb in each case
independently has at least one bivalent urea group and one further
bivalent urea or a carbamate group, or
b) a polysiloxane of the formula VI
HA-Q2'-B-Q1'-AH (VI)
is reacted with an amino acid isocyanate selected from the formulae IXa, IXb, IXc, IXd or mixtures comprising these
Q2*-NH(CO)NH-"2Q-NCO (IXa)
Q1*-NH(CO)NH-"1-NCO (IXb)
Q2*-NH(CO)NH-Q2"-NCO (IXc)
Q1*-NH(CO)NH-Q1"-NCO (IXd)
- where A is -NH-, -0- or -S- and D is -NH- in each case independently in formulae VII, I and VI,
- where Q1' and Q2' in each case independently comprising a bivalent hydrocarbon radical with 6 to 200 carbon atoms optionally comprising at least one heteroatom 0, N or S, a bivalent radical comprising aryl, arylalkylgroups or a bivalent radical comprising aryl, arylalkyl groups optionally comprising at least one heteroatom 0, N or S or polyether radicals containing alkyl, aryl or alkyl and aryl groups, in each case independently in formulae VII, I and VI,
- with Q1" and Q2" in each case independently comprising a bivalent linear, branched and/or cyclic alkyl radical with 4 to 200 carbon atoms, or a bivalent radical comprising an aryl and/or arylalkyl radical with 6 to 200 carbon atoms, in each case independently in formulae VII, I, IXa, IXb, IXc and IXd
- with -NH-Q1* and -NH-Q2* comprising in each case independent radicals which are derived from an amino acid, amino acid derivative or salt thereof.

16. Process according to Claim 15,
**characterized**
**in that** in formula IXa, IXb, IXc and/or IXd and I -NH-Q1* and -NH-Q2* in each case independently comprise radicals which are derived from an amino acid, amino acid derivative or salt thereof and -NH in -NH-Q1* and -NH-Q2* in each case is in the alpha position relative to an ester or a carboxyl group of the amino acid, the amino acid derivative or salt thereof, where the ester comprises alkyl ester with 1 to 25 carbon atoms or aryl ester.

17. Process according to Claim 15 or 16,
**characterized**
**in that** a polysiloxane of the formula VI
HA-Q2'-B-Q1'-AH (VI)
is used, where A is selected from -0, -NH, -S- or -AH selected from -OH, -NH₂, and -SH with -Q2'- and -Q1'-, as defined above, and is reacted with a diisocyanate to give a polysiloxane diisocyanate of the formula VII,
OCN-Q2"-D-(O=C)-A-Q2'-B-Q1'-A-(C=O)-D-Q1"-NCO (VII)
with -Q2"- and/or -Q1"- independently selected from a bivalent, linear, branched and/or cyclic alkyl radical with 4 to 200 carbon atoms, or a bivalent radical comprising an aryl and/or arylalkyl radical with 6 to 200 carbon atoms, where the molar ratio of HA groups in the polysiloxane to isocyanate groups in formula VII is at least 1:1, in particular the ratio is 1:100 to 1:1, preferably 1:10 to 1:1.

18. Process according to any of Claims 15 to 17,
**characterized**
**in that**
(i) a) a polysiloxane-group-containing linear and/or branched polymer block B, in particular of the formulae IIIa and/or IIIb, with at least two terminal Si-H groups or at least one terminal Si-H group and at least one lateral Si-H group, or b) a polysiloxane groups of the formula XI with at least one R¹⁷ is hydrogen, preferably two R¹⁷ are hydrogen,
(ii) is reacted with an olefinic compound comprising alkylene and optionally comprising at least one heteroatom N and/or 0, in particular alkenylenol, alkylenamine, quarternary alkylamine, alkylencarboxylic acid, alkylene ester, alkylenamide, or an olefinic polyether,
where the olefinic compound has in each case independently an allyl or vinyl group and corresponds to the formulae VIIIa and/or VIIIb
Q1'-AH (VIIIa)
Q2'-AH (VIIIb)
(iii) and the reaction takes place in the presence of a catalyst to give a polysiloxane of the formula VI
HA-Q2'-B-Q1'-AH (VI)
where in each case independently in formulae VIIIa, VIIIb and VI with AH independently selected from -OH and -NH₂ and with -Q2'- and -Q1'- in each case independently in formulae VIIIa, VIIIb and VI comprising a bivalent hydrocarbon radical with 6 to 200 carbon atoms optionally comprising at least one heteroatom 0 or N a bivalent radical comprising aryl, arylalkyl groups optionally comprising at least one heteroatom 0 or N olefinic polyether.

19. Process according to Claim 15,
**characterized**
**in that** a polysiloxane diisocyanate of the formula VII,
OCN-Q2"-D-(O=C)-A-Q2'-B-Q1'-A-(C=O)-D-Q1"-NCO (VII)
where B is a linear and/or branched polysiloxane polymer block B, with -Q2'- and -Q1'- in each case independently comprising a bivalent hydrocarbon radical with 6 to 200 carbon atoms optionally comprising at least one heteroatom 0, N, or S, a bivalent radical comprising aryl, arylalkyl groups optionally comprising at least one heteroatom 0, N or S, or polyether radicals containing alkyl, aryl or alkyl and aryl groups, where A is in each case independently -NH-, -O- or -S- and D is -NH- in each case independently in formula VII, and where -Q2"- and/or -Q1"- are independently selected from a bivalent, linear, branched and/or cyclic alkyl radical with 4 to 200 carbon atoms, or a bivalent radical comprising an aryl and/or arylalkyl radical with 6 to 200 carbon atoms,
is reacted with an amino acid, amino acid derivative, salts thereof or mixtures comprising these, in particular with a secondary amino group of the amino acid or of the amino acid derivative.

20. Process according to Claim 15 or 19,
**characterized**
**in that** the amino acid, the amino acid derivative or the salt has a secondary amino group, and in particular the secondary amino acid group is reacted with an isocyanate group, where the amino acid, the amino acid derivatives or salts thereof are selected from nonpolar amino acids selected from alanine, valine, methionine, leucine, isoleucine, proline, tryptophan, phenylalanine, basic amino acids comprising arginine, histidine, polar and neutral amino acids comprising tyrosine, threonine, glutamine, glycine, serine, cysteine, asparagine and/or acid amino acids selected from glutamic acid and aspartic acid, where the derivatives of the amino acids comprise the mono-, dicarboxylic acid esters, amides of the primary amino groups of the amino acids, amides of the carboxylic acid groups of the amino acids and/or esters with the primary hydroxyl groups or thioesters of the HS group of the amino acids, in particular the alkyl esters of the amino acids, preferably the methyl, ethyl, phenyl esters of the amino acids or salts thereof.

21. Process according to Claim 15,
**characterized**
**in that** a diisocyanate is reacted with an amino acid derivative or salt thereof to give an amino acid isocyanate, in particular to give an amino acid isocyanate selected from the formulae IXa and IXb, as defined above.

22. Composition obtainable by a process according to any of Claims 15 to 21.

23. Compositions comprising siloxane polymers according to any of Claims 1 to 14, and mixtures of these comprising a) siloxane polymers of the general formula XI, and mixtures of these or b) siloxane polymers with a central polysiloxane polymer block B selected from
(i) at least one siloxane polymer of the general formula I, and mixtures comprising this polymer,
(ii) at least one siloxane polymer of the general formula Ia, and mixtures comprising this polymer or
(iii) at least one siloxane polymer of the general formula Ib, and mixtures comprisign this polymer.

24. Intermediate for preparing siloxane polymers of the formula I according to any of Claims 1 to 14, selected from amino acid isocyanates selected from the formulae IXa,IXb, IXc and IXd, salts thereof or mixtures thereof
Q2*-NH(CO)NH-"2Q-NCO (IXa)
and/or
Q1*-NH(CO)NH-"1Q-NCO (IXb)
Q2*-NH(CO)NH-Q2"-NCO (IXc)
and/or
Q1*-NH(CO)NH-Q1"-NCO (IXd)
with Q2*, Q2", Q1* and Q1" as defined above, in particular with amino acid isocyanates of the formulae IXa*, IXb*, IXc*, IXd* or salts thereof

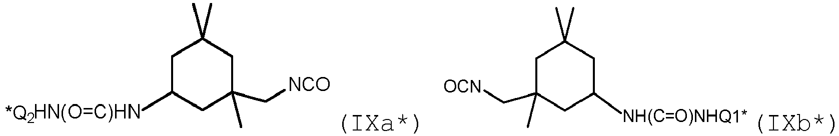

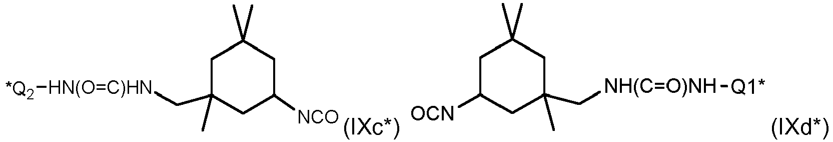

25. Formulation comprising at least one siloxane polymer according to any of Claims 1 to 14, 22, 23 or prepared according to any of Claims 15 to 20 or prepared via the intermediate according to Claim 24 and at least one auxiliary.

26. Use of the siloxane polymers according to any of Claims 1 to 14 or of the resulting compositions comprising siloxane polymers according to any of Claims 15 to 21 or of the compositions according to Claim 21 or 22 as additive in cosmetic formulations, as additive in pharmaceutical formulations, in coatings, pastes, as foam stabilizer or foam additive for polyurethane foams, in particular polyurethane rigid foams and polyurethane flexible foams, as hand improvers or impregnation compositions during the during the production of fibres, textiles, in cosmetic formulations for the treatment, post-treatment and protection of keratin fibres, and also skin and skin appendages, as additive in detergent, fabric softener formulations, in cosmetic formulations including creams, rinses, hair washing compositions, washing compositions, setting compositions, care rinses, care pastes, sprays, hair sprays, for improving the combability of keratin or textile fibres of natural or synthetic origin.

## Revendications

1. Polymère de siloxane de formule générale I, comprenant une séquence de polymère B de polysiloxane central,
(i) lequel est substitué par des résidus organo-fonctionnels ;
(ii) la séquence de polymère B présente des structures linéaires et/ou ramifiées avec au moins deux motifs difonctionnels de siloxane ;
(iii) la séquence de polymère B présente les résidus organo-fonctionnels -Q1 et -Q2 au niveau d'au moins deux atomes de silicium terminaux, d'une part, ou au niveau d'au moins un atome de silicium terminal et d'au moins un atome de silicium en chaînes latérales des motifs de siloxane du bloc de polymères B, d'autre part, dans lequel les résidus sont égaux ou différents :
Q2-B-Q1 (I)
dans laquelle -Q1 correspond à la formule générale IIa et -Q2 à la formule IIb :
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- dans lesquelles A représente -NH-, -0- ou -S- et D représente -NH-, en étant respectivement indépendants dans les formules IIa et IIb ;
- dans lesquelles A' représente -NH- et D' représente -NH-, -0- ou -S-, en étant respectivement indépendants dans les formules IIa et IIb, dans lesquelles chaque résidu Q1 et Q2 des formules IIa ou IIb présente au moins un groupe bivalent constitué par l'urée et un autre groupe bivalent constitué par l'urée ou par le carbamate ;
- dans lesquelles Q1' et Q2', étant respectivement indépendants, comprennent un résidu bivalent d'hydrocarbures avec 6 à 200 atomes de carbone, et comprenant facultativement au moins un hétéroatome choisi parmi 0, N et S, un résidu bivalent comprenant des groupes constitués par l'aryle et l'arylalkyle, comprenant facultativement au moins un hétéroatome 0, N ou S ou des résidus de polyéther contenant des groupes consistant en alkyle ou aryle, d'une part, ou en alkyle et aryle, d'autre part ;
- dans lesquelles Q1" et Q2", étant respectivement indépendants, comprennent un résidu bivalent d'alkyle linéaire, ramifié et/ou cyclique avec 4 à 200 atomes de carbone ou un résidu bivalent comprenant un résidu d'aryle et/ou d'arylalkyle avec 6 à 200 atomes de carbone ; et
- dans lesquelles -D'-Q1* et -D'-Q2* avec D', tel que défini plus haut, et dans lequel -D'-Q1* et -D'-Q2*, étant respectivement indépendants, comprennent des résidus qui sont dérivés d'un acide aminé, d'un dérivé d'acide aminé ou de leurs sels.

2. Polymère de siloxane selon la revendication 1,
**caractérisé en ce que**
dans le polymère de siloxane des formules générales I, IIa et IIb, -D'-Q1* et -D'-Q2*, étant respectivement indépendants, forment des résidus qui sont dérivés d'un acide aminé, d'un dérivé d'acide aminé ou de leurs sels et où D' représente -NH, en étant respectivement indépendant dans -D'-Q1* et - D'-Q2*, et se trouve en position alpha par rapport à un groupe consistant en un ester ou carboxyle de l'acide aminé, un dérivé de l'acide aminé ou du sel, dans lequel l'esters comprennent de l'ester d'alkyle avec 1 à 25 atomes de carbone ou de l'ester d'aryle.

3. Polymère de siloxane selon la revendication 1 ou 2,
**caractérisé en ce que**
dans le polymère de siloxane de formule générale I, la séquence de polymère B correspond à la formule générale IIIa ou IIIb, 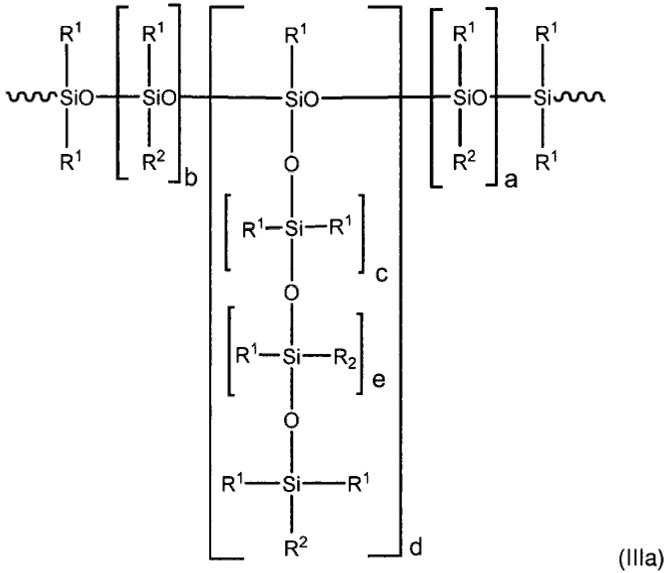 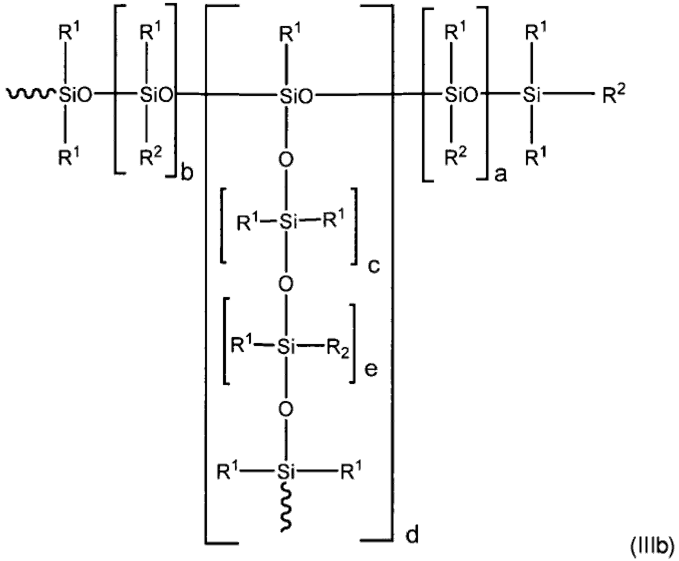 où B représente a, b, c, d et e, en étant respectivement indépendants dans les formules IIIa et IIIb pour donner lieu à un nombre entier dans lequel a est compris entre 1 et 200, dans lequel b est compris entre 0 et 200, dans lequel c est compris entre 0 et 200, dans lequel d est compris entre 0 et 200, dans lequel e est compris entre 0 et 200 et dans lequel les R¹ sont identiques ou différents, en étant respectivement indépendants dans les formules IIIa ou IIIb, où R¹ comprend des résidus d'alkyle avec 1 à 22 atomes de carbone, avec de préférence 1 à 4 atomes de carbone ou comprend des résidus de phényle, dans lequel R² dans les formules IIIa ou IIIb représente un résidu d'alkyle avec 1 à 22 atomes de carbone, avec de préférence 1 à 4 atomes de carbone, ou un résidu d'alkyle avec au moins un hétéroatome choisi parmi N, 0, S ou un résidu de phényle.

4. Polymère de siloxane selon la revendication 3,
**caractérisé en ce que**
b, c, d et e sont égaux à 0 et a représente une valeur comprise entre 20 et 100, en particulier 30 ou 80, avec une marge de fluctuation de plus ou moins 5.

5. Polymère de siloxane selon l'une des revendications 3 ou 4,
**caractérisé en ce que**
R¹ et R² sont choisis parmi les groupes consistant en alkyle avec 1, 2, 3 ou 4 atomes de carbone, en particulier parmi des groupes consistant en méthyle, ou bien au moins un R² est un groupe consistant en aminoalkyle ou une alkylamine quaternaire.

6. Polymère de siloxane selon l'une des revendications 1 à 5,
**caractérisé en ce que**
les résidus -Q1 et -Q2 dans la formule générale I, sont indépendamment choisis parmi :
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
a) dans lesquelles A représente -0-, D représente -NH-, A' représente -NH- et D' représente -NH- ;
b) dans lesquelles A représente -NH-, D représente -NH-, A' représente -NH- et D' représente-NH- ;
c) dans lesquelles A représente -S-, D représente -NH-, avec A' représente -NH- et D' représente -NH- ;
d) dans lesquelles A représente -0-, D représente -NH-, A' représente -NH- et D' représente -0- ;
e) dans lesquelles A représente -NH-, D représente -NH-, A' représente -NH- et D' représente -0- ;
f) dans lesquelles A représente -S-, D représente -NH-, A' représente -NH- et D' représente -0- ;
g) dans lesquelles A représente -0-, D représente -NH-, A' représente -NH- et D' représente -S- ;
h) dans lesquelles A représente -NH-, D représente -NH-, A' représente -NH- et D' représente -S- ; ou
i) dans lesquelles A représente -S-, D représente -NH-, A' représente -NH- et D' représente -S-.

7. Polymère de siloxane selon l'une des revendications 1 à 6,
**caractérisé en ce que**
dans les résidus -Q1 et -Q2 de formule I, les résidus -D'-Q1* et -D'-Q2*, étant respectivement indépendants, sont dérivés d'acides aminés, de dérivés d'acides aminés ou de leurs sels, comprenant :
- des acides aminés non polaires comprenant l'alanine, la valine, la méthionine, la leucine, l'isoleucine, la proline, le tryptophane, la phénylalanine ;
- des acides aminés basiques comprenant la lysine, l'arginine, l'histidine ;
- des acides aminés polaires et neutres comprenant la tyrosine, la thréonine, la glutamine, la glycine, la sérine, la cystéine, l'asparagine ; et/ou
- des acides aminés acides choisis parmi l'acide glutamique et l'acide aspartique, ainsi que leurs esters d'acide monocarboxylique, leurs esters d'acide dicarboxylique, leurs amides avec des groupes amino primaires des acides aminés, des amides des groupes des acides aminés consistant en un acide carboxylique et/ou les esters avec les groupes primaires consistant en hydroxy, des thioesters du groupe HS ou des amides avec des groupes amino secondaires des acides aminés.

8. Polymère de siloxane selon l'une des revendications 1 à 7,
**caractérisé en ce que**
dans les résidus -Q1 et -Q2 de formule I, A représente -0-, D représente -NH-, A' représente - NH- et D' représente -NH-, -0- ou -S-, et les résidus -D'-Q1* et -D'-Q2*, étant respectivement indépendants, sont dérivés d'acides aminés, de dérivés d'acides aminés ou de leurs sels, comprenant en particulier des acides aminés non polaires comprenant l'alanine, la valine, la méthionine, la leucine, l'isoleucine, la proline, le tryptophane, la phénylalanine, et/ou des acides aminés basiques comprenant la lysine, l'arginine, l'histidine, et/ou des acides aminés polaires et neutres comprenant la tyrosine, la thréonine, la glutamine, la glycine, la sérine, la cystéine, l'asparagine et/ou des acides aminés acides choisis parmi l'acide glutamique et de l'acide aspartique, ainsi que de leurs esters d'acide monocarboxylique, de leurs esters d'acide dicarboxylique, des amides des groupes amino primaires des acides aminés, des amides des groupes des acides aminés consistant en un acide carboxylique et/ou des esters avec les groupes primaires consistant en hydroxy ou des thioesters du groupe HS, ainsi que leurs sels.

9. Polymère de siloxane selon l'une des revendications 1 à 8,
**caractérisé en ce que**
dans les résidus -Q1 et -Q2 de formule I, les résidus bivalents -Q1"- et -Q2"- sont indépendamment choisis parmi des résidus bivalents d'alkyle, linéaires, ramifiés ou cycliques avec 4 à 25 atomes de carbone, en particulier avec 6, 7, 8, 9, 10, 11, 12, 13 ou 14 atomes de carbone, de préférence à partir de l'hexylène (-CH₂)₆, de l'heptyle, du 2,4-tuluolyle bivalent, du diphénylméthane, du diphénylméthane polymérisé, du 3,5,5-triméthyle-1-méthylène-3-éthylène-cyclohexane ou du 4,4'-dicyclohexylène.

10. Polymère de siloxane selon l'une des revendications 1 à 9,
**caractérisé en ce que**
dans les résidus -Q1 et -Q2 de formule I, les résidus bivalents -Q1'- et -Q2'- sont choisis parmi des résidus d'alkyle avec 3 à 22 atomes de carbone, facultativement avec au moins un hétéroatome comprenant N, O ou S, en particulier -(CH₂)ₙ-, où n représente une valeur comprise entre 3 et 22 atomes de carbone, de préférence à partir de l'hexylène (-CH₂)₆-, de l'heptyle (-CH₂)₇-, de l'octylène (-CH₂)₈-, du nonylène (-CH₂)₉-, du décylène (-CH₂)₁₀-, de l'undécylène (-CH₂)₁₁-, du dodécylène (-CH₂)₁₂- ou avec des hétéroatomes, d'un alkylène-(C=O)-, d'un alkylène-O(CO)-alkylène, d'un alkylène-(CO)O-alkylène, d'un alkylène-NH(CO)-alkylène, d'un alkylène-(CO)NH-alkylène, d'un alkylène-NH(CO)NH-alkylène, d'un alkylène-NH(CO)O-alkylène ou à partir des résidus de polyéther des groupes consistant en alkyle ou aryle, d'une part, ou en alkyle et aryle, d'autre part, et sont égaux les formules IVa ou IVb, où Q1' et Q2' sont respectivement indépendants :
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (lVb),
dans lesquelles T = un résidu bivalent d'hydrocarbures avec 2 à 4 atomes de carbone, dans lesquelles x correspond à une valeur comprise entre 0 et 200 et y à une valeur comprise entre 0 et 200, où x et y sont des nombres entiers, à la condition que x ou y soit au moins égal à 1, dans lesquelles R^{#} est de l'hydrogène ou du méthyle et dans lesquelles R" est d'un alkylène linéaire ou ramifié, et dans lesquelles de préférence T = -(CH₂)₂- ou-(CH₂)₃-.

11. Polymère de siloxane selon l'une des revendications 1 à 10,
**caractérisé en ce que**
dans les résidus -Q1 et -Q2 de formule (I), au moins l'un des résidus bivalents -Q1"- et -Q2"-, étant respectivement indépendants, est un résidu bivalent contenant le cyclohexane choisi parmi les formules Va et Vb, 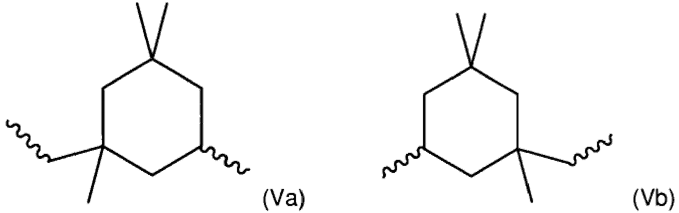 dans lesquelles, en particulier, Q2"- est un résidu bivalent contenant le cyclohexane de formule Va et -Q1" est un résidu bivalent contenant le cyclohexane de formule Vb.

12. Polymère de siloxane selon l'une des revendications 1 ou 2 et 6 à 11,
**caractérisé en ce que**
le siloxane de formule générale I correspond au polymère de siloxane de formule générale XI dans lequel :
Mₐ₁M^{A}ₐ₂M^{B}ₐ₃D_{b1}D^{A}_{b2}D^{B}_{b3}T_{c1}T^{A}_{c2}T^{B}_{c3}Q_{d1} (XI)
dans laquelle M = [R¹⁶₃SiO_{1/2}], M^{A} = [R¹⁷R¹⁶₂SiO_{1/2}], M^{B} = [R¹⁸R¹⁶₂SiO_{1/2}], D = [R¹⁶₂SiO_{2/2}], D^{A} = [R¹⁷₁R¹⁶₁SiO_{2/2}], D^{B} = [R¹⁸1R¹⁶1SiO_{2/2}], T = [R¹⁶SiO_{3/2}], T^{A} = [R¹⁷SiO_{3/2}], T^{B} = [R¹⁸SiO_{3/2}], Q = [SiO_{4/2}],
dans lequel ce qui suit s'applique :
R¹⁶ correspond à des résidus d'hydrocarbures indépendants les uns des autres, identiques ou différents, linéaires ou ramifiés, saturés ou insaturés, avec 1 à 30 atomes de carbone, ou également à des résidus d'hydrocarbures aromatiques avec 6 à 30 atomes de carbone, de préférence méthyle ou phényle, en particulier méthyle,
R¹⁷ est respectivement indépendant de -Q1 ou de-Q2,
les R¹⁸ correspondent à des résidus d'hydrocarbures et sont indépendants les uns des autres, identiques ou différents, linéaires ou ramifiés, saturés ou oléfiniquement insaturés, avec 8 à 30 atomes de carbone, ou également à des résidus d'hydrocarbures aromatiques avec 6 à 40 atomes de carbone, ou à un résidu d'alkylaryle avec 7 à 40 atomes de carbone, par l'intermédiaire d'un ou de plusieurs hétéroatomes, tel que de l'oxygène, NH, NR', dans lequel R' représente un résidu d'alkyle avec 1 à 30 atomes de carbone, contenant le cas échéant des doubles liaisons, à un résidu d'hydrocarbures aliphatique, discontinu, linéaire ou ramifié, contenant le cas échéant des doubles liaisons, avec 2 à 30 atomes de carbone, par l'intermédiaire d'une ou de plusieurs fonctionnalités, choisi parmi les groupes -OH, -O-C(O)-, -(O)C-O-, -NH-C(O)-, -(O)C-NH, -(CH₃)N-C(O)-, -(O)C-N(CH₃)-, -S(O₂)-O-, -O-S(O₂)-, -S(O₂)-NH-, -NH-S(O₂)-, -S(O₂)-N(CH₃)-, - N(CH₃)-S(O2)-, ou à des résidus d'hydrocarbures aliphatiques, discontinus, linéaires ou ramifiés, contenant le cas échéant des doubles liaisons, avec 2 à 30 atomes de carbone,
une terminaison OH, OR', NH², N(H)R', N(R')₂, où R' représente un résidu d'alkyle, contenant le cas échéant des doubles liaisons, avec 1 à 30 atomes de carbone, ou à des résidus d'hydrocarbures fonctionnalisés, linéaires ou ramifiés, aliphatiques ou cycloaliphatiques, contenant le cas échéant des doubles liaisons, avec 1 à 30 atomes de carbone, ou à un polyéther structuré par séquences ou statistiquement conformément à -(R⁵-O)ₙ-R⁶, dans lequel R⁵ est un résidu d'hydrocarbures linéaire ou ramifié contenant 2 à 4 atomes de carbone, dans lequel n est une valeur comprise entre 1 et 100, de préférence entre 2 et 60, et dans lequel R⁶ est l'hydrogène, ou à un résidu d'hydrocarbures aliphatique, linéaire ou ramifié, contenant le cas échéant des doubles liaisons, avec 1 à 30 atomes de carbone, ou à un résidu d'hydrocarbures cycloaliphatique, contenant le cas échéant des doubles liaisons, avec 5 à 40 atomes de carbone, ou à un résidu d'hydrocarbures aromatiques avec 6 à 40 atomes de carbone, ou à un résidu d'alkylaryle avec 7 à 40 atomes de carbone, ou à un résidu de - C(O)-R⁷, dans lequel R⁷ représente un résidu d'hydrocarbures aliphatique, linéaire ou ramifié, contenant le cas échéant des doubles liaisons, avec 1 à 30 atomes de carbone, ou à un résidu d'hydrocarbures cycloaliphatique, contenant le cas échéant des doubles liaisons, avec 5 à 40 atomes de carbone, ou à un résidu d'hydrocarbures aromatique avec 6 à 40 atomes de carbone, ou à un résidu d'alkylaryle avec 7 à 40 atomes de carbone avec les indices suivants :
a1 = 0 à 200, de manière préférentielle 1 à 60, en particulier 0 ;
a2 = 0 à 30, de manière préférentielle 1 à 20, en particulier 2 à 10 ;
a3 = 0 à 30, de manière préférentielle 1 à 20 ;
b1 = 2 à 5000, de manière préférentielle 10 à 1000 ;
b2 = 0 à 100, de manière préférentielle 1 à 30, en particulier 1 à 10 ou 0 ;
b3 = 0 à 100, de manière préférentielle 0 à 30, en particulier 1 à 10 ou 0 ;
c1 = 0 à 30, de manière préférentielle 1 à 30,
c2 = 0 à 30, de manière préférentielle 0 à 5, en particulier 0 ;
c3 = 0 à 30, de manière préférentielle 0 à 5, en particulier 0 ;
d1 = 0 à 30, de manière préférentielle 0 à 5, de préférence 0,
à la condition qu'au moins l'un des indices choisis parmi a2 et a3 soit différent de 0, dans lequel a2 est de préférence un nombre entier compris entre 2 et 10.

13. Polymère de siloxane selon l'une des revendications 1 à 12,
**caractérisé en ce que**
ledit polymère de siloxane est choisi parmi les polymères de siloxane des formules la et Ib ou de mélanges de ceux-ci, 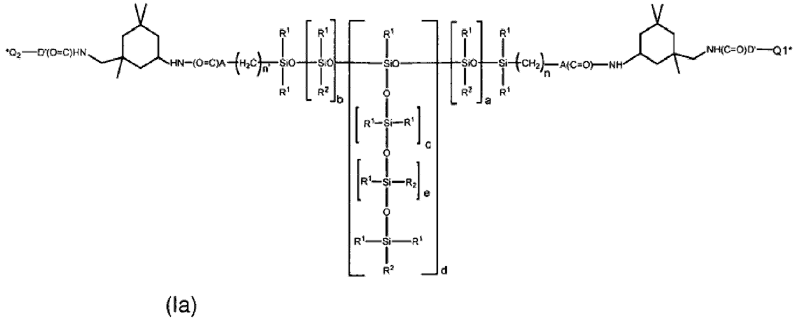 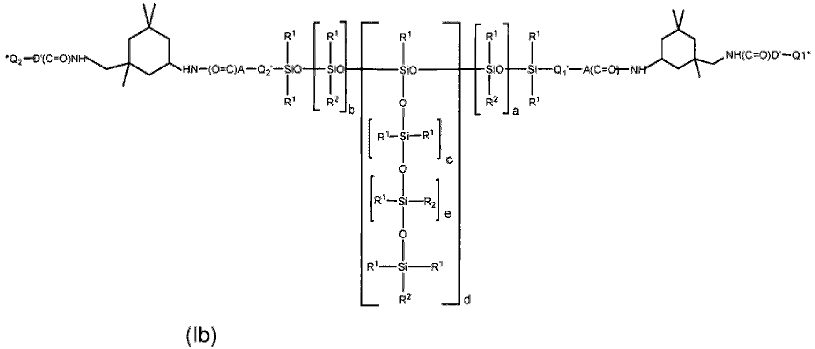 dans lesquelles n ou n', étant respectivement indépendants, est choisi parmi un nombre entier compris entre 3 et 22, dans lesquelles a est compris entre 1 et 200, dans lesquelles b est compris entre 0 et 200, dans lesquelles c est compris entre 0 et 200, dans lesquelles d est compris entre 0 et 200, dans lesquelles e est compris entre 0 et 200 et dans lesquelles les R¹, étant respectivement indépendants, sont identiques ou différents dans les formules la et Ib, dans lesquelles R¹ comprend des résidus d'alkyle avec 1 à 4 atomes de carbone ou des résidus de phényle, dans lesquelles R² est un résidu d'alkyle avec 1 à 22 atomes de carbone, de préférence avec 1 à 4 atomes de carbone, ou un résidu d'alkyle avec au moins un hétéroatome choisi parmi N, O S ou d'un résidu de phényle et dans lequel -D'-Q1* et -D'-Q2*, étant respectivement indépendants, sont dérivés d'acides aminés, des dérivés d'acides aminés ou de leurs sels, et dans lesquelles Q1' et Q2', étant respectivement indépendants dans la formule Ib, représentent un alkylène avec 2 à 40 atomes de carbone ou un polyéther des formules IVa ou IVb :
-T-O-(CH₂-CH₂-O-)ₓ-(CH₂-CH(R^{#})O-)_{y}-(SO)-R" (IVa)
-T-O-(CH₂-CH₂-O-)ₓ-(CH₂-CH(R^{#})O-)_{y}-R" (IVb)
dans lesquelles T = résidus bivalents d'hydrocarbures avec 2 à 4 atomes de carbone, dans lesquelles x = 0 à 200, y = 0 à 200, et où x et y sont des nombres entiers, à la condition que x ou y soit au moins égal à 1, dans lesquelles R^{#} est l'hydrogène ou le méthyle, dans lesquelles R" est l'hydrogène ou un alkylène linéaire ou ramifié, dans lesquelles en particulier T = -(CH₂)₂- ou -(CH₂)₃-.

14. Polymère de siloxane selon l'une des revendications 1 à 13,
**caractérisé en ce que**
ledit polymère de siloxane est choisi parmi des polymères de siloxane de formule Ia* et Ib* 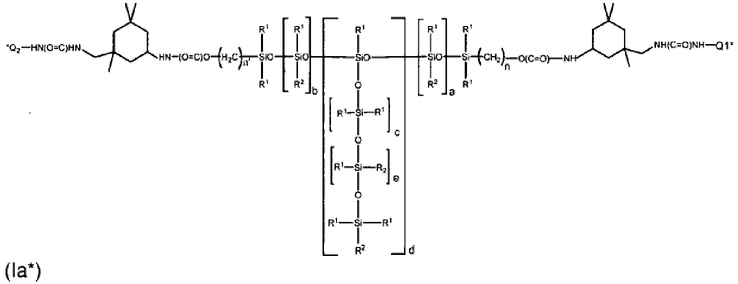 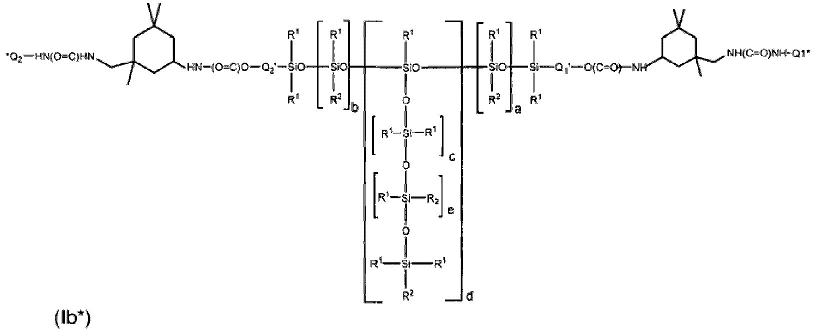 dans lesquelles n ou n', étant respectivement indépendants, est choisi parmi un nombre entier compris entre 3 et 22, dans lesquelles a est compris entre 1 et 200, dans lesquelles b est compris entre 0 et 200, dans lesquelles c est compris entre 0 et 200, dans lesquelles d est compris entre 0 et 200, dans lesquelles e est compris entre 0 et 200 et dans lesquelles R¹, étant respectivement indépendant, est égal ou différent dans les formules Ia* et Ib*, dans lesquelles R¹ comprend des résidus d'alkyle avec 1 à 4 atomes de carbone ou des résidus de phényle, dans lesquelles R² comprend un résidu d'alkyle avec 1 à 22 atomes de carbone, de préférence avec 1 à 4 atomes de carbone, dans lesquelles un résidu d'alkyle avec au moins un hétéroatome comprenant N, 0, S, ou un résidu de phényle et dans lesquelles Q1* et Q2*, en étant respectivement indépendants, sont sélectionnés à partir d'acides aminés, des dérivés d'acides aminés ou de leurs sels, dans lesquelles le fragment -NH-Q1* et -NH-Q2* forme un groupe constitué par l'urée par la réaction des groupes alpha amino secondaires des acides aminés, des dérivés d'acides aminés ou de leurs sels avec l'isocyanate, et dans lesquelles les acides aminés comprenant des acides aminés non polaires comprenant l'alanine, la valine, la méthionine, la leucine, l'isoleucine, la proline, le tryptophane, la phénylalanine, et des acides aminés basiques comprenant la lysine, l'arginine, l'histidine, et des acides aminés polaires et neutres comprenant la tyrosine, la thréonine, la glutamine, la glycine, la sérine, la cystéine, l'asparagine et/ou les acides aminés acides choisis parmi l'acide glutamique et l'acide aspartique, ainsi que leurs esters d'acide monocarboxylique, leurs esters d'acide dicarboxylique, les amides avec des groupes amino primaires des acides aminés, des amides des groupes constitués par un acide carboxylique des acides aminés et/ou les esters avec les groupes primaires constitués par l'hydroxy ou des thioesters du groupe HS ou leurs sels, et dans lequel Q1' et Q2', étant respectivement indépendants, sont identiques dans la formule Ib* :
-T-O-(CH₂-CH₂-O-)ₓ-(CH₂-CH(R^{#})O-)_{y}(SO)-R" (IVa)
-T-O-(CH₂-CH₂-O-)ₓ-(CH₂-CH(R^{#})O-)_{y}-R" (IVb),
dans lesquelles T = des résidus bivalents d'hydrocarbures avec 2 à 4 atomes de carbone, dans lesquelles x = 0 à 200, y = 0 à 200, et dans lesquelles x et y sont des nombres entiers, à la condition que x ou y soit au moins égal à 1, dans lesquelles R^{#} est l'hydrogène ou le méthyle, dans lesquelles R" est l'hydrogène ou un alkylène linéaire ou ramifié, en particulier un éthylène, et en particulier dans lequel T = -(CH₂)₂- ou -(CH₂)₃-.

15. Procédé destiné à la production d'un polymère de siloxane, ainsi qu'à la production de compositions contenant ces polymères de siloxane ou ces mélanges de polymères de siloxane, avec une séquence de polymère B de polysiloxane central :
a) en mettant en réaction un diisocyanate de polysiloxane de formule VII :
OCN-Q2"-D-(O=C)-A-Q2'-B-Q1'-A-(C=O)-D-Q1"-NCO (VII)
dans lequel au moins un acide aminé, un dérivé de l'acide aminé, de son sel ou des mélanges de ceux-ci sont mis en réaction et dans lequel est obtenu un polymère de siloxane de formule générale I :
Q2-B-Q1 (I)
dans laquelle -Q1 correspond à la formule générale IIa et -Q2 correspond à la formule IIb :
-Q1 = -Q1'-A-(C=O)-D-Q1"-A'-(C=O)-D'-Q1* (IIa)
-Q2 = -Q2'-A-(C=O)-D-Q2"-A'-(C=O)-D'-Q2* (IIb)
- dans lesquelles A représente -NH-, -O- ou -S- et D représente -NH-, en étant respectivement indépendants dans les formules IIa et IIb,
- dans lesquelles A' représente -NH- et D' représente -NH-, -0- ou -S-, en étant respectivement indépendants dans les formules IIa et IIb, dans lesquelles chaque résidu Q1 et Q2 de formule IIa ou IIb, en étant respectivement indépendant, présente au moins un groupe bivalent constitué par l'urée et un autre groupe bivalent constitué par l'urée ou un groupe constitué par le carbamate ; ou
b) en mettant en réaction un polysiloxane de formule VI :
HA-Q2'-B-Q1'-AH (VI)
dans laquelle un isocyanate d'acide aminé est choisi parmi les formules IXa, IXb, IXc, IXd ou des mélanges contenant celles-ci :
Q2*-NH(CO)NH-"2Q-NCO (IXa)
Q1*-NH(CO)NH-"1-NCO (IXb)
Q2*-NH(CO)NH-Q2"-NCO (IXc)
Q1*-NH(CO)NH-Q1"-NCO (IXd)
- dans lesquelles A représente -NH-, -O- ou -S- et D représente -NH-, en étant respectivement indépendants dans les formules VII, I et VI ;
- dans lesquelles Q1' et Q2', en étant respectivement indépendants, comprennent un résidu bivalent d'hydrocarbures avec 6 à 200 atomes de carbone, comprenant facultativement au moins un hétéroatome 0, N ou S, ou un résidu bivalent comprenant des groupes constitués par aryle ou arylalkyle, ou un résidu bivalent comprenant des groupes constitués par aryle et arylalkyle, comprenant facultativement au moins un hétéroatome 0, N ou S ou des groupes consistant en alkyle ou aryle, d'une part, ou en alkyle et aryle, d'autre part, contenant des résidus de polyéther, en étant respectivement indépendants dans les formules VII, I et VI ;
- dans lesquelles Q1" et Q2", en étant respectivement indépendants, comprennent un résidu bivalent d'alkyle linéaire, ramifié et/ou cyclique avec 4 à 200 atomes de carbone, ou un résidu bivalent comprenant un résidu d'aryle et/ou d'arylalkyle avec 6 à 200 atomes de carbone, en étant respectivement indépendants dans les formules VII, I, IXa, IXb, IXc et IXd ;
- dans lesquelles -NH-Q1* et -NH-Q2* comprennent des résidus respectivement indépendants qui sont dérivés d'un acide aminé, d'un dérivé d'acide aminé ou de son sel.

16. Procédé selon la revendication 15,
**caractérisé en ce que**
dans les formules IXa, IXb, IXc et/ou IXd et I, -NH-Q1* et -NH-Q2* comprennent des résidus respectivement indépendants qui sont dérivés d'un acide aminé, d'un dérivé d'acide aminé ou de son sel, et où -NH se trouve respectivement en position alpha dans -NH-Q1* et -NH-Q2* par rapport à un groupe consistant en l'ester ou un groupe de l'acide aminé constitué par le carboxyle, à un dérivé de l'acide aminé ou à son sel, dans lequel les esters comprennent un ester d'alkyle avec 1 à 25 atomes de carbone ou un ester d'aryle.

17. Procédé selon la revendication 15 ou 16,
**caractérisé en ce que**
il est employé un polysiloxane de formule VI :
HA-Q2'-B-Q1'-AH (VI)
dans laquelle A est choisi parmi -0, -NH, -S-, respectivement dans laquelle -AH est choisi parmi-OH, -NH₂, et -SH où -Q2'- et -Q1'- sont tels que définis plus haut, et est mis en réaction avec un diisocyanate pour donner lieu à un diisocyanate de polysiloxane de formule VII :
OCN-Q2"-D-(O=C)-A-Q2'-B-Q1'-A-(C=O)-D-Q1"-NCO (VII)
dans laquelle -Q2"- et/ou -Q1"- sont indépendamment choisis parmi un résidu bivalent d'alkyle linéaire, ramifié et/ou cyclique avec 4 à 200 atomes de carbone, ou un résidu bivalent comprenant un résidu d'aryle et/ou d'arylalkyle avec 6 à 200 atomes de carbone, dans laquelle le rapport molaire des groupes HA dans le polysiloxane par rapport aux groupes constitués par l'isocyanate dans la formule VII est au moins égal à 1 pour 1, en particulier ledit rapport molaire est compris entre 1 pour 100 et 1 pour 1, de préférence entre 1 pour 10 et 1 pour 1.

18. Procédé selon l'une des revendications 15 à 17, **caractérisé en ce que**
(i) a) une séquence de polymère B, linéaire et/ou ramifié, contenant des groupes constitués par le polysiloxane, en particulier des formules IIIa et/ou IIIb, avec au moins deux groupes Si-H terminaux ou au moins un groupe Si-H terminal et au moins un groupe Si-H en chaînes latérales, ou b) un groupes de polysiloxane de formule XI, dans lequel au moins un R¹⁷ est l'hydrogène, de préférence dans lequel deux R¹⁷ sont l'hydrogène ;
(ii) une mise en réaction est réalisée avec une liaison oléfinique comprenant un alkylène et comprenant facultativement au moins un hétéroatome N et/ou 0, en particulier l'alcénylène, une alkylène-amine, une alkylamine quaternaire, un acide alkylène carboxylique, un alkylène ester, un alkylène amide ou un polyéther oléfinique, dans lequel la liaison oléfinique, en étant respectivement indépendante, présente un groupe constitué par l'allyle ou par le vinyle et correspond aux formules VIIIa et/ou VIIIb :
Q1'-AH (VIIIa)
Q2'-AH (VIIIb)
(iii) et la mise en réaction en présence d'un catalyseur donne lieu à un polysiloxane de formule VI :
HA-Q2'-B-Q1'-AH (VI)
dans laquelle, en étant respectivement indépendants dans les formules VIIIa, VIIIb et VI, AH est sélectionné indépendamment à partir de -OH et de -NH₂ et dans laquelle -Q2'- et -Q1'-, en étant respectivement indépendants dans les formules VIIIa, VIIIb et VI comprennent un résidu bivalent d'hydrocarbure avec 6 à 200 atomes de carbone, comprenant facultativement au moins un hétéroatome 0 ou N, ou un résidu bivalent comprenant des groupes constitués par l'aryle ou l'arylalkyle, comprenant facultativement au moins un hétéroatome 0 ou N ou un polyéther oléfinique.

19. Procédé selon la revendication 15,
**caractérisé en ce que**
un diisocyanate de polysiloxane de formule VII :
OCN-Q2"-D-(O=C)-A-Q2'-B-Q1'-A-(C=O)-D-Q1"-NCO (VII)
dans laquelle B correspond à une séquence de polymère B de polysiloxane linéaire et/ou ramifié, dans laquelle -Q2'- et -Q1'-, en étant respectivement indépendants, comprennent un résidu bivalent d'hydrocarbure avec 6 à 200 atomes de carbone, comprenant facultativement au moins un hétéroatome 0, N ou S, ou un résidu bivalent comprenant des groupes constitués par aryle ou arylalkyle, comprenant facultativement au moins un hétéroatome 0, N ou S, ou des résidus de polyéther contenant des groupes consistant en alkyle ou aryle, d'une part, ou en alkyle et aryle, d'autre part, dans laquelle les A, en étant respectivement indépendants, représentent -NH-, -0- ou -S- et les D représentent -NH-, en étant respectivement indépendants dans la formule VII, et dans laquelle -Q2"- et/ou -Q1"- sont indépendamment choisis parmi un résidu bivalent d'alkyle linéaire, ramifié et/ou cyclique avec 4 à 200 atomes de carbone, ou un résidu bivalent comprenant un résidu d'aryle et/ou d'arylalkyle avec 6 à 200 atomes de carbone, est mis en réaction avec un acide aminé, un dérivé d'acide aminé, leurs sels ou des mélanges de ceux-ci, en particulier avec un groupe amino secondaire de l'acide aminé ou du dérivé d'acide aminé.

20. Procédé selon la revendication 15 ou 19,
**caractérisé en ce que**
l'acide aminé, le dérivé d'acide aminé ou son sel présente un groupe amino secondaire, et en particulier le groupe secondaire constitué par l'acide aminé est mis en réaction avec un groupe consistant en l'isocyanate, dans lequel l'acide aminé, le dérivés d'acide aminé ou leurs sels sont choisis parmi des acides aminés non polaires choisis parmi l'alanine, la valine, la méthionine, la leucine, l'isoleucine, la proline, le tryptophane, la phénylalanine, ou bien parmi des acides aminés basiques comprenant l'arginine, l'histidine, ou parmi des acides aminés polaires et neutres comprenant la tyrosine, la thréonine, la glutamine, la glycine, la sérine, la cystéine, l'asparagine et/ou parmi des acides aminés acides choisis parmi l'acide glutamique et l'acide aspartique, dans lequel les dérivés d'acides aminés comprennent des esters d'acide monocarboxylique, des esters d'acide dicarboxylique, des amides des groupes amino primaires des acides aminés, des amides des groupes consistant en l'acide carboxylique des acides aminés et/ou des esters avec les groupes primaires constitués par l'hydroxy ou les thioesters du groupe HS des acides aminés, en particulier les esters d'alkyle des acides aminés, de préférence les esters de méthyle, d'éthyle et de phényle des acides aminés ou de leurs sels.

21. Procédé selon la revendication 15,
**caractérisé en ce que**
un diisocyanate est mis en réaction avec un dérivé d'acide aminé ou son sel pour donner lieu à un isocyanate d'acide aminé, en particulier à un isocyanate d'acide aminé choisi parmi les formules IXa et IXb telles que définies plus haut.

22. Composition obtenue conformément à un procédé selon l'une des revendications 15 à 21.

23. Compositions contenant des polymères de siloxane selon l'une des revendications 1 à 14, ainsi que des mélanges de ceux-ci, comprenant : a) des polymères de siloxane de formule générale XI, ainsi que des mélanges de ceux-ci ; ou b) des polymères de siloxane avec une séquence de polymère B de polysiloxane central, choisie parmi :
(i) au moins un polymère de siloxane de formule générale I, ainsi que des mélanges comprenant ce polymère ;
(ii) au moins un polymère de siloxane de formule générale la, ainsi que des mélanges comprenant ce polymère ; ou
(iii) au moins un polymère de siloxane de formule générale Ib, ainsi que des mélanges comprenant ce polymère.

24. Produit intermédiaire destiné à la production de polymères de siloxane de formule I selon l'une des revendications 1 à 14, choisis parmi des isocyanates d'acide aminé, choisis parmi les formules IXa, IXb, IXc et IXd, leurs sels ou des mélanges de ceux-ci :
Q2*-NH(CO)NH-"2Q-NCO (IXa)
et/ou
Q1*-NH(CO)NH-"1Q-NCO (IXb)
et/ou
Q2*-NH(CO)NH-Q2"-NCO (IXc)
et/ou
Q1*-NH(CO)NH-Q1"-NCO (IXd),
dans lesquelles Q2*, Q2", Q1* et Q1" sont tels que définis plus haut, en particulier avec des isocyanates d'acide aminé des formules IXa*, IXb*, IXc*, IXd* ou leurs sels :

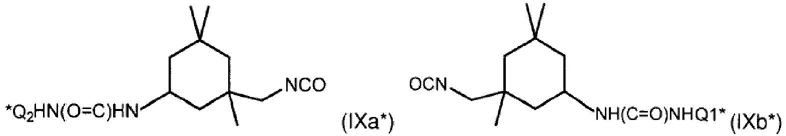

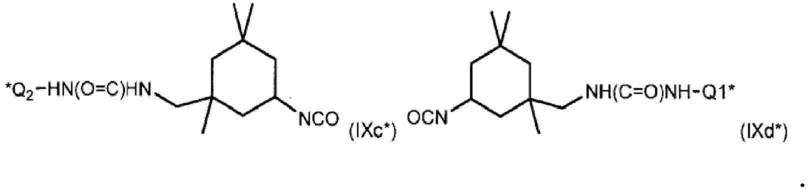

25. Formulation contenant au moins un polymère de siloxane selon l'une des revendications 1 à 14, 22, 23 ou réalisée selon l'une des revendications 15 à 20 ou réalisée au moyen du produit intermédiaire selon la revendication 24 et au moyen d'au moins un adjuvant.

26. Utilisation des polymères de siloxane selon l'une des revendications 1 à 14, ou des compositions obtenues comprenant des polymères de siloxane selon l'une des revendications 15 à 21, ou des compositions selon la revendication 21 ou 22 en tant qu'additif dans des formulations cosmétiques, en tant qu'additif dans des formulations pharmaceutiques, dans des laques ou dans des pâtes, en tant que stabilisateur de mousse ou additif moussant pour des mousses de polyuréthane, en particulier des mousses rigides de polyuréthane et des mousses souples de polyuréthane, en tant qu'agent d'amélioration tactile ou agent d'imprégnation lors de lors de la fabrication de fibres ou de textiles, dans des formulations cosmétiques en vue du traitement, de l'après-traitement et de la protection des fibres kératiniques, ainsi que de la peau et des phanères, en tant qu'additif dans des formulations pour des détergents de lavage ou des assouplissants, dans des formulations cosmétiques comprenant des crèmes, des agents de rinçage, des shampooings capillaires, des agents de lavage, des fixateurs pour cheveux, des après-shampooings, des pâtes pour le soin des cheveux, des sprays ou des laques pour cheveux en vue d'améliorer la souplesse et le démêlage des fibres kératiniques ou textiles d'origine naturelle ou synthétique.
